(19) Europäisches Patentamt
European Patent Office
Office européen des brevets

(11) EP 1 046 394 A2

# (12) EUROPEAN PATENT APPLICATION

(43) Date of publication:
**25.10.2000 Bulletin 2000/43**

(51) Int. Cl.[7]: **A61K 9/127**, A61K 48/00, C12N 15/88

(21) Application number: **00303249.7**

(22) Date of filing: **18.04.2000**

(84) Designated Contracting States:
**AT BE CH CY DE DK ES FI FR GB GR IE IT LI LU MC NL PT SE**
Designated Extension States:
**AL LT LV MK RO SI**

(30) Priority: **19.04.1999 US 294623**

(71) Applicant:
**ImaRx Pharmaceutical Corp.**
**Tucson, AZ 85719 (US)**

(72) Inventors:
- **Unger, Evan C.**
  **Tucson, AZ 85710 (US)**
- **McCreery, Thomas**
  **Tucson, AZ 85712 (US)**
- **Sadewasser, David A.**
  **San Diego, CA 92103 (US)**

(74) Representative:
**James, Anthony Christopher W.P. et al**
**Carpmaels & Ransford**
**43 Bloomsbury Square**
**London WC1A 2RA (GB)**

## (54) Novel compositions useful for delivering compounds into a cell

(57) The present invention is directed, *inter alia*, to compositions and their use for delivering compounds into a cell. In a preferred embodiment, the compositions comprise, in combination with the compound to be delivered, an organic halide, a targeting ligand, and a nuclear localization sequence, optionally in the presence of a carrier. Ultrasound may be applied, if desired. The compositions are particularly suitable for the treatment of inflammatory diseases.

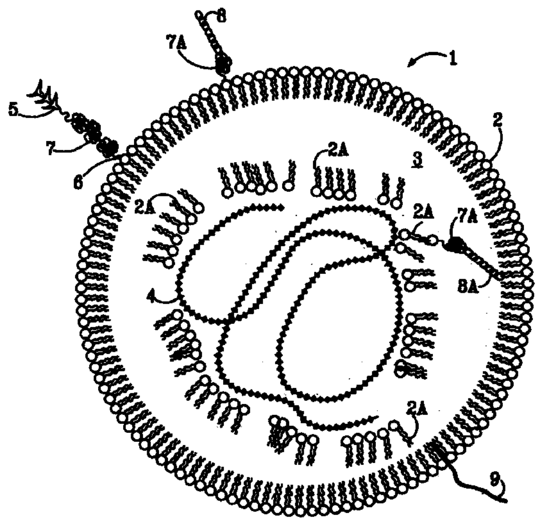


FIG. 2

EP 1 046 394 A2

## Description

## Cross-Reference to Related Applications

This application is a continuation-in-part of U.S. application Serial No. 08/841,169, filed April 29, 1997, which in turn is a continuation-in-part of U.S. application Serial No. 08/785,661, filed January 17, 1997, now abandoned, which in turn is a continuation-in-part of U.S. application Serial No. 08/640,554, filed May 1, 1996, now abandoned. The disclosures of each of the foregoing applications are hereby incorporated herein by reference, in their entirety.

## Field of the Invention

The present invention relates to novel compositions and the use thereof. More particularly, the present invention relates to novel compositions which may be used to deliver compounds into a cell.

## Background of the Invention

Rheumatoid arthritis (RA) is a chronic and systemic inflammatory disease primarily involving joints. The antigenic stimulus that elicits immune responses and subsequent inflammation in rheumatoid arthritis is not well understood. The synovium of patients with RA is generally characterized, for example, by the proliferation of macrophages at the synovial lining, infiltration of T and B cells and tissue macrophages, accumulation of polymorphonuclear cells in synovium and synovial fluid, proliferation of synovial fibroblasts, and deposition of fibrin. Once in the synovium, leukocytes produce numerous inflammatory cytokines such as, for example, tumor necrosis factor-$\alpha$ (TNF-$\alpha$), interleukin 2 (IL-2), leukemia inhibitory factor, granulocyte-macrophage colony-stimulating factor (GM-CSF), and IL-6. Adhesion molecules on the surface of the above-described leukocytes may play an integral role in the pathogenesis of RA. A review of the role of adhesion molecules in RA is provided in "Role Of Adhesion Molecules In The Pathogenesis Of Rheumatoid Arthritis," Liao, *et al*., Rheumatic *Dis. Clinics North Amer*., **1995**, *21*:715-40, "Cellular Adhesion Molecules In Rheumatoid Arthritis: Regulation By Cytokines And Possible Clinical Importance," Szekanecz, *et al.*, *.J. Invest. Med.*, **1996**, *44*:124-35, "Cell Adhesion Molecules In Rheumatoid Arthritis," Haskard, *et al., Curr. Opin. Rheumatol.,* **1995**, *7*:229-34; "Cell Adhesion Molecules In Rheumatoid Arthritis," Veale, *et al., Drugs & Aging*, **1996**, *9*:87-92; and "Adhesion Molecules In The Pathogenesis Of Rheumatoid Arthritis," Cronstein, *Curr. Opin. Rheumatol*., **1994**, *6*:300-04, the disclosures of each of which are incorporated herein by reference in their entirety.

Integrins are a family of cell surface adhesion receptors by which cells attach to extracellular matrices and mediate cell-cell adhesion events in RA. A review of integrins is provided in "The Extracellular Matrix In Epithelial Biology: Shared Molecules And Common Themes In Distant Phyla," Ashkenas, *et al., Dev. Biol*., **1996**, *180*:433-44, "Integrins: Emerging Paradigms Of Signal Transduction," Schwartz, *et al., Annu. Rev*. *Cell Dev. Biol*., **1995**, *11*:549-99, and "Integrins: Versatility, Modulation, And Signaling In Cell Adhesion," *Hynes, Cell*, **1992**, *69*:11-25, the disclosures of each of which are hereby incorporated herein be reference in their entirety. A review of cell signaling in leukocyte emigration is provided in "Traffic Signals For Lymphocyte Recirculation And Leukocyte Emigration: The Multistep Paradigm," Springer, *Cell*, **1994**, *76*:301-14, the disclosures of which are hereby incorporated herein be reference in their entirety.

Current treatment protocols include physical therapy, drug treatment with compounds such as, for example, salicylates, nonsteroidal anti-inflammatory agents, antimalarials, penicillamine, corticosteroids and immunosuppressive agents, and orthopedic surgery. These treatment protocols, however, generally have significant limitations. For example, physical therapy merely treats the symptoms and manifestations of rheumatoid arthritis and does not prevent the cause or causes thereof. Orthopedic surgery is highly invasive and is preferably used only as a last resort. Current drug treatment protocols have numerous deficiencies including, for example, non-specificity and inefficient uptake. To this end, the overall therapeutic strategy for inhibiting autoimmune inflammation at the causative level generally involves: (1) interfering with a particular ligand/receptor interaction which may be an upstream requirement for triggering an ultimate inflammatory factor such as proliferation of tumor necrosis factor (TNF); (2) immunotherapy to stimulate production of antibodies or other immune factors which may bind with and thereby remove secondary inflammatory elements; and/or (3) gene therapy aimed at interfering with the tissue-specific overproduction of autoimmune factors. Much research has concentrated on procedures for delivering therapeutic compounds to cells.

Cells are the basic structural and functional units of all living organisms. Cells contain cytoplasm surrounded by a plasma, or cell, membrane. Most bacterial and plant cells are enclosed in an outer rigid or semi-rigid cell wall. The cells contain DNA which may be arranged in (1) a nuclear membrane or (2) free in cells lacking a nucleus. While the cell membrane may contain ion channels, compounds that may be therapeutically advantageous to cells generally are too large to pass through these ion channels. Conventional interventional methods of delivery of compounds into cells have proved difficult in view of the need for the compounds to pass through the cell membrane, cell wall, and nuclear

membrane.

With respect to gene therapy, it has generally been difficult to deliver macromolecules to the cell nucleus, which is the site for genetic material in eukaryotes. The nuclear envelope forms a membrane surrounding the cell nucleus which excludes most large molecules from entering into the nucleus. In general, all transport goes through the nuclear envelope-localized nuclear pore channels (NPCs), which are typically very large structures (about $10^5$ kDa) and are generally composed of 30 or more distinct protein components. Between 100 and 5 x $10^7$ NPCs are typically present on each nuclear membrane. The number of NPCs may vary depending, for example, upon the metabolic and differentiation state of the cell. NPCs generally have a pore or molecular sieve function which may allow molecules smaller than 40-45 kDa to diffuse freely between cytoplasm and the nucleus. Larger molecules, however, generally must be transported across the NPC in an energy dependent process. Up until now, it has been difficult to achieve localization of large quantities of nuclear macromolecules. Accordingly, researchers have undertaken the development of a variety of intracellular delivery methods for inserting genes and other compounds into both plant and animal cells.

For example, calcium phosphate DNA precipitation has been used to deliver genetic material into cells in cell culture. However, a drawback of this method is that the resultant efficiency of transfection (delivery of the genetic material into the cells) and subsequent gene expression has been very low.

Improved transfection has been attained using viral vectors, *e.g*., adenovirus and retrovirus, but again, difficulties with gene expression have persisted. In addition, substantial concerns regarding antigenicity and the potential of mutant viruses and other possible deleterious effects exist.

Liposomes, which may be manufactured more readily than viral vectors, have shown promise as delivery agents. Liposomes generally have less biological concerns (in that for example, they are generally non-antigenic) but the efficiency of transfection and gene expression using liposomes has typically been lower than with viruses.

Gene guns, wherein genes are attached to heavy metal particles such as gold, have been used to fire the particles at high speed into cells. While gene guns have resulted in gene expression in culture systems, they have generally not provided desirable transfection *in vivo*. Furthermore, the blast of heavy metal particles may cause damage to the cells and may result in the introduction of undesirable foreign materials, *e.g*. gold particle fragments, into the cells.

Electroporation is another method of delivering compounds such as genetic material into cells. In this technique, pulses of electrical energy are applied to cells to create pores or openings to facilitate passage of DNA into the cells. However, electroporation may damage cells, and furthermore has generally not been shown to be highly effective *in vivo*.

Various publications disclose the use of lithotripsy shock waves for effecting intracellular gene transfer, as well as the delivery of other compounds. *See, e.g*., Delius, M., *et al*., "Extracorporeal Shock Waves for Gene Therapy," *Lancet* **May 27, 1995**, *345*:1377; Lauer, U., *et al*., "Towards A New Gene Transfer System: Shock Wave-Mediated DNA Transfer," *J Cell Biochem* **1994**, 16A:226; Gambihler, S., *et al*., "Permeabilization of the Plasma Membrane of L1210 Mouse Leukemia Cells Using Lithotripter Shock Waves," *J Membr Biol* **1994**, *141* :267-75; and Mobley, T.B., *et al*., "Low Energy Lithotripsy with the Lithostar: Treatment Results with 19,962 Genal and Ureteral Calculi," *J Urol* **1993**, *149*:1419-24, the disclosures of each of which are incorporated herein by reference in their entirety. Lithotripsy typically delivers energy in the range of 200 to 380 bars, and a frequency of 60 to 120 Hz, but may be as high as 1200 to 1300 bars. The energy and frequency ranges are typically painful to a patient and thus usually require patient sedation. In addition, lithotripsy machines are large and bulky and are typically cost prohibitive. Lauer *et al*. disclose the delivery of 250 shock waves at 25 kV with a lithotripter to deliver plasmid DNA which expressed hepatitis B virus surface proteins in a HeLa cell suspension. Gambihler *et al.* teach the permeabilization of mouse cells *in vitro* to deliver dextrans. The lithotripter shock waves are delivered at 25 kV, at a discharge rate of 60/min. Mobley *et al.* disclose the use of lithotripsy to treat renal and ureteral stones. The shock wave pressure was 200 to 380 bar and a generator range of 10 to 29 kV.

In Zhang, L., *et al.*, *Sci Agric. Sin*. **1991**, *24*:83-89, the disclosures of which are incorporated herein by reference in their entirety, there is disclosed increased gene expression by tobacco using continuous wave ultrasound at 0.5 W/$cm^2$ for 30 minutes. Zhang *et al*. do not disclose the ultrasound frequency. The high energy level is in a range necessary for poration to result in the cell wall of tobacco plants.

Rubin, *et al*., 31st Annual Meeting of the American Society of Clinical Oncology, May 20-23, 1995, the disclosures of which are incorporated herein by reference in their entirety, disclose the injection of hepatic tumors with a plasmid/cationic lipid complex with ultrasound guidance. Ultrasound is disclosed as a visual guide to monitor the injection of the tumors, rather than as an aid to deliver the complex to the liver tumors.

A problem often encountered in delivering compounds to cells is the specificity of delivery. Ideally, therapeutic compounds are preferably delivered only to particular target cells. Recently, procedures have been reported which are designed to provide more targeted delivery of therapeutics. For example, International Publication WO 93/19768, the disclosures of which are incorporated herein by reference in their entirety, describes compositions for delivering polynucleotides comprising a membrane-permeabilizing component, such as a liposome, in combination with a cell recognition component and a nuclear-localization component to treat conditions such as cystic fibrosis. International Publication WO 95/34295, the disclosures of which are incorporated herein by reference in their entirety, describes delivery

of biologically active molecules to cells by administering to cells a complex comprising the molecule linked to a signal peptide, optionally using a liposome and a nuclear localization peptide.

The present invention provides new and/or better compositions and methods for delivering compositions, including drugs and genetic material, into a cell. The methods and compositions of the invention may provide a significant advantage over prior art methodology, in that they provide for specific targeting and delivery of compounds to particular cells and increased targeting to the nucleus of the targeted cells. In addition, the methods of the present invention may be performed in cell lines which may be otherwise resistant to intracellular delivery and gene expression using other conventional means. The methods and compositions of the invention may be used to treat autoimmune conditions and inflammatory conditions such as, for example, rheumatoid arthritis. These and/or other aspects of the present invention will become apparent from the further discussions herein.

## Summary of the Invention

The present invention is directed, *inter alia*, to compositions for delivering a compound into a cell. Specifically, in one embodiment, there is provided a composition for delivering a compound into a cell comprising, in combination with the compound to be delivered, an organic halide, a targeting ligand, a nuclear localization sequence, and optionally a fusion peptide.

Another embodiment of the invention relates to a method for delivering a compound into a cell comprising administering to the cell a composition comprising, in combination with the compound to be delivered, an organic halide, a targeting ligand and a nuclear localization sequence.

Still another embodiment of the present invention relates to a method of treating a patient suffering from inflammation comprising administering to the patient a composition which comprises an organic halide, a targeting ligand for targeting cells associated with inflammation, and a nuclear localization sequence, in combination with a therapeutically effective amount of a compound to be delivered to said cells.

Yet another embodiment of the present invention relates to a method of treating a patient suffering from rheumatoid arthritis comprising administering to said patient a composition which comprises an organic halide, a targeting ligand for targeting cells associated with rheumatoid arthritis, and a nuclear localization sequence, in combination with a therapeutically effective amount of a compound to be delivered to said cells.

These, as well as other, aspects of the invention are set forth in greater detail below.

## Brief Description of the Figures

**FIGURE 1** includes schematic representations of a targeted composition according to an embodiment of the present invention at the surface of a high endothelial venule and the sequence of events leading to release of a compound of the composition in the cell nucleus. Schematic representation A includes a depiction of an oblique section of a high endothelial venule. Schematic representation B includes a depiction of a target cell in which B1 represents the cellular membrane containing cell surface proteins B2 that serve as attachment sites for carrier cells and in which C represents a cell nucleus. D represents a targeted composition according to an embodiment of the present invention, comprising a lipid coated halocarbon microbubble and surface molecules including targeting ligand D1 and nuclear localization sequence D2. Schematic representation E is an adhesion molecule bearing cells that may be associated with the walls of the high endothelial venule A. Adhesion molecule E may contain many surface proteins including a receptor E1 to the targeting ligand D1 and a receptor E2 to cell surface proteins of the ultimate target cell.

**FIGURE 2** includes a schematic representation of a targeted composition according to an embodiment of the present invention which depicts exemplary spatial relationships between the targeting ligands and the compound to be delivered to the cell. The targeted composition 1 exemplified in Figure 2 comprises lipids 2 coating a halocarbon gas or liquid 3 (or combination thereof). Also encapsulated within the lipid coating is genetic material 4 which is associated with lipids 2A. Targeting ligand 5 is attached to a head group 6 of a lipid in the lipid coating 2 by a tether 7 of a desired length. Nuclear localization sequence 8 is attached by a tether 7A, which may be the same as or different from the tether 7. Lipids 2A also include a nuclear localization sequence 8A which may be attached directly or a via a tether to a double headed lipid. A condensing agent 9 is included for the genetic material 4.

## Detailed Description of the Invention

As employed above and throughout the disclosure, the following terms, unless otherwise indicated, shall be understood to have the following meanings.

"Organic halide" (also sometimes referred to as a halogenated organic compound) refers to a compound which contains at least one carbon atom (or optionally sulfur or selenium atom, such as in the case of $SF_6$ and $SeF_6$) and at least one halogen atom selected from the group consisting of fluorine, chlorine, bromine, and iodine. Preferably, the halogen is fluorine (*i.e*., the compound is a fluorinated compound). More preferably, the organic halide is a fluorinated compound which is perfluorinated (that is, fully fluorinated, *e.g.*, a carbon compound wherein all of the hydrogen atoms which are attached directly to the carbon atoms have been replaced by fluorine atoms). The perfluorinated organic halide (perfluorinated compound) is preferably a perfluorocarbon or a perfluoroether compound. The organic halide may be in the form of a gas, a liquid (including a gaseous precursor), or a solid. Preferably, the organic halide is a liquid, with liquids which are gaseous precursors that convert to a gas upon administration being more preferred. Even more preferably, the gaseous precursor converts to a gas at the site of the cell, including, for example, close or touching proximity to the cell.

"Gaseous precursor" refers to a liquid or solid which may be activated upon exposure to a certain temperature or pressure to convert to a gas. A gaseous precursor which may be capable of converting to a gas at the site of the cell may increase the efficiency of cellular uptake of compounds, and is therefore preferred. Ideally, the gaseous precursors are liquid (or solid) at ambient (room) temperature (e.g., 25°C), and may convert to a gas at physiological temperature (e.g., 37°C), such as upon administration to a patient or cells or tissue from a patient, or otherwise conveniently at the site of the cell, such as upon application of heat (such as, for example, through the use of ultrasound). Thus, if heat is applied, for example, to the cell, the heat is preferably sufficient to convert the gaseous precursor to a gas but insufficient to harm the cell (*e.g*., by denaturing the proteins). Thus, in preferred form, the gaseous precursor may be convened to a gas at temperatures of less than about 80°C. More preferably, the gaseous precursor may be converted to a gas at temperatures of from about 30°C to about 70°C. Even more preferably, the gaseous precursor may be convened to a gas at temperatures from about 37°C to less than about 50°C.

"Carrier" refers to a composition, substance or material that is capable of transporting or carrying *in vivo* or *in vitro* a compound to be delivered to a cell. Exemplary delivery vehicles include, for example, stabilizing materials, such as lipids, polymers, proteins and surfactants, vesicles, liposomes, micelles, aerogels, clathrates, gas filled vesicles, liquid filled vesicles, gaseous precursor filled vesicles, emulsions, suspensions, dispersions and hexagonal H II phase structures.

"Targeting ligand" refers to any material or substance which may promote targeting and/or recognition of cells, tissues and/or receptors *in vivo* or *in vitro* with the compositions of the present invention. The targeting ligand may be synthetic, semi-synthetic, or naturally-occurring. Materials or substances which may serve as targeting ligands include, for example, proteins, including antibodies, antibody fragments, hormones, hormone analogs, glycoproteins and lectins, peptides, polypeptides, glycopeptides, amino acids, sugars, saccharides, including monosaccharides and polysaccharides, carbohydrates, vitamins, steroids, steroid analogs, hormones, cofactors, bioactive agents, and genetic material, including nucleosides, nucleotides, and polynucleotides. Preferably, the targeting ligands bind cells associated with inflammation. More preferably, the targeting ligands bind cells associated with rheumatoid arthritis.

"Nuclear localization sequence" (NLS) refers to any molecule which may localize, as described herein, to the cell nucleus. Such nuclear localization sequences act to target the cell nucleus, reuslting in subcellular (nuclear) import and lcalization to or concentration in the nucleus. Preferred nuclear localization sequences include, for example, peptides, proteins, receptors, transcription factors, enzymes, and the like.

"Compound to be delivered" refers to a compound which may be present in the compositions of the present invention and which may be advantageously delivered to cells. Compounds to be delivered include, for example, bioactive agents which may be used in connection with an application that is therapeutic or diagnostic in nature.

"Bioactive agent" refers to a substance which may be used in connection with an application that is therapeutic or diagnostic in nature, such as in methods for diagnosing the presence or absence of a disease in a patient and/or in methods for the treatment of disease in a patient. As used herein, "bioactive agent" refers also to substances which are capable of exerting a biological effect *in vitro* and/or *in vivo*. The bioactive agents may be neutral or positively or negatively charged. Examples of suitable bioactive agents include diagnostic agents, pharmaceuticals, drugs, synthetic organic molecules, proteins, peptides, vitamins, steroids and genetic material, including nucleosides, nucleotides and polynucleotides.

"Diagnostic agent" refers to any agent which may be used in connection with methods for imaging an internal region of a patient and/or diagnosing the presence or absence of a disease in a patient. Exemplary diagnostic agents include, for example, contrast agents for use in connection with ultrasound, magnetic resonance imaging or computed tomography of a patient including, for example, the lipid and/or vesicle compositions described herein.

"Vesicle" refers to an entity which is generally characterized by the presence of one or more walls or membranes which form one or more internal voids. Vesicles may be formulated, for example, from stabilizing compounds, such as a lipid, including the various lipids described herein, a polymer, including the various polymers described herein, or a protein, including the various proteins described herein, as well as other materials that will be readily apparent to one skilled in the art. Other suitable materials include, for example, any of a wide variety of surfactants, inorganic

compounds, and other compounds as will be readily apparent to one skilled in the art. Also, as will be apparent to one skilled in the art upon reading the present disclosure, the organic halides may themselves act as suitable carriers, and may in certain embodiments themselves form vesicles and other organized structures. Thus, the use of the organic halides of the invention in combination with a compound to be delivered, without an additional compound to serve as a carrier, is within the scope of the invention. The lipids, polymers, proteins, surfactants, inorganic compounds, and/or other compounds may be natural, synthetic or semi-synthetic. Preferred vesicles are those which comprise walls or membranes formulated from lipids. The walls or membranes may be concentric or otherwise. In the preferred vesicles, the stabilizing compounds may be in the form of a monolayer or bilayer, and the mono- or bilayer stabilizing compounds may be used to form one or more mono- or bilayers. In the case of more than one mono- or bilayer, the mono- or bilayers may be concentric, if desired. Stabilizing compounds may be used to form unilamellar vesicles (comprised of one monolayer or bilayer), oligolamellar vesicles (comprised of about two or about three monolayers or bilayers) or multilamellar vesicles (comprised of more than about three monolayers or bilayers). The walls or membranes of vesicles prepared from lipids, polymers or proteins may be substantially solid (uniform), or they may be porous or semi-porous. The vesicles described herein include such entities commonly referred to as, for example, liposomes, micelles, bubbles, microbubbles, microspheres, lipid-, protein-and/or polymer-coated bubbles, microbubbles and/or microspheres, microballoons, microcapsules, aerogels, clathrate bound vesicles, hexagonal H II phase structures, and the like. The vesicles may also comprise a targeting ligand, if desired.

"Lipid vesicle", "polymer vesicle" and "protein vesicle" refer respectively to vesicles formulated from one or more lipids, polymers and proteins.

"Liposome" refers to a generally spherical or spheroidal cluster or aggregate of amphipathic compounds, including lipid compounds, typically in the form of one or more concentric layers, for example, monolayers or bilayers. They may also be referred to herein as lipid vesicles. The liposomes may be formulated, for example, from ionic lipids and/or non-ionic lipids. Liposomes which are formulated from non-ionic lipids may also be referred to as "niosomes."

"Micelle" refers to colloidal entities formulated from lipids. In certain preferred embodiments, the micelles comprise a monolayer or hexagonal H2 phase configuration. In other preferred embodiments, the micelles may comprise a bilayer configuration.

"Aerogel" refers to generally spherical or spheroidal entities which are characterized by a plurality of small internal voids. The aerogels may be formulated from synthetic or semisynthetic materials (for example, a foam prepared from baking resorcinol and formaldehyde), as well as natural materials, such as polysaccharides or proteins.

"Clathrate" refers to a solid, semi-porous or porous particle which may be associated with vesicles. In preferred form, the clathrates may form a cage-like structure containing cavities which comprise the vesicles. One or more vesicles may be bound to the clathrate. A stabilizing material may, if desired, be associated with the clathrate to promote the association of the vesicle with the clathrate. Suitable materials from which clathrates may be formulated include, for example, porous apatites, such as calcium hydroxyapatite, and precipitates of polymers and metal ions, such as alginic acid precipitated with calcium salts.

"Emulsion" refers to a mixture of two or more generally immiscible liquids and is generally in the form of a colloid. The liquids may be homogeneously or heterogeneously dispersed throughout the emulsion. Alternatively, the liquids may be aggregated in the form of, for example, clusters or layers, including mono- or bilayers.

"Suspension" or "dispersion" refers to a mixture, preferably finely divided, of two or more phases (solid, liquid or gas), such as, for example, liquid in liquid, solid in liquid, liquid in gas, etc.) which can preferably remain stable for extended periods of time.

"Hexagonal H II phase structure" refers to a generally tubular aggregation of lipids, proteins, or polymers (especially lipids) in liquid media, for example, aqueous media, in which any hydrophilic portion(s) generally face inwardly in association with an aqueous liquid environment inside the tube. The hydrophobic portion(s) generally radiate outwardly and the complex assumes the shape of a hexagonal tube. A plurality of tubes is generally packed together in the hexagonal phase structure.

"Lipid" refers to a naturally-occurring, synthetic or semi-synthetic (*i.e.,* modified natural) compound which is generally amphipathic. Lipids typically comprise a hydrophilic component and a hydrophobic component. Exemplary lipids include, for example, fatty acids, neutral fats, phosphatides, oils, glycolipids, surface-active agents (surfactants), aliphatic alcohols, waxes, terpenes and steroids. The phrase semi-synthetic (or modified natural) denotes a natural compound that has been chemically modified in some fashion.

"Protein" refers to nitrogenous compounds comprising, and preferably consisting essentially of, more than about 100 $\alpha$-amino acid residues in peptide linkages. Included within the term "protein" are globular proteins such as albumins, globulins and histones, and fibrous proteins such as collagens, elastins and keratins. Also included within the term "protein" are "compound proteins," wherein a protein molecule is united with a nonprotein molecule, such as nucleoproteins, mucoproteins, lipoproteins and metalloproteins. The proteins may be naturally-occurring, synthetic or semi-synthetic.

"Peptide" refers to nitrogenous compounds which may contain from about 2 to about 100 amino acid residues.

"Polymer" or "polymeric" refers to molecules formed from the chemical union of two or more repeating units. Accordingly, included within the term "polymer" may be, for example, dimers, trimers and oligomers. The polymer may be synthetic, naturally-occurring or semisynthetic. In a preferred form, "polymer" refers to molecules which comprise 10 or more repeating units.

"Stabilizing material" or "stabilizing compound" refers to any material which may be employed as carriers for the compositions of the present invention, and which may be capable of improving the stability of compositions containing the gases, gaseous precursors, compounds to be delivered, targeting ligands, nuclear localization sequences, and/or other compounds described herein, to provide compositions in the form of, for example, mixtures, suspensions, emulsions, dispersions, vesicles, or the like. The improved stability involves, for example, the maintenance of a relatively balanced condition, and may be exemplified, for example, by increased resistance of the composition against destruction, decomposition, degradation, and the like. In the case of preferred embodiments involving vesicles filled, for example, with gases, gaseous precursors, liquids, and compounds to be delivered, the stabilizing compounds may serve to either form the vesicles or stabilize the vesicles, in either way serving to minimize or substantially (including completely) prevent the escape of gases, gaseous precursors, and compounds to be delivered from the vesicles until said release is desired. The term "substantially," as used in the present context of preventing escape of gases, gaseous precursors, and compounds to be delivered from the vesicles, means greater than about 50% is maintained entrapped in the vesicles until release is desired, and preferably greater than about 60%, more preferably greater than about 70%, even more preferably greater than about 80%, still even more preferably greater than about 90%, is maintained entrapped in the vesicles until release is desired. In particularly preferred embodiments, greater than about 95% of the gases, gaseous precursors, and compounds to be delivered are maintained entrapped until release is desired. The gases, gaseous precursors, liquids, and compounds to be delivered may also be completely maintained entrapped (*i.e.*, about 100% is maintained entrapped), until release is desired. Exemplary stabilizing materials include, for example, lipids, proteins, polymers, carbohydrates and surfactants. The resulting mixture, suspension, emulsion or the like may comprise walls (*i.e*., films, membranes and the like) around the compound to be delivered, gases and/or gaseous precursors, or may be substantially devoid of walls or membranes, if desired. The stabilizing may, if desired, form droplets. The stabilizing material may also comprise salts and/or sugars. In certain embodiments, the stabilizing materials may be substantially (including completely) cross-linked. The stabilizing material may be neutral, positively or negatively charged.

"Gas filled vesicle" refers to a vesicle having a gas encapsulated therein. "Gaseous precursor filled vesicle" refers to a vesicle having a gaseous precursor encapsulated therein. The vesicles may be minimally, partially, substantially, or completely filled with the gas and/or gaseous precursor. The term "substantially" as used in reference to the gas and/or gaseous precursor filled vesicles means that greater than about 30% of the internal void of the substantially filled vesicles comprises a gas and/or gaseous precursor. In certain embodiments, greater than about 40% of the internal void of the substantially filled vesicles comprises a gas and/or gaseous precursor, with greater than about 50% being more preferred. More preferably, greater than about 60% of the internal void of the substantially filled vesicles comprises a gas and/or gaseous precursor, with greater than about 70% or 75% being more preferred. Even more preferably, greater than about 80% of the internal void of the substantially filled vesicles comprises a gas and/or gaseous precursor, with greater than about 85% or 90% being still more preferred. In particularly preferred embodiments, greater than about 95% of the internal void of the vesicles comprises a gas and/or gaseous precursor, with about 100% being especially preferred. Alternatively, the vesicles may contain no or substantially no gas or gaseous precursor.

"Droplet" refers to a spherical or spheroidal entity which may be substantially liquid or which may comprise liquid and solid, solid and gas, liquid and gas, or liquid, solid and gas. Solid materials within a droplet may be, for example, particles, polymers, lipids, proteins, or surfactants.

"Detergent" refers to a surface-active agent (surfactant) which, when added to a suspending medium of colloidal particles, including, for example, certain of the lipid, polymer, protein, and/or vesicle compositions described herein, may promote uniform separation of particles. "Detergent" also refers to a surface-active agent (surfactant) which lowers the surface tension of water.

"Cross-link," "cross-linked" and "cross-linking" generally refer to the linking of two or more stabilizing materials, including lipid, protein, polymer, carbohydrate, surfactant stabilizing materials and/or bioactive agents, by one ore more bridges. The bridges may be composed of one or more elements, groups, or compounds, and generally serve to join an atom from a first stabilizing material molecule to an atom of a second stabilizing material molecule. The cross-link bridges may involve covalent and/or non-covalent associations. Any of a variety of elements, groups, and/or compounds may form the bridges in the cross-links, and the stabilizing materials may be cross-linked naturally or through synthetic means. For example, cross-linking may occur in nature in material formulated from peptide chains which are joined by disulfide bonds of cystine residues, as in keratins, insulins and other proteins. Alternatively, cross-linking may be effected by suitable chemical modification, such as, for example, by combining a compound, such as a stabilizing material, and a chemical substance that may serve as a cross-linking agent, which may cause to react by, for example,

exposure to heat, high-energy radiation, ultrasonic radiation and the like. Examples include cross-linking by sulfur to form disulfide linkages, cross-linking using organic peroxides, cross-linking of unsaturated materials by means of high-energy radiation, cross-linking with dimethylol carbamate, and the like. If desired, the stabilizing compounds and/or bioactive agents may be substantially cross-linked. The term "substantially" means that greater than about 50% of the stabilizing compounds contain cross-linking bridges. If desired, greater than about 60%, 70%, 80%, 90%, 95% or even 100% of the stabilizing compounds contain such cross-linking bridges. Alternatively, the stabilizing materials may be non-cross-linked, i.e., such that greater than about 50% of the stabilizing compounds are devoid of cross-linking bridges, and if desired, greater than about 60%, 70%, 80%, 90%, 95% or even 100% of the stabilizing compounds are devoid of cross-linking bridges.

"Vesicle stability" refers to the ability of vesicles to retain the gas, gaseous precursor and/or other bioactive agents entrapped therein after being exposed, for about one minute, to a pressure of about 100 millimeters (mm) of mercury (Hg). Vesicle stability is measured in percent (%), this being the fraction of the amount of gas which is originally entrapped in the vesicle and which is retained after release of the pressure. Vesicle stability also includes "vesicle resilience" which is the ability of a vesicle to return to its original size after release of the pressure.

"Covalent association" refers to an intermolecular association or bond which involves the sharing of electrons in the bonding orbitals of two atoms.

"Non-covalent association" refers to intermolecular interaction among two or more separate molecules which does not involve a covalent bond. Intermolecular interaction is dependent upon a variety of factors, including, for example, the polarity of the involved molecules, and the charge (positive or negative), if any, of the involved molecules. Non-covalent associations are selected from ionic interactions, dipole-dipole interactions, van der Waal's forces, and combinations thereof.

"Ionic interaction" or "electrostatic interaction" refers to intermolecular interaction among two or more molecules, each of which is positively or negatively charged. Thus, for example, "ionic interaction" or "electrostatic interaction" refers to the attraction between a first, positively charged molecule and a second, negatively charged molecule. Ionic or electrostatic interactions include, for example, the attraction between a negatively charged stabilizing material, for example, genetic material, and a positively charged lipid, for example, a cationic lipid, such as lauryltrimethylammonium bromide.

"Dipole-dipole interaction" refers generally to the attraction which can occur among two or more polar molecules. Thus, "dipole-dipole interaction" refers to the attraction of the uncharged, partial positive end of a first polar molecule, commonly designated as $\delta^+$, to the uncharged, partial negative end of a second polar molecule, commonly designated as $\delta^-$. Dipole-dipole interactions are exemplified by the attraction between the electropositive head group, for example, the choline head group, of phosphatidylcholine and an electronegative atom, for example, a heteroatom, such as oxygen, nitrogen or sulphur, which is present in a stabilizing material, such as a polysaccharide. "Dipole-dipole interaction" also refers to intermolecular hydrogen bonding in which a hydrogen atom serves as a bridge between electronegative atoms on separate molecules and in which a hydrogen atom is held to a first molecule by a covalent bond and to a second molecule by electrostatic forces.

"Van der Waal's forces" refers to the attractive forces between non-polar molecules that are accounted for by quantum mechanics. Van der Waal's forces are generally associated with momentary dipole moments which are induced by neighboring molecules and which involve changes in electron distribution.

"Hydrogen bond" refers to an attractive force, or bridge, which may occur between a hydrogen atom which is bonded covalently to an electronegative atom, for example, oxygen, sulfur, or nitrogen, and another electronegative atom. The hydrogen bond may occur between a hydrogen atom in a first molecule and an electronegative atom in a second molecule (intermolecular hydrogen bonding). Also, the hydrogen bond may occur between a hydrogen atom and an electronegative atom which are both contained in a single molecule (intramolecular hydrogen bonding).

"Hydrophilic interaction" refers to molecules or portions of molecules which may substantially bind with, absorb and/or dissolve in water. This may result in swelling and/or the formation of reversible gels.

"Hydrophobic interaction" refers to molecules or portions of molecules which do not substantially bind with, absorb and/or dissolve in water.

"Patient" refers to animals, including mammals, preferably humans.

"Region of a patient" refers to a particular area or portion of the patient and in some instances to regions throughout the entire patient. Exemplary of such regions are the gastrointestinal region, the cardiovascular region (including myocardial tissue), the renal region as well as other bodily regions, tissues, lymphocytes, receptors, organs and the like, including the vasculature and circulatory system, and as well as diseased tissue. "Region of a patient" includes, for example, regions to be treated with a compound to be delivered. The "region of a patient" is preferably internal, although, if desired, it may be external.

"Region to be targeted" or "targeted region" refer to a region of a patient where delivery of a compound to be delivered is desired.

"Tissue" refers generally to specialized cells which may perform a particular function. The term "tissue" may

refer to an individual cell or a plurality or aggregate of cells, for example, membranes, blood or organs. The term "tissue" also includes reference to an abnormal cell or a plurality of abnormal cells. Exemplary tissues include lymph nodes and other tissues which may be involved in inflammation.

"Intracellular" or "intracellularly" refers to the area within the plasma membrane of a cell, including the protoplasm, cytoplasm and/or nucleoplasm. "Intracellular delivery" refers to the delivery of a bioactive agent, such as a targeting ligand and/or the compounds to be delivered, into the area within the plasma membrane of a cell.

"Cell" and "host cell" refer to prokaryotic cells and eukaryotic cells, including plant cells, animal cells, cells of unicellular organisms, cells of multicellular organisms, etc. Especially preferred are animal cells, more preferably mammalian cells and most especially human cells, including but not limited to living cells, tissues, and organs. Eukaryotic cells are cells of higher organisms in which genetic material is enclosed by a nuclear membrane. Prokaryotic cells are cells of lower organisms that lack a well defined nucleus and contain genetic material that is not enclosed within a membrane of its own. The cells may be present *in vivo* or *in vitro* (*e.g.* in cell culture).

"Biocompatible" refers to materials which are generally not injurious to biological functions and which will not result in any degree of unacceptable toxicity, including allergenic responses and disease states.

"Alkyl" refers to liner, branched or cyclic hydrocarbon groups. Preferably, the alkyl is a linear or branched hydrocarbon group, more preferably a linear hydrocarbon group. Exemplary linear and branched alkyl groups include, for example, methyl, ethyl, n-propyl, i-propyl, n-butyl, t-butyl, n-pentyl, hexyl, heptyl, octyl, nonyl, and decyl groups. Exemplary cyclic hydrocarbon groups (cycloalkyl groups) include, for example, cyclopentyl, cyclohexyl and cycloheptyl groups. "Fluoroalkyl" refers to an alkyl group which is substituted with one or more fluorine atoms, including, for example, fluoroalkyl groups of the formula $CF_3(CF_2)_n(CH_2)_m$-, wherein each of m and n is independently an integer from 0 to about 22. Exemplary fluoroalkyl groups include perfluoromethyl, perfluoroethyl, perfluoropropyl, perfluorobutyl, perfluorocyclobutyl, perfluoropentyl, perfluorohexyl, perfluoroheptyl, perfluorooctyl, perfluorononyl, perfluorodecyl, perfluoroundecyl and perfluorododecyl.

"Acyl" refers to an alkyl-CO- group wherein alkyl is as previously described. Preferred acyl groups comprise alkyl of 1 to about 30 carbon atoms. Exemplary acyl groups include acetyl, propanoyl, 2-methylpropanoyl, butanoyl and palmitoyl. "Fluoroacyl" refers to an acyl group that is substituted with one or more fluorine atoms, up to and including perfluorinated acyl groups.

"Aryl" refers to an aromatic carbocyclic radical containing about 6 to about 10 carbon atoms. The aryl group may be optionally substituted with one or more aryl group substituents which may be the same or different, where "aryl group substituent" includes alkyl, alkenyl, alkynyl, aryl, aralkyl, hydroxy, alkoxy, aryloxy, aralkoxy, carboxy, aroyl, halo, nitro, trihalomethyl, cyano, alkoxycarbonyl, aryloxycarbonyl, aralkoxycarbonyl, acyloxy, acylamino, aroylamino, carbamoyl, alkylcarbamoyl, dialkylcarbamoyl, arylthio, alkylthio, alkylene and -NRR', where R and R' are each independently hydrogen, alkyl, aryl and aralkyl. Exemplary aryl groups include substituted or unsubstituted phenyl and substituted or unsubstituted naphthyl.

"Alkylaryl" refers to alkyl-aryl groups (*e.g.*, $CH_3$-($C_6H_4$)-) and aryl-alkyl groups (*e.g.*, ($C_6H_5$)-$CH_2$-) where aryl and alkyl are as previously described. Exemplary alkylaryl groups include benzyl, phenylethyl and naphthyl-methyl. "Fluoroalkylaryl" refers to an alkylaryl group that is substituted with one or more fluorine atoms, up to and including perfluorinated alkylaryl groups.

"Alkylene" refers to a straight or branched bivalent aliphatic hydrocarbon group having from 1 to about 30 carbon atoms. The alkylene group may be straight, branched or cyclic. The alkylene group may be also optionally unsaturated and/or substituted with one or more "alkyl group substituents," including halogen atoms, such as fluorine atoms. There may be optionally inserted along the alkylene group one or more oxygen, sulphur or substituted or unsubstituted nitrogen atoms, wherein the nitrogen substituent is alkyl as previously described. Exemplary alkylene groups include methylene (-$CH_2$-), ethylene (-$CH_2CH_2$-), propylene (-$(CH_2)_3$-), cyclohexylene (-$C_6H_{10}$-), -CH=CH-CH=CH-, -CH=CH-$CH_2$-, -$(CF_2)_n(CH_2)_m$-, wherein n is an integer from about 1 to about 22 and m is an integer from 0 to about 22, -$(CH_2)_n$-N(R)-$(CH_2)_m$-, wherein each of m and n is independently an integer from 0 to about 30 and R is hydrogen or alkyl, methylenedioxy (-O-$CH_2$-O-) and ethylenedioxy (-O-$(CH_2)_2$-O-). It is preferred that the alkylene group has about 2 to about 3 carbon atoms.

"Halo," "halide" or "halogen" refers to chlorine, fluorine, bromine or iodine atoms.

"Genetic material" refers generally to nucleosides, nucleotides, polynucleosides and polynucleotides, including deoxyribonucleic acid (DNA) and ribonucleic acid (RNA). The genetic material may be made by synthetic chemical methodology known to one of ordinary skill in the art, or by the use of recombinant technology, or by a combination thereof. The DNA and RNA may optionally comprise unnatural nucleotides and may be single or double stranded. "Genetic material" also refers to sense and anti-sense DNA and RNA, tat is, a nucleotide sequence which is complementary to a specific sequence of nucleotides in DNA and/or RNA. "Genetic material" also includes complexes associated with DNA or RNA including, for example, ribozymes. The genetic material may be included in the compositions alone or in combination with a condensing agent.

"Condensing agent" refers to any material whose presence may cause genetic material, such as DNA, to

occupy a reduced spatial area relative to the spatial area that the genetic material may occupy in the absence of the condensing agent. The condensing agents are typically positively charged and, therefore, may repel polyanionic materials as exemplified, for example, by the sugar phosphate backbone of DNA. It is believed that this charge repulsion contributes to the ability of the condensing agents to cause genetic material to "condense", *i.e.*, to occupy reduced spatial areas. For example, polylysine, which is an exemplary polycationic condensing agent, may act to crosslink the polyanionic nature of DNA which normally accounts for intrastrand charge repulsion. Other exemplary condensing agents, in addition to polylysine, include, for example, spermine and spermidine, although other materials, including other polycation materials, would be apparent to one of ordinary skill in the art once armed with the teachings of the present disclosure. Preferred condensing agents include cationic lipids, especially fluorinated cationic lipids. The condensing agent may be incorporated in the present compositions, for example, covalently or non-covalently. It is also contemplated that organic halides, such as fluorinated compounds, particularly perfluorocarbons, which are a preferred component of the present compositions, may enhance the ability of condensing agents to condense genetic material by providing a non-polar microenvironment for the bases.

"Pharmaceutical agent" or "drug" refers to any therapeutic or prophylactic agent which may be used in the treatment (including the prevention, diagnosis, alleviation, or cure) of a malady, affliction, disease or injury in a patient, such as, for example, rheumatoid arthritis. Therapeutically useful peptides, polypeptides and polynucleotides may be included within the meaning of the term pharmaceutical or drug.

Methods of introducing compounds into a cell (also referred to variously herein as methods for delivering a compound into a cell, methods of intracellular delivery, methods of promoting, effecting, facilitating or enhancing the uptake of a compound into a cell, and the like) include transfection, which refers to the introduction of genetic material, *i.e.*, a nucleotide sequence (*e.g.*, DNA or RNA) into a host cell. Transfection may also sometimes be referred to as transformation. DNA (or RNA) which is new to the cell into which it is incorporated is typically referred to as heterologous DNA (or RNA) or exogenous DNA (or RNA). Some bacterial species may take up exogenous DNA without discriminating between uptake of DNA from a similar or same species or from a completely different species or organism. Exogenous DNA may also be taken up by cells, but may or may not be incorporated into nuclear material in a hereditable manner. The objective of transfection of a host cell may be to effect expression of one or more carefully selected sequences.

"Expression" and "gene expression" refer to the transcription and/or translation of a nucleic acid sequence resulting in the production of an amino acid, peptide and/or protein. The nucleic acid sequence may or may not be incorporated into the genetic material of the host cell. For example, the nucleic acid sequence may be incorporated into the genome of a host cell or may simply be introduced into the cell without incorporation into the genome.

Gene expression, upon administration of a composition of the present invention, may be effected (including, for example, obtained, promoted, facilitated or enhanced), and preferably may be enhanced in that the expression of the nucleic acid sequences as compared to conventional transfection techniques including, for example, calcium phosphate precipitation, viral vectors, microinjection, shock wave such as, for example, lithotripsy, and electroporation, may be increased. Methods of measuring enhanced gene expression will be known to skilled artisans once armed with the present disclosure and include, for example, enzyme-linked immunosorbent assay (ELISA), as well as other methods, including those disclosed in Sambrook, *et al*., *Molecular Cloning: A Laboratory Manual*, 2nd ed., Cold Spring Harbor Laboratory Ness, Cold Spring Harbor, NY (1989), the disclosures of which are hereby incorporated herein by reference in their entirety. Thus, as a result of the methods of the present invention, a product (*e.g.*, a protein) may be produced. In addition, the methods described herein may be employed to prevent production of a product (for example, as a result of an antisense sequence being delivered into a cell).

Without intending to be bound by any theory or theories of operation, it is believed that delivery of nucleic acid sequences and other compounds in accordance with the methods of the present invention may induce a cell to take up the compound to be delivered thereto. Included within the definition of delivery of a compound into a cell in accordance with the methods of the present invention are active and passive mechanisms of cellular uptake. Ion channels and other means of transport utilized by cells to incorporate extracellular materials, including compounds to be delivered thereto, into the intracellular milieu are encompassed by the present invention.

"Nucleotide sequence and nucleic acid sequence" refer to single and double stranded DNA and RNA sequences, including and not limited to oligonucleotide sequences of about 100 kb to about 1,000,000 kb (including whole chromosomes), preferably of about 4 kb to about 6 kb, more preferably about 1,000 nucleotides in length, more preferably about 500 nucleotides in length, more preferably about 250 nucleotides in length, more preferably about 100 nucleotides in length, more preferably about 50 nucleotides in length, more preferably about 25 nucleotides in length, more preferably about 10 nucleotides in length, even more preferably about 3 to about 10 bp in length. Embodied by the term "nucleotide sequence" are all or part of a gene, at least a portion of a gene, a gene fragment, a sense sequence, an antisense sequence, an antigene nucleic acid, a phosphorothioate oligodeoxynucleotide, and an alteration, deletion, mismatch, transition, transversion, mutation, conservative substitution, and homolog of a sequence. The phrase "at least a portion of," and "all or part of," as used herein, means that the entire gene need not be represented by the

sequence so long as the portion of the gene represented is effective to block or exhibit, depending on the type of sequence used, gene expression. The sequences may be incorporated into an expression vector such as, and not limited to, a plasmid, phagemid, cosmid, yeast artificial chromosome (YAC), virus (e.g., adenovirus, vaccinia virus, retrovirus), and defective virus (also known as a "helper virus"). The nucleotide sequence may also be administered naked, that is without an expression vector.

A "precursor" to a targeting ligand refers to any material or substance which may be convened to a targeting ligand. Such conversion may involve, for example, anchoring a precursor to a targeting ligand. Exemplary targeting precursor moieties include, for example, maleimide groups, disulfide groups, such as ortho-pyridyl disulfide, vinylsulfone groups, azide groups, and α-iodo acetyl groups;

"Receptor" refers to a molecular structure within a cell or on the surface of a cell which is generally characterized by the selective binding of a specific substance. Exemplary receptors include cell-surface receptors for peptide hormones, neurotransmitters, antigens, complement fragments, immunoglobulins and cytoplasmic receptors for steroid hormones.

"In combination with" refers to the incorporation of compounds to be delivered, targeting ligands, and nuclear localization sequences in a composition of the present invention, optionally including a carrier, such as in the form of emulsions, suspensions and vesicles. The compound to be delivered, targeting ligand, and nuclear localization sequence can be combined with the carrier in any of a variety of ways. For example, one or more of the compound to be delivered, targeting ligand, and nuclear localization sequence may be associated covalently and/or non-covalently with the carrier (*e.g*., stabilizing materials or compounds). In the case of vesicles, targeting ligands, for example, may be integrated within the layer(s) or wall(s) of the vesicle present on the external surface, the compound to be delivered may be entrapped within the internal void of the vesicle, and the nuclear localization sequence may be integrated within the layer(s) or wall(s) of the vesicle on the internal surface, for example, by being interspersed among stabilizing materials which form or are contained within the vesicle layer(s) or wall(s). In addition, it is contemplated, for example, that the compound to be delivered (such as, for example, a bioactive agent), nuclear localization sequence and/or targeting ligand may be located on the surface of a vesicle or non-vesicular carrier. Also, the compound to be delivered, targeting ligand, and nuclear localization sequence may be concurrently entrapped within the internal void of the vesicle and/or integrated within the layer(s) or wall(s) of the vesicles and/or located on the surface of a vesicle or non-vesicular carrier. In any case, the compound to be delivered, targeting ligand, and nuclear localization sequence may interact chemically with the walls of the vesicles, including, for example, the inner and/or outer surfaces of the vesicle and may remain substantially adhered thereto. Such interaction may take the form of, for example, non-covalent association or bonding, ionic interactions, electrostatic interactions, dipole-dipole interactions, hydrogen bonding, van der Waal's forces, covalent association or bonding, cross-linking or any other interaction, as will be readily apparent to one skilled in the art, in view of the present disclosure. In certain embodiments, the interaction may result in the stabilization of the vesicle. The compound to be delivered may also interact with the inner or outer surface of the vesicle or the non-vesicular carrier material in a limited manner. Such limited interaction would permit migration of the compound to be delivered, for example, from the surface of a first vesicle to the surface of a second vesicle, or from the surface of a first non-vesicular stabilizing material to a second non-vesicular carrier material. Alternatively, such limited interaction may permit migration of the compound to be delivered, for example, from within the walls of a vesicle and/or non-vesicular carrier material to the surface of a vesicle and/or non-vesicular carrier material, and vice versa, or from inside a vesicle or non-vesicular carrier material to within the walls of a vesicle or non-vesicular carrier material and vice versa.

The present invention is directed, in part, to compositions for delivering one or more compounds into a cell. Embodiments are provided which comprise compositions comprising, in combination with the compound or compounds to be delivered, an organic halide, a targeting ligand which may target tissues, cells and/or receptors *in vivo*, and a nuclear localization sequence. In preferred embodiments, the compositions may further comprise a carrier material. Also in certain preferred embodiments, the compositions may further comprise a fusion peptide.

The following is a detailed discussion of the various components which may be included in the compositions of the present invention.

***Organic Halides***

There are a wide variety of organic halides which may be employed in the compositions and methods of the present invention. Where the organic halide is a carbon based halide compound, the organic halide preferably contains from 1 to about 30 carbon atoms, preferably from 1 to about 24 carbon atoms, more preferably from 1 to about 12 carbon atoms, even more preferably from about 5 to about 12 carbon atoms, and still more preferably from about 6 to about 10 carbon atoms. Thus, there may be in the organic halide compound, for example, 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 carbon atoms. Sulfur or selenium based halide compounds, such as sulfur hexafluoride and selenium hexafluoride, are also within the scope of the invention described herein and the phrase "organic halide" as used herein. The organic halides contemplated herein may also, for example,

have carbon atoms interrupted by one or more heteroatoms, such as -O- heteroatoms (as in the case of, for example, ether compounds) or they may have other substituents, such as amines, etc. Preferred organic halides of the present invention are perhalogenated compounds, especially perfluorinated compounds such as perfluorocarbons and perfluoroethers.

Table 1 lists representative organic halides useful in the present invention. Other organic halides suitable for use in the present invention will be readily apparent to one skilled in the art, once armed with the present disclosure. All such organic halides are intended to fall within the scope of the term organic halide, as used herein.

Table 1

Organic Halides

| Compound | Boiling Point (°C) |
|---|---|
| 1. Mixed-halogenated Compounds | |
| 1-bromo-nonafluorobutane | 43 |
| perfluorooctyliodide | 160-161 |
| perfluorooctylbromide | 142 |
| 1-chloro-1-fluoro-1-bromomethane | 38 |
| 1,1,1-trichloro-2,2,2-trifluoroethane | 45.7 |
| 1,2-dichloro-2,2-difluoroethane | 46 |
| 1,1-dichloro-1,2-difluoroethane | 45 |
| 1,2-dichloro-1,1,3-trifluoropropane | 50.4 |
| 1-bromoperfluorobutane | 43 |
| 1-bromo-2,4-difluorobenzene | 44 |
| 2-iodo-1,1,1-trifluoroethane | 53 |
| 5-bromovaleryl chloride | 43 |
| 1,3-dichlorotetrafluoroacetone | 43 |
| bromine pentafluoride | 40.3 |
| 1-bromo-1,1,2,3,3,3-hexafluoropropane | 35.5 |
| 2-chloro 1,1,1,4,4,4-hexafluoro-2-butene | 33 |

| | |
|---|---|
| 2-chloropentafluoro-1,3-butadiene | 37 |
| iodotrifluoroethylene | 30 |
| 1,1,2-trifluoro-2-chloroethane | 30 |
| 1,2-difluorochloroethane | 35.5 |
| 1,1-difluoro-2-chloroethane | 35.1 |
| 1,1-dichlorofluoroethane | 31.8 |
| heptafluoro-2-iodopropane | 39 |
| bromotrifluoroethane | -57.8 |
| chlorotrifluoromethane | -81.5 |
| dichlorodifluoromethane | -29.8 |
| dibromofluoromethane | 23 |
| chloropentafluoroethane | -38.7 |
| bromochlorodifluoromethane | -4 |
| dichloro-1,1,2,2-tetrafluoroethane | 3.1-3.6 |

2. Fluorinated Compounds

| | |
|---|---|
| 1,1,1,3,3-pentafluoropentane | 40 |
| perfluorotributylamine | 178 |
| perfluorotripropylamine | 130 |
| 3-fluorobenzaldehyde | 56 |
| 2-fluoro-5-nitrotoluene | 53 |
| 3-fluorostyrene | 40 |
| 3,5-difluoroaniline | 40 |
| 2,2,2-trifluoroethylacrylate | 45 |
| 3-(trifluoromethoxy)-acetophenone | 49 |
| 1,1,2,2,3,3,4,4-octafluorobutane | 44.8 |
| 1,1,1,3,3-pentafluorobutane | 40 |
| 1-fluorobutane | 32.5 |
| 1,1,2,2,3,3,4,4-octafluorobutane | 44.8 |
| 1,1,1,3,3-pentafluorobutane | 40 |
| perfluoro-4 methylquinolizidine | 149 |
| perfluoro-N-methyl-decahydroquinone | 150-155 |

| | |
|---|---|
| perfluoro-N-methyl-decahydroisoquinone | 150-155 |
| perfluoro-N-cyclohexyl-pyrrolidine | 145-152 |
| tetradecaperfluoroheptane | 76 |
| dodecaperfluorocyclohexane | 52 |

3. Perfluorinated Compounds

a. Perfluorocarbons

| | |
|---|---|
| perfluoromethane | -129 |
| perfluoroethane | -78.3 |
| perfluoropropane | -36 |
| perfluorobutane | -2 |
| perfluoropentane | 29.5 |
| perfluorohexane | 59-60 |
| perfluoroheptane | 81 |
| perfluorooctane | 102 |
| perfluorononane | 125 |
| perfluorodecane | ~ 143 |
| perfluorododecane | melting pt 75-77 |
| perfluoro-2-methyl-2-pentene | 51 |
| perfluorocyclohexane | 52 |
| perfluorodecalin | 142 |
| perfluorododecalin | --- |
| perfluoropropylene | -28 |
| perfluorocyclobutane | -6 |
| perfluoro-2-butyne | -25 |
| perfluoro-2-butene | 1.2 |
| perfluorobuta-1,3-diene | 6 |

b. Perfluoroether Compounds

| | |
|---|---|
| perfluorobutylethyl ether | 60 |
| bis(perfluoroisopropyl) ether | 54 |
| bis(perfluoropropyl) ether | 59 |

| | |
|---|---|
| perfluorotetrahydropyran | 34 |
| perfluoromethyl tetrahydrofuran | 27 |
| perfluoro-t-butyl methyl ether | 36 |
| perfluoroisobutyl methyl ether | --- |
| perfluoro-n-butyl methyl ether | 35.4 |
| perfluoroisopropyl ethyl ether | --- |
| perfluoro-n-propyl ethyl ether | 23.3 |
| perfluorocyclobutyl methyl ether | --- |
| perfluorocyclopropyl ethyl ether | --- |
| perfluoroisopropyl methyl ether | 36 |
| perfluoro-n-propyl methyl ether | --- |
| perflourodiethyl ether | 3-4.5 |
| perfluorocyclopropyl methyl ether | --- |
| perfluoromethyl ethyl ether | -23 |
| perfluorodimethyl ether | -59 |
| c. Other | |
| sulfur hexafluoride | m.p. -50.5, sublimes -63.8 |
| selenium hexafluoride | m.p. -34.6 sublimes -46.6 |

Preferred organic halides which may be employed in the methods and compositions of the present invention include, for example, 1-bromo-nonafluorobutane, 1,1,1,3,3-pentafluoropentane, perfluorohexane, perfluorocyclohexane, 1-bromo-1,1,2,3,3,3-hexafluoropropane, heptafluoro-2-iodopropane, 1,1,2,2,3,3,4,4-octafluorobutane, 1-fluorobutane, tetradecaperfluoroheptane and dodecaperfluorocylclohexane. Particularly preferred are perfluorohexane (especially n-perfluorohexane) and perfluorocyclohexane. A wide variety of other organic halides useful in the present invention will be readily apparent to those of skill in the art once armed with the present disclosure. Suitable additional organic halides include those disclosed, for example, in Long, Jr., U.S. Patent Nos. 4,987,154, 4,927,623, and 4,865,836, the disclosures of each of which are hereby incorporated herein by reference in their entirety.

The amount of organic halide employed in the present invention may vary, as one skilled in the art will recognize, once armed with the present disclosure, and may be dependent on such factors as the particular organic halide employed, the type and nature of the compound to be delivered, the other components employed in the composition, the age, weight, cells or patient (animal) to be treated, the particular diagnostic, therapeutic or other application intended (including the disease state, if any, to be treated), and the like. Typically, lower amounts may be used initially and then increased until the desired delivery effect is achieved. Representative amounts are exemplified in the examples set forth herein. Of course, higher or lower amounts may be employed, as will be recognized by the skilled artisan.

***Carriers***

As noted above, if desired, the compositions of the present invention may further comprise a carrier. The carrier employed may comprise a wide variety of materials. Carriers may include, for example, lipids, polymers, proteins, surfactants, inorganic compounds, metal ions, and the like, alone or in combination with water and/or another solvent, including an organic solvent. Alternatively, the carrier may simply comprise water and/or a solvent. The lipids, proteins, and polymers, for example, may be in liquid form or solid form, the latter including, for example, particles, fibers, sheets, layers, and the like, or they may take the form of a vesicle or other stable, organized form, which may include, for example, such forms commonly referred to as, for example, liposomes, micelles, bubbles, microbubbles, microspheres, lipid-, polymer-, and/or protein-coated bubbles, microbubbles and/or microspheres, microballoons, aerogels, hydrogels, clathrates, hexagonal HII phase structures, and the like. The internal void of the vesicle or other stable form may, for example, be filled with a material, such as a liquid (including, for example, a gaseous precursor), gas, solid, or solute material, or any combination thereof, including, for example, the compound to be delivered, the organic halide, targeting ligand, and/or nuclear localization sequence. Typically, the carrier is provided as an aqueous milieu, such as water, saline (including phosphate buffered saline), and the like, with or without other carrier components, although other non-aqueous solvents may also be employed, if desired. The carrier may comprise a mixture in the form of an emulsion, suspension, dispersion, solution, and the like. Lipid (including oil) in water emulsions are especially preferred. The carrier may also include buffers.

Preferably, the compositions of the invention comprise carriers in the form of lipids. A wide variety of lipids may be used as stabilizing materials in the present invention. The lipids may be of either natural, synthetic or semi-synthetic origin, including for example, fatty acids, fluorinated lipids, neutral fats, phosphatides, phospholipids, oils, fluorinated oils, glycolipids, lysolipids, surface active agents (surfactants and fluorosurfactants), aliphatic alcohols, waxes, terpenes and steroids. Exemplary lipids include, for example, phosphocholines, such as those associated with platelet activation factors (PAF) (Avanti Polar Lipids, Alabaster, AL), including 1-alkyl-2-acetoyl-sn-glycero 3-phosphocholines, and 1 -alkyl-2-hydroxy-sn-glycero 3-phosphocholines, which target blood clots; phosphatidylcholine with both saturated and unsaturated lipids, including dioleoylphosphatidylcholine; dimyristoylphosphatidylcholine; dipentadecanoylphosphatidylcholine; dilauroylphosphatidylcholine; dipalmitoylphosphatidylcholine (DPPC); distearoylphosphatidylcholine (DSPC); and diarachidonylphosphatidylcholine (DAPC); phosphatidylethanolamines, such as dioleoylphosphatidylethanolamine, dipalmitoylphosphatidylethanolamine (DPPE) and distearoylphosphatidylethanolamine (DSPE); phosphatidylserine; phosphatidylglycerols, including distearoylphosphatidylglycerol (DSPG); phosphatidylinositol; sphingolipids such as sphingomyelin; glycolipids such as ganglioside GM1 and GM2; glucolipids; sulfatides; glycosphingolipids; phosphatidic acids, such as dipalmitoylphosphatidic acid (DPPA) and distearoylphosphatidic acid (DSPA); palmitic acid; stearic acid; arachidonic acid; oleic acid; lipids bearing polymers, such as chitin, hyaluronic acid, polyvinylpyrrolidone or polyethylene glycol (PEG), also referred to as "pegylated lipids" with preferred lipid bearing polymers including DPPE-PEG (DPPE-PEG), which refers to the lipid DPPE having a PEG polymer attached thereto, including, for example, DPPE-PEG5000, which refers to DPPE having attached thereto a PEG polymer having a mean average molecular weight of about 5000; lipids bearing sulfonated mono-, di-, oligo- or polysaccharides; cholesterol, cholesterol sulfate and cholesterol hemisuccinate; tocopherol hemisuccinate; lipids with ether and ester-linked fatty acids; polymerized lipids (a wide variety of which are well known in the art); diacetyl phosphate; dicetyl phosphate; stearylamine; cardiolipin; phospholipids with short chain fatty acids of about 6 to about 8 carbons in length; synthetic phospholipids with asymmetric acyl chains, such as, for example, one acyl chain of about 6 carbons and another acyl chain of about 12 carbons; ceramides; non-ionic liposomes including niosomes such as polyoxyalkylene (*e.g*., polyoxyethylene) fatty acid esters, polyoxyalkylene (*e.g*., polyoxyethylene) fatty alcohols, polyoxyalkylene (*e.g*., polyoxyethylene) fatty alcohol ethers, polyoxyalkylene (*e.g*., polyoxyethylene) sorbitan fatty acid esters (such as, for example, the class of compounds referred to as TWEEN® including, for example, TWEEN® 20, TWEEN® 40 and TWEEN® 80, commercially available from ICI Americas, Inc., Wilmington, DE), glycerol polyethylene glycol oxystearate, glycerol polyethylene glycol ricinoleate, alkyloxylated (e.g., ethoxylated) soybean sterols, alkyloxylated (*e.g*., ethoxylated) castor oil, polyoxyethylene-polyoxypropylene polymers, and polyoxyalkylene (*e.g*., polyoxyethylene) fatty acid stearates; sterol aliphatic acid esters including cholesterol sulfate, cholesterol butyrate, cholesterol isobutyrate, cholesterol palmitate, cholesterol stearate, lanosterol acetate, ergosterol palmitate, and phytosterol n-butyrate; sterol esters of sugar acids including cholesterol glucuronide, lanosterol glucuronide, 7-dehydro-cholesterol glucuronide, ergosterol glucuronide, cholesterol gluconate, lanosterol gluconate, and ergosterol gluconate; esters of sugar acids and alcohols including lauryl glucuronide, stearoyl glucuronide, myristoyl glucuronide, lauryl gluconate, myristoyl gluconate, and stearoyl gluconate; esters of sugars and aliphatic acids including sucrose laurate, fructose laurate, sucrose palmitate, sucrose stearate, glucuronic acid, gluconic acid and polyuronic acid; saponins including sarsasapogenin, smilagenin, hederagenin, oleanolic acid, and digitoxigenin; glycerol dilaurate, glycerol trilaurate, glycerol dipalmitate, glycerol and glycerol esters including glycerol tripalmitate, glycerol distearate, glycerol tristearate, glycerol dimyristate, glycerol trimyristate; long chain alcohols including n-decyl alcohol, lauryl alcohol, myristyl alcohol, cetyl alcohol, and n-octadecyl alcohol; 6-(5-cholesten-3β-yloxy)- 1 -thio-β-D-galactopyranoside; digalactosyldiglyceride; 6-(5-cholesten-3 β-yloxy)hexyl-6-amino-6-deoxy- 1 -thio-β-D-galactopyranoside; 6-(5-cholesten-3 β-yloxy)hexyl-6-amino-6-deoxyl- 1 -thio-α-D-mannopyranoside; 12-(((7'-diethylaminocoumarin-3-yl)carbonyl)methylamino)-octadecanoic acid; N-[ 1 2-(((7'-diethylaminocoumarin-3-yl)carbonyl)methylamino)-octadecanoyl]-2-aminopalmitic acid; cholesteryl(4'-trimethylammonio)butanoate; N-succinyldioleoylphosphatidylethanolamine; 1,2-dioleoyl-sn-glycerol; 1,2-dipalmitoyl-sn-3-succinylglycerol; 1,3-dipalmitoyl-2-succinylglycerol; 1-hexadecyl-2-palmitoylglycerophosphoethanolamine and palmitoylhomocysteine, and/or any combinations thereof. In preferred embodiments, the stabilizing materials comprise phospholipids, including one or more of DPPC, DPPE, DPPA, DSPC, DSPE, DSPG, DSPA and DAPC.

Examples of polymerizable lipids which may be polymerized include lipids containing unsaturated lipophilic chains, such as alkenyl or alkynyl, containing up to about 50 carbon atoms; phospholipids such as phosphoglycerides and sphingolipids carrying polymerizable groups; and saturated and unsaturated fatty acid derivatives with hydroxyl groups, such as triglycerides of d-12-hydroxyoleic acid, including castor oil and ergot oil. Polymerization may be designed to include hydrophilic substituents such as carboxyl or hydroxyl groups, to enhance dispersability so that the backbone residue resulting from biodegradation is water soluble. Suitable polymerizable lipids are also described, for example, by Klaveness et al, U.S. Patent No. 5,536,490, the disclosures of which are hereby incorporated by reference herein in their entirety.

Suitable fluorinated lipids include, for example, compounds of the formula $C_nF_{2n+1}(CH_2)_mC(O)OOP(OO^-)O(CH_2)_wN^+(CH_3)_3C_nF_{2n+1}(CH_2)_mC(O)O$ where m is 0 to about 18, n is 1 to about 12; and w is 1 to about 8. Examples of and methods for the synthesis of these, as well as other fluorinated lipids useful in the present invention, are set forth in copending U.S. Application Serial No. 08/465,868, filed June 6, 1995; Reiss et al, U.S. Patent No. 5,344,930; Frezard et al., *Biochem Biophys Acta*, *1192*:61-70 (1994); and Frezard et al, *Art. Cells Blood Subs and Immob Biotech*., *22*:1403-1408 (1994), the disclosures of each of which are hereby incorporated herein by reference in their entirety. One specific example of a difluoroacylglycerylphosphatidylcholine, nonafluorinated diacylglycerylphosphatidylcholine, is represented by compound A, below. Once armed with the teachings of the present application, one skilled in the art will appreciate that analogous fluorinated derivatives of other common phospholipids (*e.g*., diacylphosphatidylserine, diacylphosphatidylethanolamine, diacylphosphatidylglycerol, diacylphosphatidylglycerol, and the like) as well as fluorinated derivatives of fatty acyl esters and free fatty acids may also function in accordance with the scope of the invention. Additionally lipid based and fluorinated (including perfluorinated) surfactants may be used as stabilizing materials in the present invention.

Exemplary polymerizable and/or fluorinated lipid compounds which may be utilized in the compositions of the present invention are illustrated below.

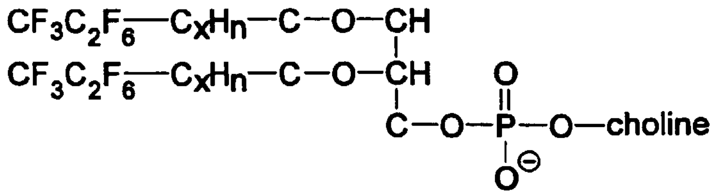

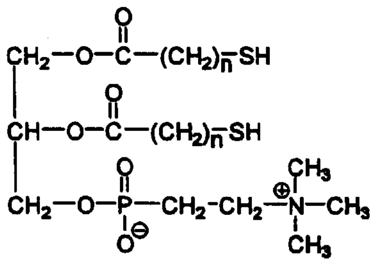

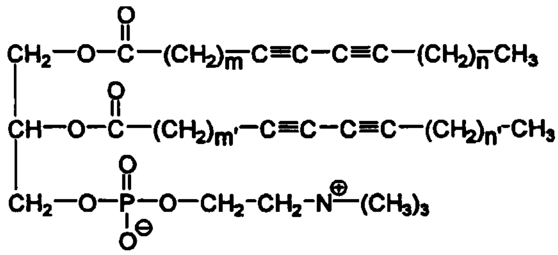

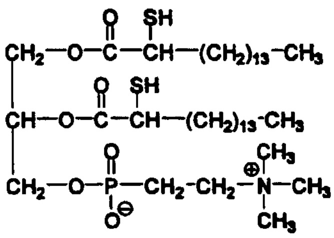

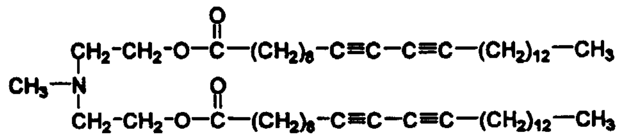

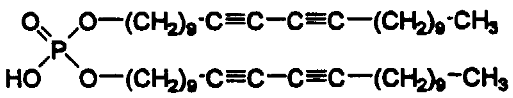

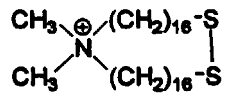

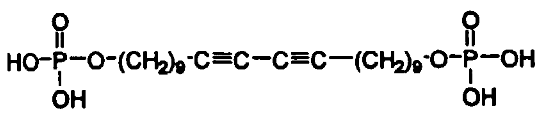

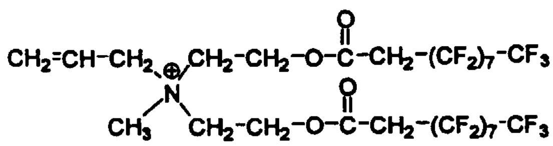

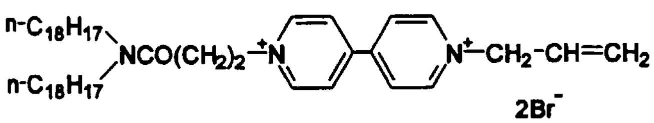

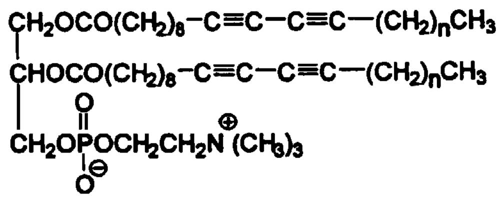

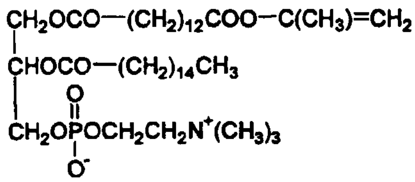

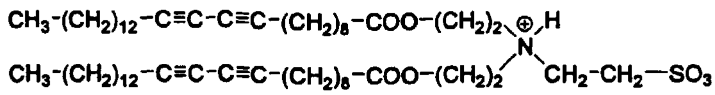

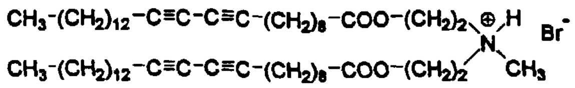

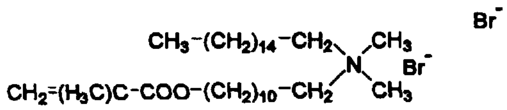

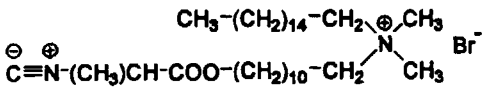

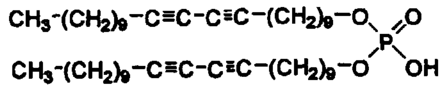

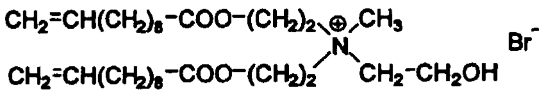

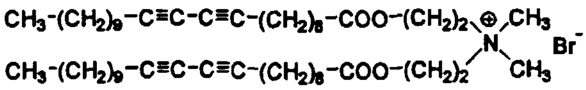

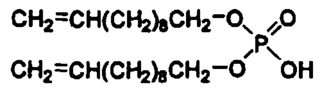

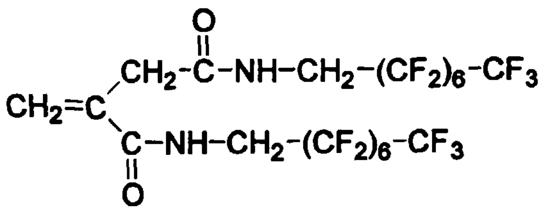

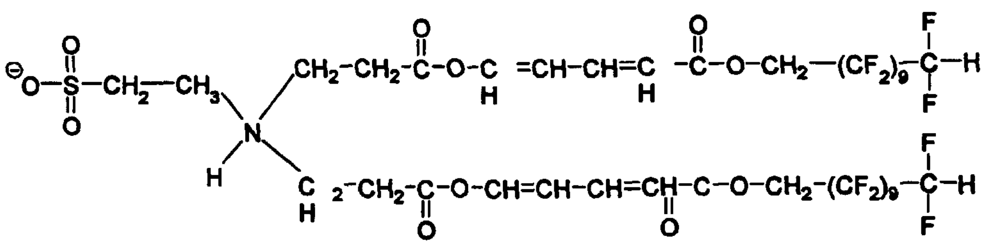

In formula A, above, x is an integer from about 8 to about 18, and n is 2x. Most preferably x is 12 and n is 24. In formulas B, C, K and L, above, m, n, m' and n' are, independently, an integer of from about 8 to about 18, preferably about 10 to about 14.

Other lipids which may be employed in the present compositions include, for example,

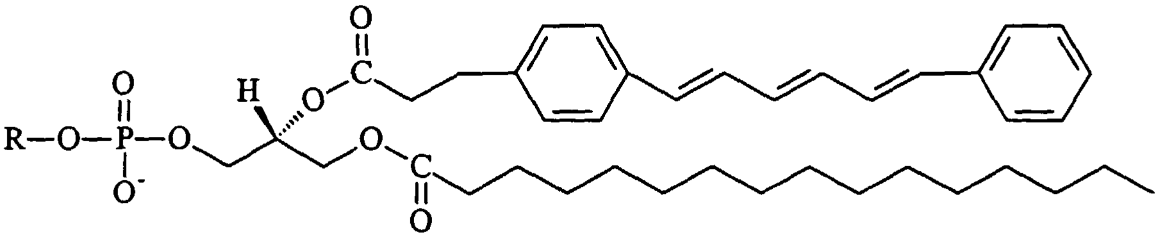

where R is choline,

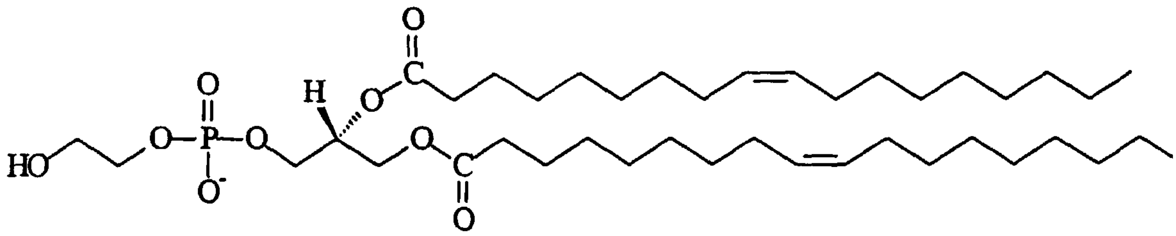

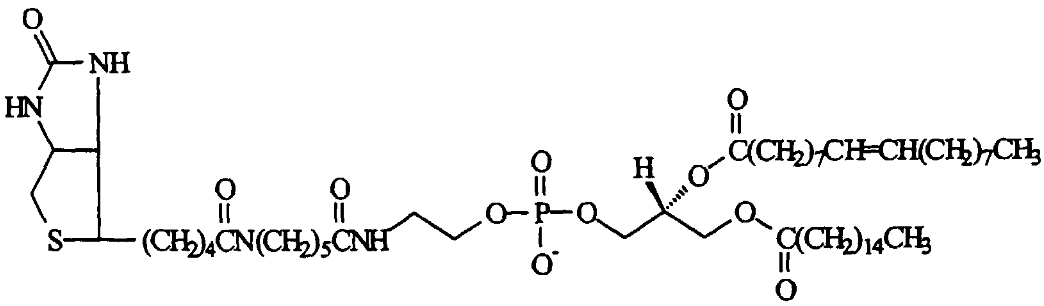

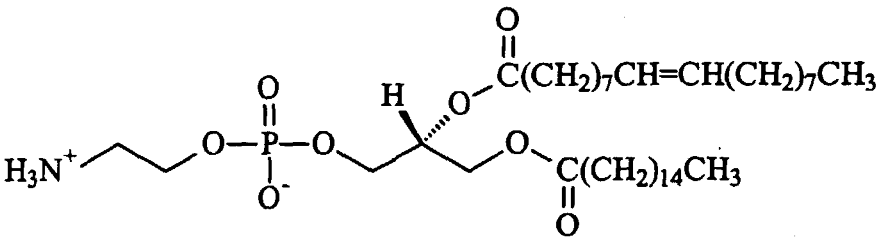

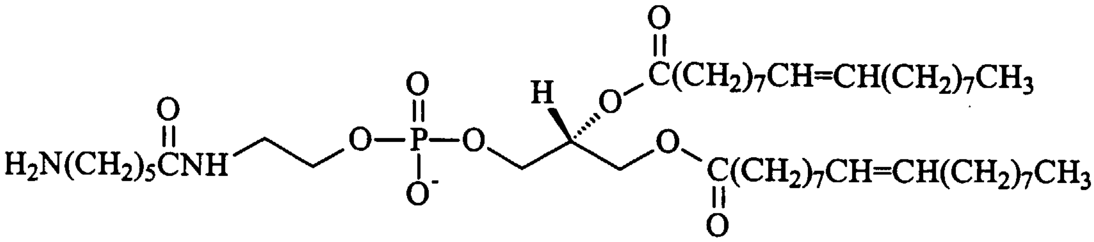

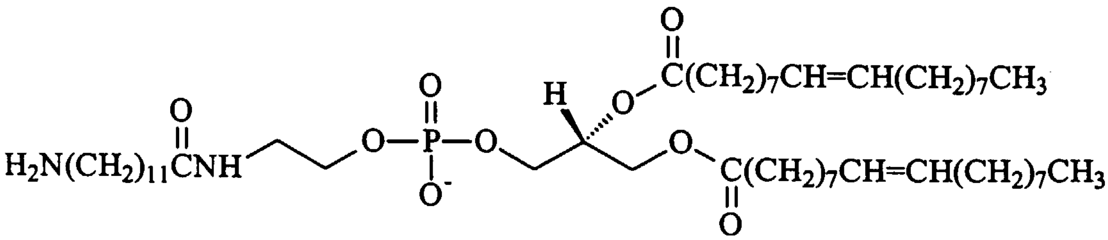

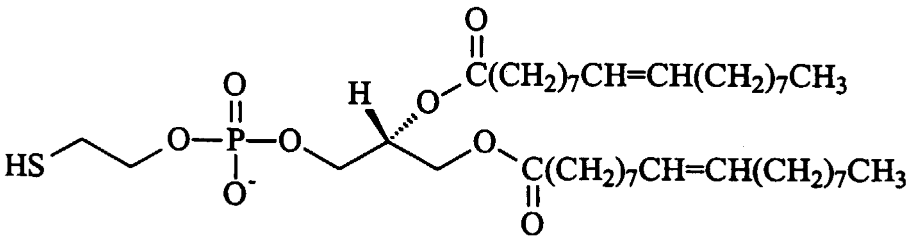

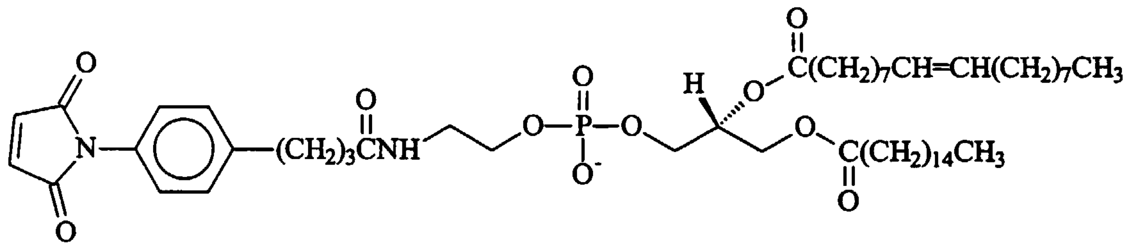

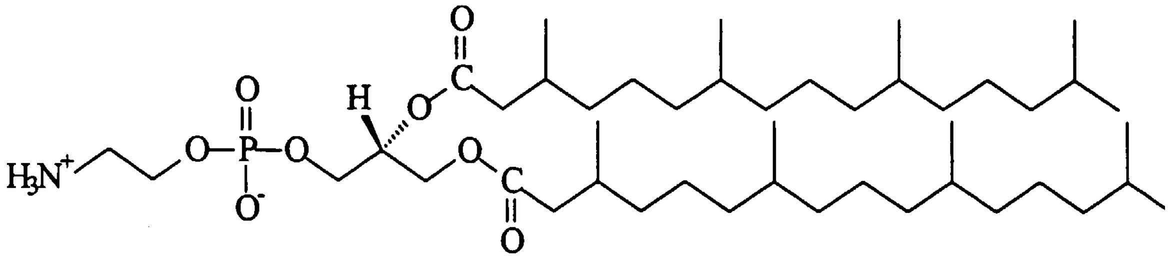

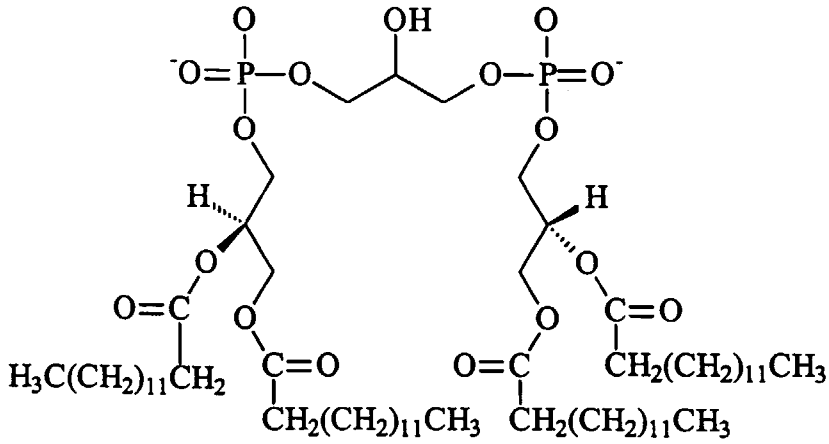

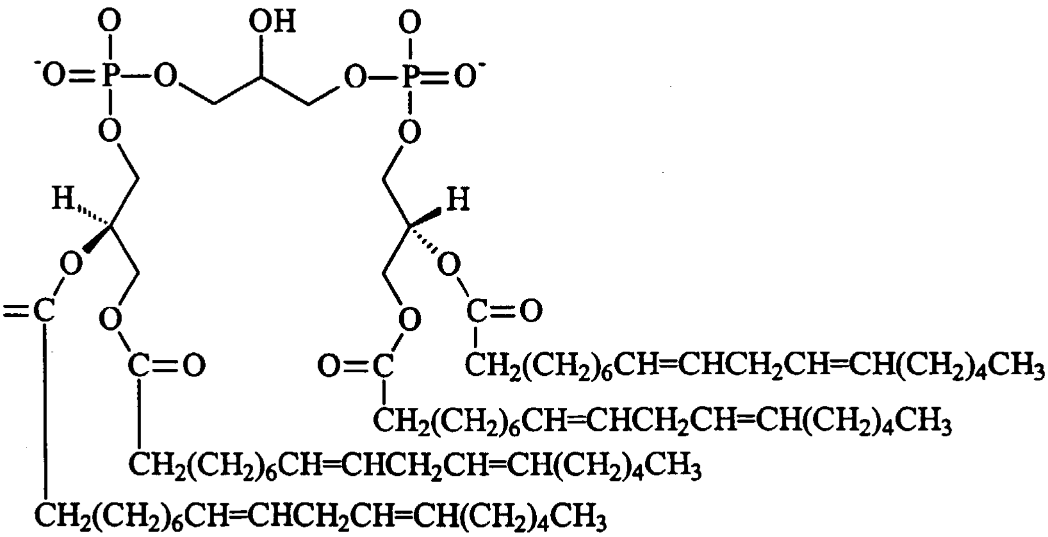

deuterated lipids, such as

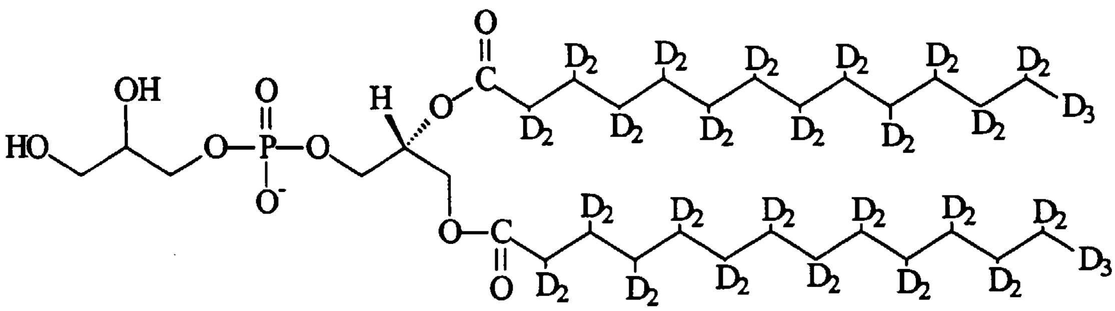

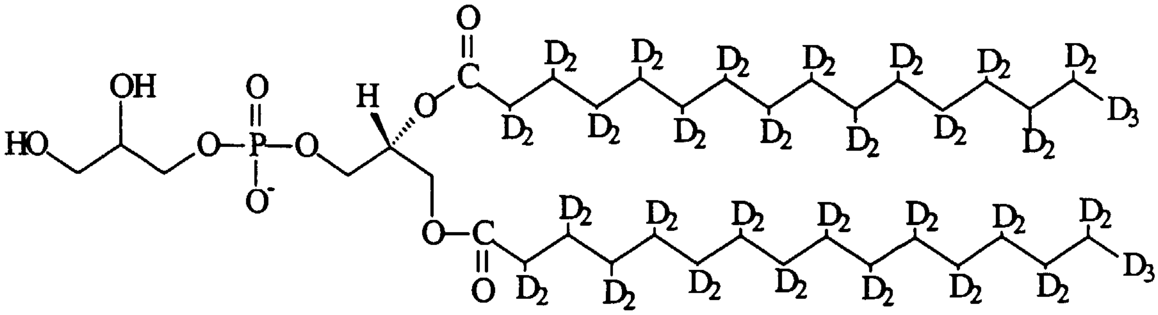

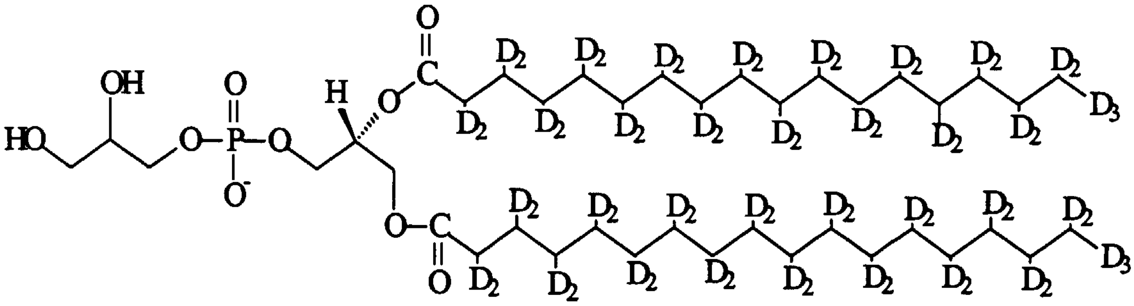

If desired, a cationic lipid may be used, such as, for example, N-[1-(2,3-dioleoyloxy)propyl)-N,N,N-trimethylammonium chloride (DOTMA), 1 ,2-dioleoyloxy-3-(trimethylammonio)propane (DOTAP); and 1,2-dioleoyl-3-(4'-trimethylammonio)-butanoyl-sn-glycerol (DOTB). If a cationic lipid is employed in the lipid compositions, the molar ratio of cationic lipid to non-cationic lipid may be, for example, from about 1:1000 to about 1:100. Preferably, the molar ratio of cationic lipid to non-cationic lipid may be from about 1:2 to about 1:10, with a ratio of from about 1:1 to about 1:2.5 being preferred. Even more preferably, the molar ratio of cationic lipid to non-cationic lipid may be about 1:1.

If desired, aggregates or cochleates may be constructed of one or more charged lipids in association with one or more polymer bearing lipids, optionally in association with one or more neutral lipids. The charged lipids may either be anionic (*i.e.*, negatively charged, that is, carrying a net negative charge) or cationic (*i.e.*, positively charged, that is, carrying a net positive charge). Typically, the lipids are aggregated in the presence of a multivalent species, such as a counter ion, opposite in charge to the charged lipid.

Exemplary anionic lipids include phosphatidic acid and phosphatidyl glycerol and fat acid esters thereof, amides of phosphatidyl ethanolamine such as anandamides and methanandamides, phosphatidyl serine, phosphatidyl inositol and fatty acid esters thereof, cardiolipin, phosphatidyl ethylene glycol, acidic lysolipids, sulfolipids, and sulfatides, free fatty acids, both saturated and unsaturated, and negatively charged derivatives thereof. Phosphatidic acid and phosphatidyl glycerol and fatty acid esters thereof are preferred anionic lipids.

When the charged lipid is anionic, a multivalent (divalent, trivalent, etc.) cationic material may be used to form aggregates. Useful cations include, for example, cations derived from alkaline earth metals, such as beryllium ($Be^{+2}$), magnesium ($Mg^{+2}$), calcium ($Ca^{+2}$), strontium ($Sr^{+2}$), and barium ($Ba^{+2}$); amphoteric ions such as aluminum ($Al^{+3}$), gallium ($Ga^{+3}$), germanium ($Ge^{+3}$), tin ($Sn^{+4}$), and lead ($Pb^{+2}$ and $Pb^{+4}$); transition metals such as titanium ($Ti^{+3}$ and $Ti^{+4}$), vanadium ($V^{+2}$ and $V^{+3}$), chromium ($Cr^{+2}$ and $Cr^{+3}$), manganese ($Mn^{+2}$ and $Mn^{+3}$), iron ($Fe^{+2}$ and $Fe^{+3}$), cobalt ($Co^{+2}$ and $Co^{+3}$), nickel ($Ni^{+2}$ and $Ni^{+3}$), copper ($Cu^{+2}$), zinc ($Zn^{+2}$), zirconium ($Zr^{+4}$), niobium ($Nb^{+3}$), molybdenum ($Mo^{+2}$ and $Mo^{+3}$), cadmium ($Cd^{+2}$), indium ($In^{+3}$), tungsten ($W^{+2}$ and $W^{+4}$), osmium ($Os^{+2}$, $Os^{+3}$ and $Os^{+4}$), iridium ($Ir^{+2}$, $Ir^{+3}$ and $Ir^{+4}$), mercury ($Hg^{+2}$), and bismuth ($Bi^{+3}$); and rare earth lanthanides, such as lanthanum ($La^{+3}$), and gadolinium ($Gd^{+3}$). Cations in all of their ordinary valence states will be suitable for forming aggregates and crosslinked lipids. Preferred cations include calcium ($Ca^{+2}$), magnesium ($Mg^{+2}$), and zinc ($Zn^{+2}$) and paramagnetic cations such as manganese (preferably $Mn^{+2}$) and gadolinium ($Gd^{+3}$). Particularly preferred is calcium ($Ca^{+2}$). Some of the above ions (e.g., lead and nickel) may have associated toxicity and thus may be inappropriate for *in vivo* use.

One or more cations, such as zinc ($Zn^{+2}$) or manganese ($Mn^{+2}$ and $Mn^{+3}$) may be optionally included in the present compositions. It is contemplated that these cations may, for example, improve transfection efficiency and moderate gene expression. Chelates such as ethylenediaminetetraacetic acid (EDTA), 1,4,7,10-tetraazocyclododecane-N',N',N",N"-tetraacetic acid (DOTA), and diethylenetriaminepentaacetic acid (DTPA) may also be employed to deliver such cations to the target cells. Preferably, the cations are derivatized for incorporation into a stabilizing or DNA-condensing media. For example, the cyclic anhydride of DTPA may be covalently bound to polylysine. Preferably, the chelate is covalently bound to a lipid as described, for example, in U.S. Patent No. 5,512,294, the disclosures of which is hereby incorporated herein by reference, in their entirety. In embodiments involving chelates covalently bound to lipids, from about 0.1 to about 10 mole % of the lipid employed may preferably be derivatized to form the chelate-bearing lipids.

When the charged lipid is cationic, an anionic material may be used to form aggregates. Preferably, the anionic material is multivalent, such as, for example, divalent. Examples of useful anionic materials include monatomic and polyatomic anions such as carboxylate ions, sulfide ion, sulfite ions, sulfate ions, oxide ions, nitride ions, carbonate ions, and phosphate ions. Anions of EDTA, DTPA, and DOTA may also be used. Other examples of useful anionic materials include anions of polymers and copolymers of acrylic acid, methacrylic acid, other polyacrylates and methacrylates, polymers with pendant $SO_3H$ groups, such as sulfonated polystyrene, and polystyrenes containing carboxylic acid groups.

Examples of cationic lipids include those listed above. A preferred cationic lipid for formation of aggregates is N-[1-(2,3-dioleoyloxy)propyl]-N,N,N-trimethylammonium chloride (DOTMA). Synthetic cationic lipids may also be used. Synthetic cationic lipids include common natural lipids derivatized to contain one or more basic functional groups. Examples of lipids which can be so modified include dimethyldioctadecylammonium bromide, sphingolipids, sphingomyelin, lysolipids, glycolipids such as ganglioside GM1, sulfatides, glycosphingolipids, cholesterol and cholesterol esters and salts, N-succinyldioleoylphosphatidylethanolamine, 1,2,-dioleoyl-sn-glycerol, 1,3-dipalmitoyl-2-succinylglycerol, 1,2-dipalmitoyl-sn-3-succinylglycerol, 1-hexadecyl-2-palmitoylglycerophosphatidylethanolamine and palmitoylhomocystiene.

Specially synthesized cationic lipids also function in the embodiments of the invention, such as those described in U.S. Patent No. 5,830,430, the disclosures of which are hereby incorporated herein by reference in their entirety, and include, for example, N,N'-bis (dodecyaminocarbonyl-methylene)-N,N'-bis((β-N,N,N-trimethylammoniumethylaminocarbonylmethylene-ethylenediamine tetraiodide; N,N"-bis-hexadecylaminocarbonylmethylene)-N,N',N"-tris(β-N,N,N-trimethylammoniumethylaminocarbonylmethylenediethylenetriamine hexaiodide; N,N'-bis(dodecylaminocarbonylmethylene)-N,N"-bis(β-N,N,N-trimethylammoniumethylaminocarbonylmethylene)cyclohexylene- 1,4-diamine tetraiodide; 1,1,7,7-tetra-(β-N,N,N,N-tetramethylammoniumethylaminocarbonyl-methylene)-3-hexadecyl- aminocarbonylmethylene- 1,3,7-triaazaheptane heptaiodide; and N,N,N',N'-tetraphosphoethanolaminocarbonylmethylene)diethylenetriamine tetraiodide.

Additional suitable cationic lipids include dimyristyloxypropyl-3-dimethylhydroxyethylammonium bromide (DMRIE), dilauryloxypropyl-3-dimethylhydroxyethylammonium bromide (DLRIE), N-[1-(2,3-dioleoyloxyl)propal]-n,n,n-trimethylammonium sulfate (DOTAP), dioleoylphosphatidylethanolamine (DOPE), dipalmitoylethylphosphatidylcholine (DPEPC), dioleoylphosphatidylcholine (DOPC), polylysine, lipopolylysine, didoceylmethylammonium bromide(DDAB), 2,3-dioleoyloxy-N-[2-(sperminecarboxamidoethyl]-N,N-dimethyl-1-propanaminium trifluoroacetate (DOSPA), cetyltrimethylammonium bromide (CTAB), lysyl-PE, 3,β-[N,(N',N'-dimethylaminoethane)carbamoyl]cholesterol (DC-Cholesterol, also known as DC-Chol), (-alanyl cholesterol, N-[1-(2,3-dioleoyloxy)propyl]-N,N,N-trimethylammonium chloride (DOTMA), dipalmitoylphosphatidylethanolamine-5-carboxyspermylamide (DPPES), dicaproylphosphatidylethanolamine (DCPE), 4-dimethylaminopyridine (DMAP), dimyristoylphosphatidylethanolamine (DMPE), dioleoylethylphosphocholine (DOEPC), dioctadecylamidoglycyl spermidine (DOGS), N-[1-(2,3-dioleoyloxy)propyl]-N-[1-(2-hydroxyethyl)]-N,N-dimethylammonium iodide (DOHME), Lipofectin (DOTMA + DOPE, Life Technologies, Inc., Gaithersburg, MD), Lipofectamine (DOSPA + DOPE, Life Technologies, Inc., Gaithersburg, MD), Transfectace (Life Technologies, Inc., Gaithersburg, MD), Transfectam (Promega Ltd., Madison, WI), Cytofectin (Life Technologies Inc., Gaithersburg, MD). Other representative cationic lipids include but are not limited to phosphatidylethanolamine, phospatidylcholine, glycero-3-ethylphosphatidylcholine and fatty acyl esters thereof, di- and trimethylammonium propane, di-and triethylammonium propane and fatty acyl esters thereof. A preferred derivative from this group is N-[1-(2,3-dioleoyloxy)propyl]-N,N,N-trimethylammonium chloride (DOTMA). Additionally, a wide array of synthetic cationic lipids function as compounds useful in the invention. These include common natural lipids derivatized to contain one or more basic functional groups. Examples of lipids which may be so modified include but are not limited to dimethyldioctadecylammonium bromide, sphingolipids, sphingomyelin, lysolipids, glycolipids such as ganglioside GM1, sulfatides, glycosphingolipids, cholesterol and cholesterol esters and salts, N-succinyldioleoylphosphatidylethanolamine, 1,2,-dioleoyl-sn-glycerol, 1,3-dipalmitoyl-2-succinylglycerol, 1,2-dipalmitoyl-sn-3-succinylglycerol, 1 -hexadecyl-2-palmitoylglycerophosphatidyl-ethanolamine and palmitoylhomocystiene.

In the case of carriers which include both cationic and non-cationic lipids, a wide variety of lipids, as

described above, may be employed as the non-cationic lipid. Preferably, the non-cationic lipid comprises one or more phospholipids, such as DPPC, DPPE and dioleoylphosphatidylethanolamine. Instead of the cationic lipids listed above, lipids bearing cationic polymers, such as polylysine or polyarginine, as well as alkyl phosphonates, alkyl phosphinates, and alkyl phosphites, may be used in the stabilizing materials.

Saturated and unsaturated fatty acids which may be included as stabilizing materials in the carriers include molecules that contain from about 12 carbon atoms to about 22 carbon atoms, in linear or branched form. Hydrocarbon groups consisting of isoprenoid units and/or prenyl groups can be used. Suitable saturated fatty acids include, for example, lauric, myristic, palmitic, and stearic acids. Suitable unsaturated fatty acids include, for example, lauroleic, physeteric, myristoleic, palmitoleic, petroselinic, and oleic acids. Suitable branched fatty acids include, for example, isolauric, isomyristic, isopalmitic, and isostearic acids.

Other useful lipids or combinations thereof apparent to one skilled in the art are also encompassed by the present invention. For example, carbohydrate-bearing lipids may be used, as described in U.S. Patent No. 4,310,505, the disclosures of which are hereby incorporated herein by reference in their entirety.

In addition to stabilizing materials and/or vesicles formulated from lipids, embodiments of the present invention may involve vesicles formulated, in whole or in part, from proteins or derivatives thereof. Suitable proteins include, for example, albumin, hemoglobin, α-1-antitrypsin, α-fetoprotein, aminotransferases, amylase, C-reactive protein, carcinoembryonic antigen, ceruloplasmin, complement, creatine phosphokinase, ferritin, fibrinogen, fibrin, transpeptidase, gastrin, serum globulins, myoglobin, immunoglobulins, lactate dehydrogenase, lipase, lipoproteins, acid phosphatase, alkaline phosphatase, α-1-serum protein fraction, α-2-serum protein fraction, β-protein fraction, γ-protein fraction and γ-glutamyl transferase. Other stabilizing materials and vesicles formulated from proteins that may be used in the present invention are described, for example, in U.S. Patent Nos. 4,572,203, 4,718,433, 4,774,958, and 4,957,656, the disclosures of each of which are hereby incorporated herein by reference in their entirety. Other suitable protein-based stabilizing materials and vesicles would be apparent to one of ordinary skill in the art in view of the present disclosure.

In addition to stabilizing materials and/or vesicles formulated from lipids and/or proteins, embodiments of the present invention may also involve stabilizing materials or vesicles formulated from polymers which may be of natural, semi-synthetic (modified natural) or synthetic origin. Polymer denotes a compound comprised of two or more repeating monomeric units, and preferably 10 or more repeating monomeric units. Semi-synthetic polymer (or modified natural polymer) denotes a natural polymer that has been chemically modified in some fashion. Suitable natural polymers include naturally occurring polysaccharides, such as, for example, arabinans, fructans, fucans, galactans, galacturonans, glucans, mannans, xylans (such as, for example, insulin), levan, fucoidan, carrageenan, galatocarolose, pectic acid, pectins (including high methoxy pectin and low methoxy pectin; with low methoxy pectin preferably denoting pectin in which less than about 40% of the carboxylic acid groups are esterified and/or amidated, and high methoxy pectin preferably denoting pectin in which about 40% or more of the carboxylic acid groups are esterified and/or amidated), amylose, pullulan, glycogen, amylopectin, cellulose, dextran, dextrin, dextrose, glucose, polyglucose, polydextrose, pustulan, chitin, agarose, keratin, chondroitin, dermatan, hyaluronic acid, alginic acid, xanthin gum, starch and various other natural homopolymer or heteropolymers, such as those containing one or more of the following aldoses, ketoses, acids or amines: erythrose, threose, ribose, arabinose, xylose, lyxose, allose, altrose, glucose, dextrose, mannose, gulose, idose, galactose, talose, erythrulose, ribulose, xylulose, psicose, fructose, sorbose, tagatose, mannitol, sorbitol, lactose, sucrose, trehalose, maltose, cellobiose, glycine, serine, threonine, cysteine, tyrosine, asparagine, glutamine, aspartic acid, glutamic acid, lysine, arginine, histidine, glucuronic acid, gluconic acid, glucaric acid, galacturonic acid, mannuronic acid, glucosamine, galactosamine, and neuraminic acid, and naturally occurring derivatives thereof. Accordingly, suitable polymers include, for example, proteins, such as albumin. Exemplary semi-synthetic polymers include celluloses, such as carboxymethylcellulose, hydroxymethylcellulose, hydroxypropylmethylcellulose, methylcellulose, and methoxycellulose. Exemplary synthetic polymers include polyphosphazenes, polyalkylenes (*e.g*., polyethylene), such as, for example, polyethylene glycol (including, for example, the class of compounds referred to as PLURONICS®, commercially available from BASF, Parsippany, NJ), polyoxyalkylenes (*e.g.*, polyoxyethylene, such as polyoxyethylene glycol), and polyethylene terephthlate, polypropylenes (such as, for example, polypropylene glycol), polyurethanes, polyvinyls (such as, for example, polyvinyl alcohol (PVA), polyvinyl chloride and polyvinyl pyrrolidone), polyamides including nylon, polystyrene, polylactic acids, fluorinated hydrocarbon polymers, fluorinated carbon polymers (such as, for example, polytetrafluoroethylene), acrylate, methacrylate, and polymethylmethacrylate, and derivatives thereof, and pluronics and alcohols. Preferred are synthetic polymers or copolymers prepared from monomers, such as acrylic acid, methacrylic acid, ethyleneimine, crotonic acid, acrylamide, ethyl acrylate, methyl methacrylate, 2-hydroxyethyl methacrylate (HEMA), lactic acid, glycolic acid, ε-caprolactone, acrolein, cyanoacrylate, bisphenol A, epichlorhydrin, hydroxyalkyl-acrylates, siloxane, dimethylsiloxane, ethylene oxide, ethylene glycol, hydroxyalkyl-methacrylates, N-substituted acrylamides, N-substituted methacrylamides, N-vinyl-2-pyrrolidone, 2,4-pentadiene-1 -ol, vinyl acetate, acrylonitrile, styrene, p-amino-styrene, p-aminobenzylstyrene, sodium styrene sulfonate, sodium 2-sulfoxyethylmethacrylate, vinyl pyridine, aminoethyl methacrylates, 2-methacryloyloxytrimethylammonium chloride, and polyvinylidene, as well polyfunctional crosslinking monomers such as N,N'-methylenebisacrylamide, ethylene glycol

dimethacrylates, 2,2'-(p-phenylene-dioxy)diethyl dimeth-acrylate, divinylbenzene, triallylamine and methylenebis(4-phenylisocyanate), including combinations thereof. Preferable polymers include polyacrylic acid, polyethyleneimine, polymethacrylic acid, polymethylmethacrylate, polysiloxane, polydimethylsiloxane, polylactic acid, poly(ε-caprolactone), epoxy resin, poly(ethylene oxide), poly(ethylene glycol), and polyamide (nylon) polymers. Preferable copolymers include polyvinylidene-polyacrylonitrile, polyvinylidene-polyacrylonitrile-polymethylmethacrylate, polystyrene-polyacrylonitrile and poly d-1, lactide co-glycolide polymers; most preferably polyvinylidene-polyacrylonitrile. The polymers employed may also comprise fluorinated polymers, including those described in Lohrmann, U.S. Patent No. 5,562,892, the disclosures of which are hereby incorporated herein by reference in their entirety. Furthermore, the polymers may be in the form of vesicles, such as for example, those described in Unger, U.S. Patent No. 5,205,290, the disclosures of which are hereby incorporated herein by reference in their entirety. Other suitable monomers and polymers will be apparent to one skilled in the art in view of the present disclosure.

Stabilizing materials and vesicles may be prepared from other materials. The materials may be basic and fundamental, and may form the primary basis for creating or establishing the stabilizing materials. For example, surfactants and fluorosurfactants may be basic and fundamental materials for preparing stabilizing materials and vesicles. On the other hand, the materials may be auxiliary, and act as subsidiary or supplementary agents which may enhance the functioning of the basic stabilizing material(s), or contribute some desired property in addition to that afforded by the basic stabilizing material(s), such as surfactants and/or polymers.

It is not always possible to determine whether a given material is a basic or an auxiliary agent, since the functioning of the material is determined empirically, for example, by the results produced with respect to producing stabilizing materials or vesicles. As an example of how the basic and auxiliary materials may function, it has been observed that the simple combination of a lipid and water or saline when shaken will often give a cloudy solution subsequent to autoclaving for sterilization. Such a cloudy solution may function as a contrast agent, but is aesthetically objectionable and may imply instability in the form of undissolved or undispersed lipid particles. Cloudy solutions may also be undesirable where the undissolved particulate matter has a diameter of greater than about 7 μm, and especially greater than about 10 μm. Manufacturing steps, such as sterile filtration, may also be problematic with solutions which contain undissolved particulate matter. Thus, propylene glycol may be added to remove this cloudiness by facilitating dispersion or dissolution of the lipid particles. Propylene glycol may also function as a wetting agent which can improve vesicle formation and stabilization by increasing the surface tension on the vesicle membrane or skin. It is possible that propylene glycol can also function as an additional layer that may coat the membrane or skin of the vesicle, thus providing additional stabilization. The surfactants described in U.S. Patent Nos. 4,684,479, 5,215,680, and 5,562,893 the disclosures of each of which are hereby incorporated by reference herein in their entirety, may be used as basic or auxiliary stabilizing materials in the present invention.

Oils and fluorinated oils are auxiliary and basic stabilizing materials that may be used in the compositions of the present invention. Suitable oils include, for example, soybean oil, peanut oil, canola oil, olive oil, safflower oil, corn oil, mazola oil, cod liver oil, almond oil, cottonseed oil, ethyl oleate, isopropyl myristate, isopropyl palmitate, mineral oil, myristyl alcohol, octyldodecanol, persic oil, sesame oil, myristyl oleate, cetyl oleate, and myristyl palmitate. Other suitable oils include biocompatible oils consisting of saturated, unsaturated, and/or partially hydrogenated fatty acids, silicon-based oils including, for example, vinyl-terminated, hydride-terminated, silanol-terminated, amino-terminated, epoxy-terminated, carbinol-terminated fluids, and other silicon-based oils such as mercapto-modified silicon fluids and saturated, unsaturated, or aryl-alkyl- or alkyl-aryl- substituted silicon oils, synthetic oils such as triglycerides composed of saturated and unsaturated chains of $C_{12}$-$C_{24}$ fatty acids, such as for example the glycerol triglyceride ester of oleic acid, terpenes, linolene, squalene, squalamine, or any other oil commonly known to be ingestible which is suitable for use as a stabilizing compound in accordance with the teachings herein. The oils described herein may be fluorinated, such as fluorinated triolein. A "fluorinated oil" refers to an oil in which at least one hydrogen atom of the oil is replaced with a fluorine atom. Preferably, at least two or more of the hydrogen atoms in the oil are replaced with fluorine atoms. Fluorinated triglyceride oils may be prepared by reacting a reactive fluorinated species, such as, for example, a fluorine gas, with unsaturated triglyceride oils to produce the desired fluorinated triglyceride. Other oils are described, for example, in U.S. Patent No. 5,344,930, the disclosures of which are hereby incorporated by reference herein in their entirety.

Additional auxiliary and basic stabilizing materials which may be used in the present invention are described, for example, in U.S. Patent No. 5,736,121, the disclosures of which are hereby incorporated herein by reference in their entirety.

Compounds used to make mixed micelle systems may be used as basic or auxiliary stabilizing materials, and include, for example, sodium dodecyl sulfate, cetylammonium halides, cetylalkylammonium halides, lauryltrimethylammonium bromide (dodecyl-), cetyltrimethylammonium bromide (hexadecyl-), myristyltrimethylammonium bromide (tetradecyl-), alkyldimethylbenzylammonium chloride (where alkyl is $C_{12}$, $C_{14}$ or $C_{16}$), benzyldimethyldodecylammonium bromide/chloride, benzyldimethyl hexadecylammonium bromide/chloride, benzyldimethyl tetradecylammonium halide (e.g, bromide/chloride, cetyldimethylethylammonium halide bromide/chloride, or cetylpyridinium bromide/chloride.

It may be possible to enhance the stability of stabilizing materials or vesicles by incorporating in the stabilizing materials and/or vesicles at least a minor amount, for example, about 1 to about 10 mole %, based on the total amount of lipid used, of a negatively charged lipid. Suitable negatively charged lipids include, for example, phosphatidylserine, phosphatidic acid, and fatty acids. Without intending to be bound by any theory of operation, it is believed that such negatively charged lipids provide added stability by counteracting the tendency of vesicles to rupture by fusing together. Thus, the negatively charged lipids may act to establish a uniform negatively charged layer on the outer surface of the vesicle, which will be repulsed by a similarly charged outer layer on other vesicles which are proximate thereto. In this way, the vesicles may be less prone to come into touching proximity with each other, which may lead to a rupture of the membrane or skin of the respective vesicles and consolidation of the contacting vesicles into a single, larger vesicle. A continuation of this process of consolidation will, of course, lead to significant degradation of the vesicles.

The lipids used, especially in connection with vesicles, are preferably flexible. This means, in the context of the present invention, that the vesicles can alter their shape, for example, to pass through an opening having a diameter that is smaller than the diameter of the vesicle.

In preferred embodiments, the stabilizing material and/or vesicle composition may contain, in whole or in part, a fluorinated (including perfluorinated) compound. Suitable fluorinated compounds include, for example, fluorinated surfactants, including alkyl surfactants, and fluorinated amphiphilic compounds. A wide variety of such compounds may be employed, including, for example, the class of compounds which are commercially available as ZONYL® fluorosurfactants (the DuPont Company, Wilmington, DE), including ZONYL® phosphate salts (*e.g.*, $[F(CF_2CF_2)_{3-8}CH_2CH_2O]_{1,2}$-$P(O)(O^-NH_4^+)_{2,1}$) which have terminal phosphate groups, and ZONYL® sulfate salts which have terminal sulfate groups (*e.g.*, $F(CF_2CF_2)_{3-8}CH_2CH_2SCH_2CH_2N^+(CH_3)_3$ $^-OSO_2OCH_3$). Suitable ZONYL® surfactants also include, for example, ZONYL® fluorosurfactants identified as Telomer B, including Telomer B fluorosurfactants which are pegylated (*i.e.*, have at least one polyethylene glycol group aft ached thereto), also known as PEG-Telomer B, available from the DuPont Company. Other suitable fluorosurfactants are described in U.S. Patent Nos. 5,276,146, 5,344,930 and 5,562,893, and U.S. Application Serial No. 08/465,868, filed June 6, 1995, the disclosures of each of which are hereby incorporated by reference herein in their entirety.

Other suitable fluorinated surfactants and fluorinated lipid compounds for use as the stabilizing materials in the present invention are described in U.S. application Serial No. 08/887,215, filed July 2, 1997, the disclosures of which are hereby incorporated by reference herein in their entirety. Such fluorinated surfactants and fluorinated lipids include the compounds of formulas (I), (II), (III), (IV), (IVa), (V), (Va), (VI) and (VII), as described therein.

For example, the stabilizing material may be a fluorinated fatty acyl derivative, such as, for example, that of formula (I):

$$CF_3\text{-}(CF_2)_n\text{-}(CH_2)_m\text{-}C(=O)\text{-}OH \qquad (I)$$

where n is an integer of from about 7 to about 13, preferably from about 9 to about 11; and m is an integer of from 1 to about 4, preferably 1 to about 2.

The stabilizing material may be a PEG Telomer compound of formula (II):

$$C_xF_{2x+1}\text{-}(CH_2)_z\text{-}(OCH_2CH_2)_z\text{-}OH \qquad (II)$$

where x is an integer of from about 6 to about 12, preferably from about 8 to about 10, more preferably about 9; and z is an integer of from about 8 to about 20; preferably from about 8 to about 16; still more preferably from about 8 to about 12; even more preferably about 8 to about 10; most preferably about 9.

The stabilizing material may be a fluorinated carbohydrate derivative, such as, for example, that of formula (III):

$$C_xF_{2x+1}\text{-}(CH_2)_2\text{-}(OCH_2CH_2)_z\text{-}O\text{-}A \qquad (III)$$

where x is an integer of from about 6 to about 12; preferably from about 8 to about 10; more preferably 9; z is an integer of from about 8 to about 20; preferably from about 8 to about 16; more preferably from about 8 to about 12; still more preferably from about 8 to about 10; most preferably about 9; and A is a monosaccharide or a disaccharide. Suitable monosaccharides and disaccharides include, for example, allose, altrose, glucose, dextrose, mannose, glycerose, gulose, idose, galactose, talose, fructose, psicose, sorbose, rhamnose, tagatose, ribose, arabinose, xylose, lyxose, ribulose, xylulose, erythrose, threose, erythrulose, fucose, sucrose, lactose, maltose, isomaltose, trehalose, cellobiose and the like. Preferably, the monosaccharide or disaccharide is glucose, dextrose, fructose, mannose, galactose, glucosamine, galactosamine, maltose, sucrose or lactose.

The stabilizing material may also be a fluorinated lipophilic derivative, such as, for example, that of formula

(IV), which includes the compounds described in U.S. application Serial No. 08/465,868, filed June 6, 1995, the disclosures of which are hereby incorporated by reference herein in their entirety:

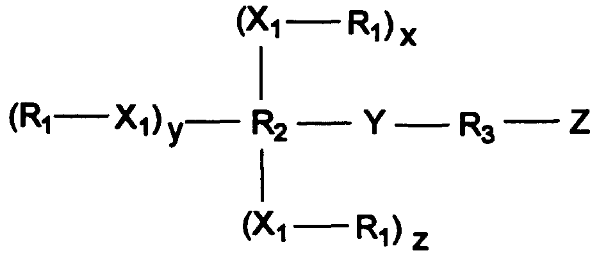

(IV)

where each of x, y and z is independently 0 or 1; each $X_1$ is independently -O-, -S-, -SO-, $-SO_2-$, $-NR_4-$, $-C(=X_2)-$, $-C(=X_2)-O-$, $-O-C(=X_2)-$, $-C(=X_2)-NR_4-$ or $-NR_4-C(=X_2)-$; $X_2$ is O or S; Y is a direct bond or $-X_3-M(=O)(OR_5)_q-O-$, where q is 1 or 2; $X_3$ is a direct bond or -O-; M is P or S; Z is hydrogen, the residue of a hydrophilic polymer, a saccharide residue or $-N(R_6)_r$, where r is 2 or 3; each $R_1$ is independently an alkyl group of 1 to about 30 carbon atoms or a fluorinated alkyl group of 1 to about 30 carbon atoms; $R_2$ is a direct bond or an alkylene linking group of 1 to about 10 carbon atoms; $R_3$ is a direct bond or an alkylene diradical of 1 to about 10 carbon atoms; each of $R_4$ and $R_5$ is independently hydrogen or an alkyl group of 1 to about 8 carbon atoms; and each $R_6$ is independently hydrogen, an alkyl group of 1 to about 8 carbon atoms or a residue of a hydrophilic polymer; provided that at least one of x, y and z is 1, at least one of $R_1$ is a fluorinated alkyl group of 1 to about 30 carbon atoms; provided that when $R_2$ is a direct bond, two of x, y and z are each 0.

In formula (IV), each of x, y and z is independently 0 or 1, provided that at least one of x, y and z is 1. In some embodiments, two of x, y and z are each 0. In other embodiments, one of x, y and z is 0 or 1 and the other two of x, y and z are each 1, with one of x, y and z being 0 and the other two of x, y and z being 1 being more preferred. In other embodiments, each of x, y and z is 1.

Each $X_1$ is independently -O-, -S-, -SO-, $-SO_2-$, $-NR_4-$, $-C(=X_2)-$, $-C(=X_2)-O-$, $-O-C(=X_2)-$, $-C(=X_2)-NR_4-$ or $-NR_4-C(=X_2)-$. Preferably, each $X_1$ is independently -O-, -S-, $-C(=X_2)-$, $-C(=X_2)-O-$, $-O-C(=X_2)-$, $-C(=X_2)-NR_4-$ or $-NR_4-C(=X_2)-$. More preferably, each $X_1$ is independently $-C(=X_2)-O-$ or $-O-C(=X_2)-$, most preferably $-C(=X_2)-O-$.

Each $X_2$ is O or S, preferably O.

Y is a direct bond or $-X_3-M(=O)(OR_5)_q-O-$, where q is 1 or 2. Preferably, Y is $-X_3-M(=O)(OR_5)_q-O-$. M is P or S, preferably P. $X_3$ is a direct bond or -O-, preferably, a direct bond.

Z is hydrogen atom, the residue of a hydrophilic polymer, a saccharide residue or $-N(R_6)_r$, where r is 2 or 3. In preferred embodiments, Z is $-N(R_6)_r$.

Each $R_1$ is independently an alkyl group of 1 to about 30 carbon atoms or a fluorinated alkyl group of 1 to about 30 carbon atoms, provided that at least one of $R_1$ is a fluorinated alkyl group of 1 to about 30 carbon atoms. Thus, when only one of x, y and z is 1, $R_1$ is necessarily a fluorinated alkyl group of 1 to about 30 carbon atoms. In preferred embodiments, where one or none of x, y and z is 0, and preferably where one of x, y and z is 0 and the other two of x, y and z are each 1, at least one of $R_1$ is an alkyl group of 1 to about 30 carbon atoms and at least one of $R_1$ is a fluorinated alkyl group of 1 to about 30 carbon atoms. In other embodiments, each $R_1$ is independently a fluorinated alkyl group of 1 to about 30 carbon atoms. When a fluorinated alkyl group of 1 to about 30 carbon atoms, $R_1$ is preferably a polyfluorinated alkyl group of 1 to about 30 carbon atoms, with a perfluorinated alkyl group of 1 to about 30 carbon atoms being more preferred. When a fluorinated alkyl group of 1 to about 30 carbon atoms, $R_1$ is preferably $C_nF_{2n+1}-(CH_2)_m-$, where n is 1 to about 16, preferably about 9 to about 14, and m is 0 to about 18, preferably 1 to about 10, more preferably 1 to about 4.

$R_2$ is a direct bond or an alkylene linking group of 1 to about 10 carbon atoms, provided that when $R_2$ is a direct bond, two of x, y and z are each 0. Preferably, $R_2$ is a direct bond or an alkylene linking group of 1 to about 4 carbon atoms. More preferably, $R_2$ is an alkylene linking group of about 3 carbons. Even more preferably, $R_2$ is $-CH_2-CH_2-CH_2-$.

$R_3$ is a direct bond or an alkylene diradical of 1 to about 10 carbons. Preferably, $R_3$ is a direct bond or an alkylene diradical of 1 to about 4 carbon atoms. More preferably, $R_3$ is an alkylene diradical of about 2 carbon atoms. Even more preferably, $R_3$ is $-CH_2CH_2-$.

Each of $R_4$ and $R_5$ is independently a hydrogen atom or an alkyl group of 1 to about 8 carbon atoms, preferably of 1 to about 4 carbon atoms. More preferably, each of $R_4$ and $R_5$ is a hydrogen atom.

$R_6$ is a hydrogen atom, an alkyl group of 1 to about 8 carbon atoms or a residue of a hydrophilic polymer.

Preferably, $R_6$ is a hydrogen atom or an alkyl group of 1 to about 4 carbon atoms. More preferably, $R_6$ is a hydrogen atom or a methyl group, with a methyl group being even more preferred.

When any symbol appears more than once in a particular formula or substituent, such as, for example, in formula (IV), its meaning in each instance is independent of the other, unless otherwise indicated. This independence of meaning is subject to any of the stated provisos. Also, when each of two or more adjacent symbols is defined as being "a direct bond" to provide multiple, adjacent direct bonds, the multiple and adjacent direct bonds devolve into a single direct bond.

Z and $R_6$ in the definition of Z in formula (IV), can be the residue of a hydrophilic polymer. Exemplary polymers from which Z and/or $R_6$ can be derived include polymers in which the repeating units contain one or more hydroxy groups (polyhydroxy polymers), including, for example, poly(vinyl alcohol); polymers in which the repeating units contain one or more amino groups (polyamine polymers), including, for example, peptides, polypeptides, proteins and lipoproteins, such as albumin and natural lipoproteins; polymers in which the repeating units contain one or more carboxy groups (polycarboxy polymers), including, for example, carboxymethylcellulose, alginic acid and salts thereof, such as sodium and calcium alginate, glycosaminoglycans and salts thereof, including salts of hyaluronic acid, phosphorylated and sulfonated derivatives of carbohydrates, genetic material, such as interleukin-2 and interferon, and phosphorothioate oligomers; and polymers in which the repeating units contain one or more saccharide moieties (polysaccharide polymers), including, for example, carbohydrates. The molecular weight of the polymers from which Z and/or $R_6$ are derived may vary, and is generally about 50 to about 5,000,000, with polymers having a molecular weight of about 100 to about 50,000 being preferred. More preferred polymers have a molecular weight of about 150 to about 10,000, with molecular weights of 200 to about 8,000 being even more preferred.

Preferred polymers from which Z and/or $R_6$ are derived include, for example, poly(ethylene glycol) (PEG), poly(vinylpyrrolidine), polyoxomers, polysorbate and poly(vinyl alcohol), with PEG polymers being particularly preferred. Preferred among the PEG polymers are PEG polymers having a molecular weight of from about 100 to about 10,000. More preferably, the PEG polymers have a molecular weight of from about 200 to about 8,000, with PEG 2,000, PEG 5,000 and PEG 8,000, which have molecular weights of 2,000, 5,000 and 8,000, respectively, being even more preferred. Other suitable hydrophilic polymers, in addition to those exemplified above, will be readily apparent to one skilled in the art based on the present disclosure. Generally, polymers from which Z and/or $R_6$ are derived include polymers that can be incorporated in the fluorinated amphiphilic compounds via alkylation or acylation reactions.

As with the various polymers exemplified above, the polymeric residues can contain functional groups in addition, for example, to those typically involved in linking the polymeric residues to the fluorinated amphiphilic compounds. Such functionalities include, for example, carboxyl, amine, hydroxy and thiol groups. These functional groups on the polymeric residues can be further reacted, if desired, with materials which are generally reactive with such functional groups and which can assist in targeting specific tissues in the body including, for example, diseased tissue. Exemplary materials which can be reacted with the additional functional groups include, for example, proteins, including antibodies, carbohydrates, peptides, glycopeptides, glycolipids, lectins and nucleosides.

In addition to residues of hydrophilic polymers, Z in formula (IV) can be a saccharide residue. Exemplary saccharides from which Z can be derived include, for example, monosaccharides or sugar alcohols, such as erythrose, threose, ribose, arabinose, xylose, lyxose, fructose, sorbitol, mannitol and sedoheptulose, with preferred monosaccharides being fructose, mannose, xylose, arabinose, mannitol and sorbitol; and disaccharides, such as lactose, sucrose, maltose and cellobiose. Other saccharides include, for example, inositol and ganglioside head groups. Other suitable saccharides, in addition to those exemplified above, will be readily apparent to one skilled in the art based on the present disclosure. Generally, saccharides from which Z is derived include saccharides that can be incorporated in the fluorinated amphiphilic compounds via alkylation or acylation reactions.

Preferred fluorinated compounds that are within the scope of formula (IV) are the fluorinated compounds of the formula (IVa):

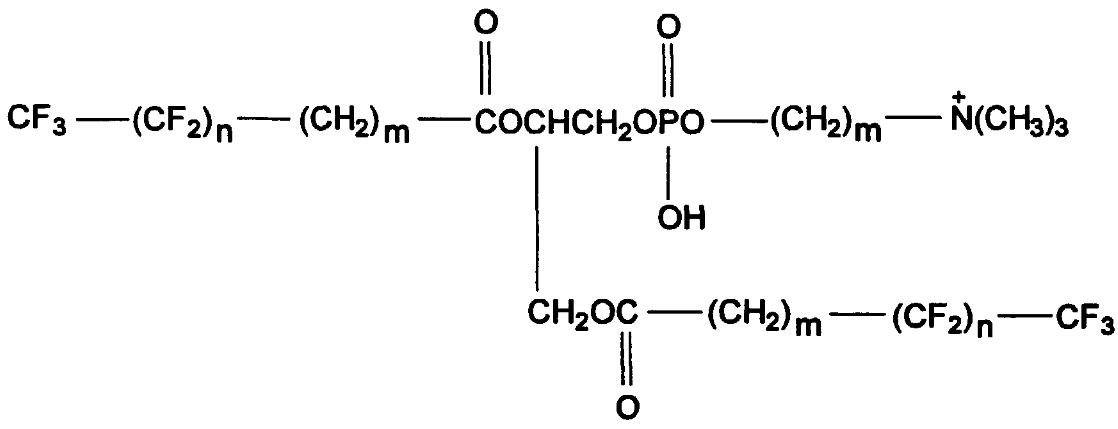

(IVa)

where n is an integer of from about 7 to about 13, preferably from about 9 to about 11; and m is an integer of from about 1 to about 4, preferably 1 to about 2.

The stabilizing material may also be a fluorinated amphiphilic moiety of formula (V):

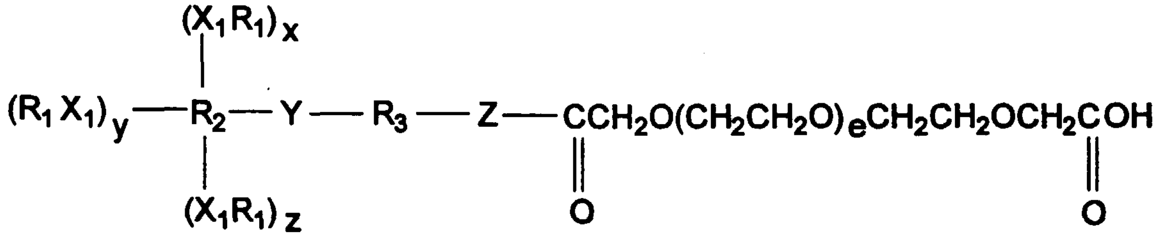

(V)

where R,, $R_2$, $R_3$, $X_1$, Y, Z, x, y and z are as defined in formula (IV), including the preferred embodiments thereof; and where e is an integer of from 1 to about 30, preferably about 3 to about 20, more preferably about 4 to about 16, still more preferably about 4 to about 12, most preferably about 7 to about 9.

In a more preferred embodiment, the compound of formula (V) may be a compound of the formula (Va):

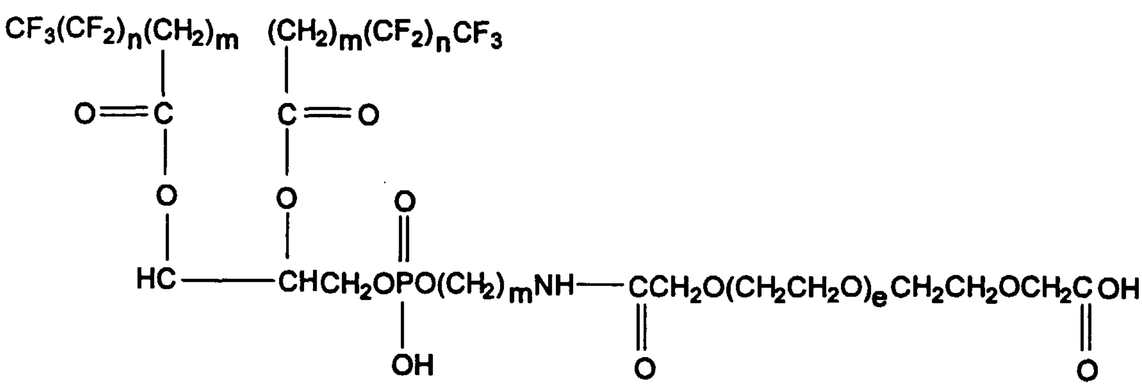

(Va)

where n and m are as defined above in formula (IVa) and where e is as defined above in formula (V).

The stabilizing material may also be a fluorinated fatty acyl derivative, such as, for example, that of formula (VI):

$$CF_3\text{-J-T-C(=O)-OH} \qquad (VI)$$

Still further, the stabilizing material may be a fluorinated lipophilic derivative, such as, for example, that of formula (VII):

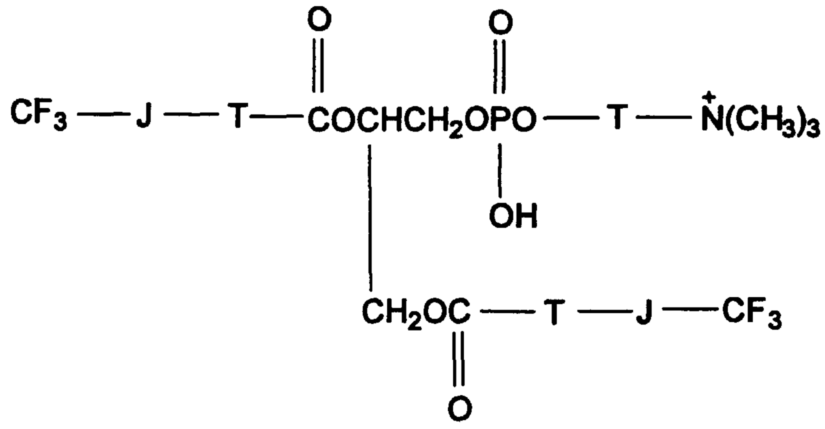

(VII)

In the above formulas (VI) and (VII), J is $(-(C{=}C)_{p1}-(CF_2)_{p2}-(C{=}C)_{p3}-(CF_2)_{p4}-(C{=}C)_{p5}-(CF_2)_{p6}-(C{=}C)_{p7}-(CF_2)_{p8}-(C{=}C)_{p9}-(CF_2)_{p10}-(C{=}C)_{p11}-(CF_2)_{p12}-(C{=}C)_{p13}-,)$, where p1, p2, p3, p4, p5, p6, p7, p8, p9, p10, p11, p12 and p13 are independently an integer of 0, 1 or 2; provided that the sum of (p1 + p2 + p3 + p4 + p5 + p6 + p7 + p8 + p9 + p10 + p11 + p12 + p13) is an integer of from about 7 to about 13, and provided that at least one of p2, p4, p6, p8, p10 or p12 is an integer of at least 1; and where T is $(-(C{=}C)_{t1}-(CH_2)_{t2}-(C{=}C)_{t3}-(CH_2)_{t4}-)$, where t1, t2, t3, and t4 are independently an integer of 0, 1 or 2; provided that the sum of (t1 + t2 + t3 + t4) is an integer of from 1 to about 4.

Other suitable fluorinated compounds that may be used as stabilizing materials and/or vesicles are described in U.S. Patent No. 5,562,893, the disclosures of which are hereby incorporated by reference herein in their entirety. For example, synthetic organic monomeric repeating units may be used to form polymers suitable as stabilizing materials, including hydroxyacids, lactones, lactides, glycolides, acyl containing compounds, aminotriazol, orthoesters, anyhdrides, ester imides, imides, acetals, urethanes, vinyl alcohols, enolketones, and organosiloxanes.

The method of introducing fluorine into any of these materials is known in the art. For example, the introduction of perfluoro-t-butyl moieties is described in U.S. Patent No. 5,234,680, the disclosures of which are hereby incorporated by reference herein in their entirety. These methods generally involve the reaction of perfluoroalkyl carbanions with host molecules as follows:

$$(CF_3)_3C^- + R\text{-}X \rightarrow (CF_3)_3C\text{-}R,$$

where R is a host molecule and X is a leaving group, such as bromine, chlorine, iodine or a sulfonato group. After adding a leaving group to the foregoing stabilizing material using methods well known in the art, perfluoro-t-butyl moieties can then be easily introduced to these derivatized stabilizing materials as described above. Additional methods are known in the art for the introduction of trifluoromethyl groups into various organic compounds. For example, trifluoromethyl groups may be introduced by nucleophilic perfluoroalkylation using perfluoroalkyl-trialkylsilanes.

Fluorine can be introduced into any of the aforementioned stabilizing materials or vesicles either in their monomeric or polymeric form. Preferably, fluorine moieties are introduced into monomers, such as fatty acids, amino acids or polymerizable synthetic organic compounds, which are then polymerized for subsequent use as stabilizing materials and/or vesicles.

Introduction of fluorine into stabilizing materials and/or vesicles may also be accomplished by forming vesicles in the presence of a perfluorocarbon gas. For example, when vesicles are formed from proteins, such as human serum albumin in the presence of a perfluorocarbon gas, such as perfluoropropane, using mechanical cavitation, fluorine from the gas phase becomes bound to the protein vesicles during formation. The presence of fluorine in the vesicles and/or stabilizing materials can be detected by NMR of vesicle debris which has been purified from disrupted

vesicles. Fluorine can also be introduced into stabilizing materials and/or vesicles using other methods, such as sonication, spray-drying or emulsification techniques.

Another way in which fluorine can be introduced into the stabilizing material and/or vesicle is by using a fluorine-containing reactive compound. The term "reactive compound" refers to compounds which are capable of interacting with the stabilizing material and/or vesicle in such a manner that fluorine moieties become covalently attached to the stabilizing material and/or vesicle. When the stabilizing material is a protein, preferred reactive compounds are either alkyl esters or acyl halides which are capable of reacting with the protein's amino groups to form an amide linkage via an acylation reaction. The reactive compound can be introduced at any stage during vesicle formation, but is preferably added to the gas phase prior to vesicle formation. For example, when vesicles are to be made using mechanical or ultrasound cavitation techniques, the reactive compound can be added to the gas phase by bubbling the gas to be used in the formation of the vesicles (starting gas) through a solution of the reactive compound into the gas phase. The resultant gas mixture, which now contains the starting gas and the reactive compound, is then used to form vesicles. The vesicles are preferably formed by sonication of human serum albumin in the presence of a gas mixture, as described in U.S. Patent No. 4,957,656, the disclosures of which are hereby incorporated herein by reference in their entirety.

Suitable fluorine containing alkyl esters and acyl halides for use as stabilizing materials and/or vesicle forming materials in the present invention include, for example, diethyl hexafluoroglutarate, diethyl tetrafluorosuccinate, methyl heptafluorobutyrate, ethyl heptafluorobutyrate, ethyl pentafluoropropionate, methyl pentafluoropropionate, ethyl perfluorooctanoate, methyl perfluorooctanoate, nonafluoropentanoyl chloride, perfluoropropionyl chloride, hexafluoroglutaryl chloride and heptafluorobutyryl chloride.

Other fluorine containing reactive compounds can also be synthesized and used as the stabilizing materials and/or vesicle forming materials in the present invention, including, for example, aldehydes, isocyanates, isothiocyanates, epoxides, sulfonyl halides, anhydrides, acid halides and alkyl sulfonates, which contain perfluorocarbon moieties, including $-CF_3$, $-C_2F_5$, $-C_3F_4$ and $-C(CF_3)_3$. These reactive compounds can be used to introduce fluorine moieties into any of the aforementioned stabilizing materials by choosing a combination which is appropriate to achieve covalent attachment of the fluorine moiety.

Fluorine may preferably be introduced in quantities sufficient to decrease the permeability of the vesicle to the aqueous environment. This may advantageously result in a slower rate of gas exchange with the aqueous environment which is evidenced by enhanced pressure resistance. Although the specific amount of fluorine necessary to stabilize the vesicle will depend on a variety of factors, including the components of the vesicle and the gas contained therein, after introduction of fluorine the vesicle will preferably contain 0.01 to 20% by weight, and more preferably about 1 to 10% by weight fluorine.

It may be desirable to use a fluorinated liquid, especially a liquid perfluorocarbon or a liquid perfluoroether, which are liquids at the temperature of use, including, for example, the *in vivo* temperature of the human body, to assist or enhance the stability of the compositions of the present invention. Suitable liquid perfluorocarbons and liquid perfluoroethers include, for example, perfluoroheptane, perfluorooctane, perfluorononane, perfluorodecane, perfluorodecalin, perfluorododecalin, perfluorooctyliodide, perfluorooctylbromide, perfluorotripropylamine, perfluorotributylamine, perfluorobutylethyl ether, bis(perfluoroisopropyl) ether and bis(perfluoropropyl) ether. Among these, perfluorooctylbromide is preferred. Although not intending to be bound by any theory of operation, in the case of vesicle compositions, the fluorinated liquid compound may be situated at the interface between the gas and the membrane or wall surface of the vesicle. Thus, an additional stabilizing layer of fluorinated liquid compound may be formed on the internal surface of the stabilizing composition which may also prevent any gas from diffusing through the vesicle membrane.

Other surfactants which may also be used in the compositions of the present invention are partially fluorinated phosphocholine surfactants. In these fluorinated surfactants, the dual alkyl compounds may be fluorinated at the terminal alkyl chains and the proximal carbons may be hydrogenated.

Still other suitable surfactants that may be used in the compositions of the present invention include, for example, compounds identified as TRITON-X® (*e.g.*, octoxynols; available from Rohm & Haas, Philadelphia, PA), BRIJ® (*e.g.*, polyoxyethylene ethers; available from ICI-Americas, Wilmington, DE), and FLUORADS® (*e.g.*, fluorochemical surfactants; available from 3M, St. Paul, MN).

Preferred embodiments of the invention may involve vesicles which comprise three components: (1) a neutral lipid, for example, a nonionic or zwitterionic lipid, (2) a negatively charged lipid, and (3) a lipid bearing a stabilizing material, for example, a hydrophilic polymer. Preferably, the amount of the negatively charged lipid will be greater than about 1 mole % of the total lipid present, and the amount of lipid bearing a hydrophilic polymer will be greater than about 1 mole % of the total lipid present. Exemplary and preferred negatively charged lipids include phosphatidic acids. The lipid bearing a hydrophilic polymer will desirably be a lipid covalently linked to the polymer, and the polymer will preferably have a weight average molecular weight of from about 400 to about 100,000. Suitable hydrophilic polymers are preferably selected from the group consisting of polyethylene glycol (PEG), polypropylene glycol, polyvinyl alcohol, and polyvinyl pyrrolidone and copolymers thereof, with PEG polymers being preferred. Preferably, the PEG polymer has a

molecular weight of from about 1000 to about 7500, with molecular weights of from about 2000 to about 5000 being more preferred. The PEG or other polymer may be bound to the lipid, for example, DPPE, through a covalent bond, such as an amide, carbamate or amine linkage. In addition, the PEG or other polymer may be linked to a targeting ligand, or other phospholipids, with a covalent bond including, for example, amide, ester, ether, thioester, thioamide or disulfide bonds. Where the hydrophilic polymer is PEG, a lipid bearing such a polymer will be said to be "pegylated." In preferred form, the lipid bearing a hydrophilic polymer may be DPPE-PEG, including, for example, DPPE-PEG5000, which refers to DPPE having a polyethylene glycol polymer of a mean weight average molecular weight of about 5000 attached thereto (DPPE-PEG5000). Another suitable pegylated lipid is distearoylphosphatidylethanolamine-polyethylene glycol 5000 (DSPE-PEG5000).

In preferred embodiments, the lipid compositions may include about 77.5 mole % DPPC, 12.5 mole % of DPPA, and 10 mole % of DPPE-PEG5000. Also preferred are compositions which comprise about 80 to about 90 mole % DPPC, about 5 to about 15 mole % DPPA and about 5 to about 15 mole % DPPE-PEG5000. Especially preferred are compositions which comprise DPPC, DPPA and DPPE-PEG5000 in a mole % ratio of 82:10:8, respectively. DPPC is substantially neutral, since the phosphatidyl portion is negatively charged and the choline portion is positively charged. Consequently, DPPA, which is negatively charged, may be added to enhance stabilization in accordance with the mechanism described above. DPPE-PEG provides a pegylated material bound to the lipid membrane or skin of the vesicle by the DPPE moiety, with the PEG moiety free to surround the vesicle membrane or skin, and thereby form a physical barrier to various enzymatic and other endogenous agents in the body whose function is to degrade such foreign materials. The DPPE-PEG may provide more vesicles of a smaller size which are safe and stable to pressure when combined with other lipids, such as DPPC and DPPA, in the given ratios. It is also theorized that the pegylated material, because of its structural similarity to water, may be able to defeat the action of the macrophages of the human immune system, which would otherwise tend to surround and remove the foreign object. The result is an increase in the time during which the stabilized vesicles may function as contrast media.

The terms "stable" or "stabilized" mean that the vesicles may be substantially resistant to degradation, including, for example, loss of vesicle structure or encapsulated photoactive agent, gas, gaseous precursor and/or targeting ligand, for a useful period of time. Typically, the vesicles employed in the present invention have a desirable shelf life, often retaining at least about 90% by volume of its original structure for a period of at least about two to three weeks under normal ambient conditions. In preferred form, the vesicles are desirably stable for a period of time of at least about 1 month, more preferably at least about 2 months, even more preferably at least about 6 months, still more preferably about 18 months, and yet more preferably up to about 3 years. The vesicles described herein, including gas and/or gaseous precursor filled vesicles, may also be stable even under adverse conditions, such as temperatures and pressures which are above or below those experienced under normal ambient conditions.

The stabilizing materials and/or vesicles used in the present invention may be controlled according to size, solubility and heat stability by choosing from among the various additional or auxiliary stabilizing materials described herein. These materials can affect the parameters of the vesicles, especially vesicles formulated from lipids, not only by their physical interaction with the membranes, but also by their ability to modify the viscosity and surface tension of the surface of the vesicle. Accordingly, the vesicles used in the present invention may be favorably modified and further stabilized, for example, by the addition of one or more of a wide variety of (i) viscosity modifiers, including, for example, carbohydrates and their phosphorylated and sulfonated derivatives; polyethers, preferably with molecular weight ranges between 400 and 100,000; and di- and trihydroxy alkanes and their polymers, preferably with molecular weight ranges between 200 and 50,000; (ii) emulsifying and/or solubilizing agents including, for example, acacia, cholesterol, diethanolamine, glyceryl monostearate, lanolin alcohols, lecithin, mono- and di-glycerides, mono-ethanolamine, oleic acid, oleyl alcohol, poloxamer, for example, poloxamer 188, poloxamer 184, poloxamer 181, PLURONICS® (BASE, Parsippany, NJ), polyoxyethylene 50 stearate, polyoxyl 35 castor oil, polyoxyl 10 oleyl ether, polyoxyl 20 cetostearyl ether, polyoxyl 40 stearate, polysorbate 20, polysorbate 40, polysorbate 60, polysorbate 80, propylene glycol diacetate, propylene glycol monostearate, sodium lauryl sulfate, sodium stearate, sorbitan mono-laurate, sorbitan mono-oleate, sorbitan mono-palmitate, sorbitan monostearate, stearic acid, trolamine, and emulsifying wax; (iii) suspending and/or viscosity-increasing agents, including, for example, acacia, agar, alginic acid, aluminum mono-stearate, bentonite, magma, carbomer 934P, carboxymethylcellulose, calcium and sodium and sodium 12, carrageenan, cellulose, dextran, gelatin, guar gum, locust bean gum, veegum, hydroxyethyl cellulose, hydroxypropyl methyl-cellulose, magnesium-aluminum-silicate, ZEOLITES®, methylcellulose, pectin, polyethylene oxide, povidone, propylene glycol alginate, silicon dioxide, sodium alginate, tragacanth, xanthan gum, $\alpha$-d-gluconolactone, glycerol and mannitol; (iv) synthetic suspending agents, such as polyethylene glycol (PEG), polyvinylpyrrolidone (PVP), polyvinylalcohol (PVA), polypropylene glycol (PPG), and polysorbate; and (v) tonicity raising agents which stabilize and add tonicity, including, for example, sorbitol, mannitol, trehalose, sucrose, propylene glycol and glycerol.

The present stabilizing materials and/or vesicles are desirably formulated in an aqueous environment which can induce the stabilizing material (*e.g.*, a lipid because of its hydrophobic-hydrophilic nature) to form vesicles, which may be the most stable configuration which can be achieved in such an environment. The diluents which can be

employed to create such an aqueous environment include, for example, water, including deionized water, normal saline, physiological saline, or water containing one or more dissolved solutes, such as salts or sugars.

Proteins (including peptides) useful as carriers in accordance with the present invention include molecules comprising, and preferably consisting essentially of, α-amino acids in peptide linkages. A wide variety of proteins may be employed as carriers in the present invention, including natural, synthetic, or semi-synthetic proteins. Included within the term "protein" are globular proteins, such as albumins, globulins and histories, and fibrous proteins such as collagens, elastins and keratins. Also included are "compound proteins", wherein a protein molecule is united with a nonprotein molecule, such as nucleproteins, mucoproteins, lipoproteins, and metalloproteins. Preferable proteinaceous macromolecules include for example, albumin, collagen, polyarginine, polylysine, polyhistidine, γ-globulin and β-globulin, with albumin, polyarginine, polylysine, and polyhistidine being more preferred. Fluorinated peptides and synthetic pseudopeptides are also useful as carriers. Fluorinated peptides useful in the present invention include those described in Lohrmann, U.S. Patent No. 5,562,892, the disclosures of which are hereby incorporated herein by reference in their entirety. Cationic peptides may also be usefully employed as carriers in the present invention. Various peptides suitable for use in the present invention will be apparent to one skilled in the art based on the present disclosure.

The compositions of the present invention may also involve vesicles or other organized stable forms formulated from proteins, peptides and/or derivatives thereof. Vesicles which are formulated from proteins and which would be suitable for use in the methods of the present invention are described, for example, in Feinstein, U.S. Patent Nos. 4,572,203, 4,718,433, and 4,774,958, and Cerny et al., U.S. Patent No. 4,957,656, all of the disclosures of each of which are hereby incorporated by reference in their entirety. Other protein-based vesicles, in addition to those described in the aforementioned patents, would be apparent to one of ordinary skill in the art, once armed with the present disclosure.

Metal ions may also be employed as carriers in the present invention. Suitable metal ions include calcium ions, magnesium ions, zinc ions, and the like, as well as a wide variety of inorganic compounds. Other suitable metal ions as well as other suitable inorganic compounds will be readily apparent to those skilled in the art once armed with the present invention.

Other useful agents that may be employed in the carrier of the present invention include osmotic agents, anti-microbials, viscosity raising agents, suspending agents, humectants and anti-humectants, depending upon the particular formulation desired.

One or more emulsifying or stabilizing agents may also be employed as or be included in the carrier. These agents help to maintain the size of any discrete units (*e.g.*, liquid droplets, particles, gas bubbles, etc.) of the organic halide and/or compounds to be delivered that may have formed the composition. The size of these discrete units will generally affect the size of any resultant gas bubbles that may form from any gaseous precursors. The emulsifying and stabilizing agents also may be used to generally coat or stabilize the organic halides, compounds to be delivered, etc. Stabilization is desirable to maximize the intracellular delivery effect. Although stabilization is preferred, this is not an absolute requirement. Because any gas resulting from organic halide gaseous precursors is generally more stable than air, they may still be designed to provide useful delivery means; for example, they may pass through the pulmonary circulation following peripheral venous injection, even when not specifically stabilized by one or more coating or emulsifying agents. One or more coating or stabilizing agents is preferred however, as are flexible stabilizing materials. Also, it should be noted that compositions stabilized by polysaccharides, gangliosides, and polymers are generally more effective than those stabilized by albumin and other proteins. Also, liposomes prepared using aliphatic compounds are preferred, since microspheres stabilized with these compounds are much more flexible and stable to pressure changes.

The carrier of the invention may also comprise a wide variety of viscosity modifiers, including and not limited to carbohydrates and their phosphorylated and sulfonated derivatives; polyethers, preferably with molecular weights ranging from about 400 to about 8000; di- and trihydroxy alkanes and their polymers, preferably with molecular weights ranging from about 800 to about 8000. Glycerol propylene glycol, polyethylene glycol, polyvinyl pyrrolidone, and polyvinyl alcohol may also be useful as carriers or stabilizers in the present invention. Particles which are porous or semi-solid such as hydroxyapatite, metal oxides and coprecipitates of gels, *e.g.*, hyaluronic acid with calcium may be used and may formulate a center or nidus to stabilize compositions of the invention.

Emulsifying and/or solubilizing agents may also be used in a carrier, particularly in conjunction with lipids or liposomes. Such agents include and are not limited to, acacia, cholesterol, diethanolamine, glyceryl monostearate, lanolin alcohols, lecithin, mono- and di-glycerides, mono-ethanolamine, oleic acid, oleyl alcohol, poloxamer, polyoxyethylene 50 stearate, polyoxyl 35 castor oil, polyoxyl 10 oleyl ether, polyoxyl 20 cetostearyl ether, polyoxyl 40 stearate, polysorbate 20, polysorbate 40, polysorbate 60, polysorbate 80, propylene glycol diacetate, propylene glycol monostearate, sodium lauryl sulfate, sodium stearate, sorbitan, sorbitan monolaurate, sorbitan monooleate, sorbitan monopalmitate, sorbitan monostearate, stearic acid, trolamine, and emulsifying wax. All lipids with perfluoro fatty acids as a component of the lipid in lieu of the saturated or unsaturated hydrocarbon fatty acids found in lipids of plant or animal origin may be used. Suspending and/or viscosity-increasing agents that may be particularly useful with lipid or liposome solutions include but are not limited to, acacia, agar, alginic acid, aluminum monostearate, bentonite, magma, carbomer

934P, carboxymethylcellulose, calcium and sodium and sodium 12, glycerol, carrageenan, cellulose, dextrin, gelatin, guar gum, hydroxyethyl cellulose, hydroxypropyl methylcellulose, magnesium aluminum silicate, methylcellulose, pectin, polyethylene oxide, polyvinyl alcohol, povidone, propylene glycol, alginate, silicon dioxide, sodium alginate, tragacanth, and xanthum gum. A preferred product of the present invention incorporates lipid as a mixed solvent system in a ratio of 8:1:1 or 9:1:1 normal saline:glycerol:propylene glycol.

The amount of carrier material employed in connection with the subject invention may vary, as one skilled in the art will recognize upon being placed in possession of the subject disclosure, and may be dependent on such factors as the particular carrier used, the type and nature of the compound to be delivered, the age, weight, cells or patient (animal) to be treated, the particular diagnostic, therapeutic or other application intended (including the disease state, if any, to be treated), and the organic halide (if any) used. Generally, smaller amounts of carrier are employed, and increased until the desired delivery result is obtained. Representative amounts are set forth in the examples herein. Of course, higher or lower amounts may be employed, as will be recognized by the skilled artisan.

***Compounds to be Delivered***

Compounds to be delivered to target cells include bioactive agents such as, for example, prodrugs, diagnostic agents, pharmaceutical agents, drugs, synthetic organic molecules, proteins, peptides, vitamins, steroids, steroid analogs and genetic material.

In a preferred embodiment of the present invention, the compound to be delivered may be genetic material, including genetic material directed to TNF. For example, the compound(s) to be delivered to target cells may be TNF inhibitors which bind the tumor necrosis factor receptor (TNF-R) while preferably avoiding the promotion of inflammation. Generally speaking, TNF-R may be found in elevated levels on cells implicated with the out of control immune response associated with rheumatoid arthritis (RA). These receptors may m turn be activated by TNF binding which may further proceed to promote inflammation. Thus, genes encoding mutant TNF may be delivered to cells. Exemplary TNF molecules which may be included in the present compositions, and the preparation of such molecules, are described, for example, in Perez, *et al., Cell*, **1990**, *63*, 251-258, Nakamura, *et al., Int J. Cancer,* **1991**, *48*, 744-748, Barbara, *et al., EMBO J.,* **1994**, *13*, 843-850, and Nakamura, *et al., Agric. Biol. Chem.*, **1990**, *54*, 3241-3250, the disclosures of each of which are hereby incorporated herein by reference in their entirety.

In another preferred embodiment of the invention, mutant TNF-R may be used to interfere with promotion of inflammation. For example, nucleic acid molecules encoding a fusion protein comprising the TNF-binding portion of TNF-R and the $F_C$ region of an immunoglobulin such as IgG may be used. Thus, expression of the fusion protein on the surface of a cell may advantageously result in TNF binding without the initiation of an immune response and subsequent inflammation caused by the particular cell targeted. Exemplary TNF-R molecules which may be included in the present compositions, and the preparation of such molecules, are described, for example, in Barbara, *et al., EMBO J.,* **1994**, *13*, 843-850, Stroka, *et al., Transplant Proc*., **1997**, *29*, 882, Cope, *et al., J. Rheum.,* **1995**, 22, 383-384, and Yamagishi, *et al., Preotein Engineering,* **1990**, *3*, 713-720, the disclosures of each of which are hereby incorporated herein by reference in their entirety.

In another preferred embodiment of the invention, nucleic acid molecules encoding mutant IL-1 and mutant gamma interferon (IFN-γ) and their receptors may be used. Both IL-1 and IFN-γ are involved in inflammation. Thus, expression of mutant IL-1, IFN-γ, and receptors for each can be used to interfere with inflammation. Exemplary IL-1β molecules which may be included in the present compositions, and the preparation of such molecules, are described, for example, in Chrunyk, *et al., J. Biol. Chem.,* **1993**, *268*, 18053-18061, the disclosures of which are hereby incorporated herein by reference in their entirety. Exemplary IFN-γ receptor molecules which may be included in the present compositions, and the preparation of such molecules, are described, for example, in Schreiber, *et al., Int. J. Immunopharmac.*, **1992**, *14*, 413-419 and Farrar, *et al., J. Biol. Chem*., **1991**, *266*, 19626-19635, the disclosures of each of which are hereby incorporated herein by reference in their entirety. Exemplary IFN-γ molecules which may be included in the present compositions, and the preparation of such molecules, are described, for example, in Haelewyn, *et al.*, *Biochem. J*., **1997**, *324*, 591-595 and Wang, *et al., Science in China, Series B (Chem and Life Sciences and Earth Sci.)*, **1992**, *35*, 84-91, the disclosures of each of which are hereby incorporated herein by reference in their entirety.

The compounds to be delivered to target cells may also be ribozymes, which are enzymes that generally target and degrade nucleic acid molecules. Exemplary ribozymes which may be included in the present compositions, and the preparation of such molecules, are described, for example, in Hodgson, *et al., Nuc. Acids Res*., **1994**, 22, 1620-1625, Doudna, J.A., in *Methods In Molecular Biology*, **1997**, Turner, P.C. Ed., Vol 74, Humana Press, Totowa, NJ, and Bartel, *et al*., Science, **1993**, *261*, 1411-1418, the disclosures of each of which are hereby incorporated herein by reference in their entirety.

In another preferred embodiment, the compounds to be delivered to target cells may be antisense oligonucleotides directed to TNF, TNF-R, IL-1, IL-1R, IFN-γ, and IFN-γR. Exemplary oligonucleotides directed to TNF-R

include, for example, the compounds described in Pampfer, *et al*., *Biol. Reproduct*., **1995**, *52*, 1316-1326 and Ojwang, *et al., Biochem.*, **1997**, 36, 6033-6045, the disclosures of each of which are hereby incorporated herein by reference in their entirety. Exemplary oligonucleotides directed to IL-1 include, for example, the compounds described in Burch, *et al., J. Clin. Invest.*, **1991**, *88*, 1190-1196 and Maier, *et al.*, *Science*, **1990**, *249*, 1570-1574, the disclosures of each of which are hereby incorporated herein by reference in their entirety. Exemplary oligonucleotides directed to TNF include, for example, the compounds described in Wu, *et al., Proc. Am. Assoc. Cancer Res*., **1993**, *34*, 439, the disclosures of which are hereby incorporated herein by reference in their entirety. Exemplary oligonucleotides directed to IFN-$\gamma$ include the compounds described, for example, in Boeve, *et al., J. Leuk. Biol*., **1994**, *55*, 169-174, the disclosures of which are hereby incorporated herein by reference in their entirety. Exemplary oligonucleotides directed to IFN-$\gamma$ receptor include, for example, the compounds described in Doherty, *et al., J. Surg. Res*., **1996**, *64*, 68-74, the disclosures of which are hereby incorporated herein by reference in their entirety.

In another preferred embodiment, the compounds to be delivered to target cells may be telomerase compounds. Exemplary telomerase compounds which may be included in the present compositions, and the preparation of such compounds, include those described, for example, in U.S. Patent 5,698,686, the disclosures of which are hereby incorporated herein by reference in their entirety.

The compositions and methods of the present invention permit the delivery of sequences coding for the gene expression of a variety of proteins, and antisense sequences which block gene expression of a variety of proteins. As a result, a number of inflammatory diseases may be prevented and/or treated with the transfection methods of the present invention.

Intracellular delivery and transfection in accordance with the methods of the present invention may be performed *in vivo*, *ex vivo*, and *in vitro*. Included within the above three methods is human gene therapy including methods which involve excising cells to be treated from a patient. The cells may be treated with an appropriate nucleotide sequence and transfection with ultrasound may be carried out in cell culture. The transfected cells may be analyzed for gene expression of the appropriate protein. The successfully transfected cells, measured by gene expression, may then be returned to the body of the patient. Transfection with ultrasound may thereby desirably and advantageously result in the treatment of diseases by gene therapy. Diseases to be treated with the methods of the present invention include and are not limited to inflammatory diseases such as, for example, rheumatoid arthritis. Many other diseases may, of course, be treated with the methods of the present invention, as will be apparent to the skilled artisan once armed with the teachings of the present disclosure, and the treatment of all such diseases are to be considered within the scope of the present methods.

In addition to a coding sequence or antisense sequence, the nucleotide sequence administered to cells may have additional sequences to assist in the expression of the sequence. Suitable expression vectors, promoters, enhancers, and other expression control elements are known in the art and may be found, for example, in Sambrook et al., *Molecular Cloning: A Laboratory Manual*, Second Edition, Cold Spring Harbor Laboratory Press, Cold Spring Harbor, NY (1989), the disclosures of which are hereby incorporated herein by reference, in their entirety. Promoters such as SV40, RSV, CMV, cd5k, IL5R $\alpha$ pgk-1, sr$\alpha$, TK, and the like may also be useful in the present invention. Transcription and/or translation control elements may be operatively linked to the sequence. For example, in an upstream position, a promoter may be followed by a translation initiation signal, comprising a ribosome binding site and an initiation codon, and in a downstream position may be a transcription termination signal. The transcription and translation control elements may be ligated in any functional combination or order. The transcription and translation control elements used in any particular embodiment of the invention are preferably chosen with reference to the type of cell into which the expression vector will be introduced, so that an expression system is created. The selection of promoters, enhancers, and other expression control elements and the preparation of expression vectors suitable for use in the present invention will be well within the ambit of one skilled in the art once armed with the present disclosure. Also, introduction of the expression vector incorporating a sequence into a host cell may be performed in a variety of ways known in the art. Expression vectors which may be utilized in the present compositions and which are commercially available from Promega (Madison, WI) include, for example, SV40 (Simian Virus 40), CMV (Human cytomegalovirus), RSV (Rous Sarcoma virus) and adenovirus, as well as various plasmids including pBr322. Additional suitable expression vectors can be obtained from Invitrogen, Inc. (Carlsbad, CA) and Clontech (Palo Alto, CA).

Mammalian cells may be primed to be more susceptible to uptake of DNA for gene therapy by the addition of various media, buffers, and chemicals known to those of skill in the art and set forth in Sambrook, *supra*, the disclosures of which are hereby incorporated herein in their entirety.

Administration of nucleotide sequences *in vivo* may include, if desired, more than one sequence. For example, a single carrier may contain more than one sequence or carriers containing different sequences may be co-administered. In addition, one sequence may be delivered in a carrier and another naked sequence coadministered. Additional sequences, such as promoter sequences, may be delivered together with a sequence for therapeutic delivery, to increase expression thereof. For example, a heat shock protein nucleic acid sequence is an example of an upregulating gene sequence which may be used to increase expression of a second gene sequence.

A wide variety of other compounds may be delivered to cells using the compositions of the present invention including, for example, pharmaceuticals, drugs, diagnostic agents, synthetic organic molecules, proteins, peptides, vitamins, steroids, and genetic material, as well as other bioactive agents. Exemplary of such other compounds include, for example, the following: mitotic inhibitors such as the vinca alkaloids; radiopharmaceuticals such as radioactive iodine, phosphorus and cobalt isotopes; hormones such as progestins, estrogens and antiestrogens; anti-helminthics, antimalarials and antituberculosis drugs; biologicals such as immune sera, antitoxins and antivenins; rabies prophylaxis products; bacterial vaccines; viral vaccines; aminoglycosides; respiratory products such as xanthine derivatives, theophylline and aminophylline; thyroid therapeutics such as iodine salts and anti-thyroid agents; cardiovascular products including chelating agents and mercurial diuretics and cardiac glycosides; glucagon; blood products such as parenteral iron, hemin, hematoporphyrins and their derivatives; targeting ligands such as peptides, antibodies, and antibody fragments; biological response modifiers such as muramyl dipeptide, muramyl tripeptide, microbial cell wall components, lymphokines (e.g. bacterial endotoxin such as lipopolysaccharide and macrophage activation factor); subunits of bacteria (such as Mycobacteria and Comebacteria); the synthetic dipeptide N-acetyl-muramyl-L-alanyl-D-isoglutamine; antifungal agents such as ketoconazole, nystatin, griseofulvin, flucytosine (5-fc), miconazole, and amphotericin B; toxins such as ricin; immunosuppressants such as cyclosporins; and antibiotics such as β-lactam and sulfazecin; hormones such as growth hormone, melanocyte stimulating hormone, estradiol, beclomethasone dipropionate, betamethasone, betamethasone acetate, betamethasone sodium phosphate, betamethasone disodium phosphate, betamethasone sodium phosphate, cortisone acetate, dexamethasone, dexamethasone acetate, dexamethasone sodium phosphate, flunisolide, hydrocortisone, hydrocortisone acetate, hydrocortisone cypionate, hydrocortisone sodium phosphate, hydrocortisone sodium succinate, methylprednisolone, methylprednisolone acetate, methylprednisolone sodium succinate, paramethasone acetate, prednisolone, prednisolone acetate, prednisolone sodium phosphate, prednisolone tebutate, prednisone, triamcinolone, triamcinolone acetonide, triamcinolone diacetate, triamcinolone hexacetonide, fludrocortisone acetate, oxytocin, and vasopressin, as well as their derivatives; vitamins such as cyanocobalamin neionic acid; retinoids and derivatives such as retinol palmitate and α-tocopherol; peptides and enzymes such as manganese superoxide dismutase and alkaline phosphatase; anti-allergens such as amelexanox; anti-coagulation agents such as phenprocoumon and heparin; tissue plasminogen activators (TPA), streptokinase, and urokinase; circulatory drugs such as propranolol; metabolic potentiators such as glutathione; antibiotics such as p-aminosalicyclic acid, isoniazid, capreomycin sulfate cycloserine, ethambutol hydrochloride ethionamide, pyrazinamide, rifampin, streptomycin sulfate dapsone, chloramphenicol, neomycin, ceflacor, cefadroxil, cephalexin, cephadrine erythromycin, clindamycin, lincomycin, amoxicillin, ampicillin, bacampicillin, carbenicillin, dicloxicillin, cyclacillin, picloxicillin, hetacillin, methicillin, nafcililn, oxacillin, penicillin (G and V), ticarcillin rifampin and tetracycline; antivirals such as acyclovir, DDI, Foscarnet, zidovudine, ribavirin and vidarabine monohydrate; antianginals such as diliazem, nifedipine, verapamil, erythritol tetranitrate, isosorbide dinitrate, nitroglycerin (glyceryl trinitrate) and pentaerythritol tetranitrate; anti-inflammatories such as difluisal, ibuprofin, indomethacin, meclofenamate, mefenamic acid, naproxen, oxyphenbutazone, phenylbutazone, piroxicam, sulindac, tolmetin, aspirin, and salicylates; antiprotozoans such as chloraquine, hydroxychloraquine, metranidazole, quinine and meglumine antimonate; antirheumatics such as penicillamine; narcotics such as paregoric; opiates such as codeine, heroin, methadone, morphine, and opium; cardiac glycosides such as deslanoside, digitoxin, digoxin, digitalin, and digitalis; neuromuscular blockers such as atracurium nesylate, gallamine triethiodide, hexaflorenium bromide, metrocurine iodide, pancurium bromide, succinylcholine chloride (suxamethonium chloride), tubocurarine chloride and vecuronium bromide; sedatives such as amorbarital, amobarbital sodium, aprobarbital, butabarbital sodium, choral hydrate, ethchlorvynol, ethinamate, flurazepam hydrochloride, glutethimide, methotrimeprazine hydrochloride, methyprylon, midazolam hydrochloride, paraldehyde, pentobarbital, pentobarbital sodium, secobarbital sodium, tulbutal, temazepam and trizolam; local anesthetics such as bupivacaine hydrochloride, chloroprocaine hydrochloride, etidocaine hydrochloride, lidocaine hydrochloride, mepivacaine hydrochloride, procaine hydrochloride, and tetracaine hydrochloride; general anaesthetics such as droperidol, etamine hydrochloride, methohexital sodium and thiopental sodium; antineoplastic agents such as methotrexate, fluorouracil, adriamycin, mitomycin, ansamitomycin, bleomycin, cystein arabinoside, arabinosyl adenine, mercaptopolylysine, vincristine, busulfan, chlorambucil, azidothymidine, melphalan (*e.g.* PAM, L-PAM or phenylalanine mustard), mercaptopurine, mitotane, procarbazine hydrochloride dactinomycin (actinomycin D), danorubicin hydrochloride, dosorubicin hydrochloride, taxol, plicamycin (mithramycin), aminoglutethimide, estramustine phosphate sodium, flutamide, leuprolide acetate, leuprolide acetate, megestrol acetate, tamoxifen citrate, testolactone, trilostane, amsacrine (m-AMSA), asparaginase, etoposide (VP-16), interferon α-2a, interferon α-2b, teniposide (VM-26), vinblastine sulfate (VLB), vincristine sulfate, hydroxyurea, procarbaxine, and dacarbazine.

The useful dosage of compound to be administered or delivered, as well as the mode of administration, may vary depending upon, for example, the type and nature of the compound to be delivered, the age, weight, cells or patient (animal) to be treated, the particular diagnostic, therapeutic, or other application intended (including the disease state, if any, to be prevented and/or treated), and the organic halide (if any) and carrier (if any) employed. Typically, dosage may be initiated at lower levels and may be increased until the desired therapeutic effect is achieved. The desired dosage, including any therapeutically or diagnostically effective dosage amounts, will be well within the ambit of one skilled in the art, armed with the prevailing medical literature and with the present disclosure. Representative amounts are provided in the examples herein. Of course, higher or lower amounts may be employed, as will be recognized by the skilled artisan.

A wide variety of different methods may be used to mix the organic halide, compound to be delivered, and/or carrier, and incorporate the compound to be delivered with or into any organic halide and/or carrier. Methods include shaking by hand, vortexing, mechanical shaking (*e.g*. with an Espe CapMix, Espe Medizin-Dental GMBH, Seefeld, Germany), extruder (*e.g*. with a Lipex Biomembranes Extruder Device, Vancouver, B.C., Canada), microemulsification (*e.g*. with a Microfluidizer, Microfluidics Corp., Newton, MA), mixing with static in line mixers (Cole-Parmer Instrument Co., Vernon Hills, IL), spray drying (*e.g*. with a Bucchi spray dryer, Brinkmann Ind., Inc., Westbury, MA), mechanical stirring/mixing (e.g. with a Silverson Mixer, Silverson Machines, Ltd., Waterside Chesham Bucks, England) and sonication. In general it is desirable to mix the carrier (*e.g*. lipids such as DPEPC and DOPE) together with the organic halide prior to adding the compound to be delivered (*e.g*., DNA). After adding the compound to be delivered, an association between the compoud, carrier and organic halide may desirably form. If desired, additional mixing may then be performed by one of the above techniques. In other situations involving, for example, calcium precipitation, the compound to be delivered (*e.g*. DNA), organic halide, and cations may be added together with one or more stabilizing agents to form precipitates of DNA/carrier/organic halide in a single step process. Again, one of a variety of mixing techniques as described above may be employed to decrease the size of the resultant particles.

The carriers may be combined with the compound to be delivered and the organic halide in varying amounts and percentages, as will be understood by those skilled in the art once armed with the present disclosure. Typically, smaller amounts of all components of the composition may be employed, and may be increased selectively in increments until the desired delivery effect is achieved. Generally, when the compound to be delivered is employed with a carrier, the ratio of organic halide and any carrier to the compound to be delivered may range from about 6:1 to about 1:6, and all combinations and subcombinations of ranges therein. Preferably, the carrier to compound to be delivered ratio is about 6:1. Other exemplary ratios are provided by the examples herein. Of course, other ratios can be suitably employed over a wide variety of ranges as desired, as will be recognized by the skilled artisan, and all such ratios are within the scope of the present invention.

The resulting composition may be stored as a lyophilized, or freeze dried, material for inhalation or hydration prior to use or as a preformed suspension. Cryopreservatives known to skilled artisans once armed with the present disclosure may be used in the lyophilized form of the composition. To prevent agglutination or fusion of vesicles as a result of lyophilization, it may be useful to include additives which prevent such fusion or agglutination from occurring. Additives which may be useful include sorbitol, mannitol, sodium chloride, glucose, trehalose, polyvinylpyrrolidone and poly(ethylene glycol) (PEG), for example, PEG polymers having a molecular weight of from about 400 to about 10,000, with PEG polymers having molecular weights of from about 1000 to about 5000 being preferred including, for example, PEG polymers having molecular weights of about 1000, about 3000 (such as PEG3350) and about 5000 being more preferred. These and other additives are described in the literature, such as in the U.S. Pharmacopeia, USP XXII, NF XVII, The United States Pharmacopeia, The National Formulary, United States Pharmacopeial Convention Inc., 12601 Twinbrook Parkway, Rockville, MD 20852, the disclosures of which are hereby incorporated herein by reference in their entirety. Lyophilized preparations generally have the advantage of greater shelf life. As noted above, if desired, the lyophilized composition may be (and preferably is) rehydrated prior to use.

The lipids used, especially in connection with vesicles, are preferably flexible. This means, in the context of the present invention, that the vesicles can alter their shape, for example, to pass through an opening having a diameter that is smaller than the diameter of the vesicle.

Also useful in the vesicles of the present invention are a wide variety of surfactants (*i.e*., surface-active agents), including polyoxyalkylene fatty acid esters (such as polyoxyethylene fatty acid esters), polyoxyalkylene fatty alcohols (such as polyoxyethylene fatty alcohols), polyoxyalkylene fatty alcohol ethers (such as polyoxyethylene fatty alcohol ethers), polyoxyalkylene sorbitan fatty esters (such as, for example, the class of compounds referred to as TWEEN®, commercially available from ICI Americas, Inc., Wilmington, DE), including poly(oxyethylene) poly(oxypropylene) copolymers (such as Pluronics), polysorbates (such as TWEEN® 20, TWEEN® 40, and TWEEN® 80), polyoxyethylene alcohols (such as Brij), and plasmalogens (the term applied to a number of a group of phospholipids present in platelets that liberate higher fatty aldehydes, *e.g*. palmital, on hydrolysis and may be related to the specialized function of platelets in blood coagulation and plasmalogens are also present in cell membranes of muscle and the myelin sheath of nerve fibers).

Cationic lipids (including those in the form of cationic liposomes) are particularly preferred, especially as employed in an aqueous milieu. A preferred cationic lipid is DPEPC, alone or in admixture with the neutral fusogenic lipid dioleoylphosphatidylethanol-amine (DOPE). Also in a preferred embodiment, the lipid or polymer and organic halide are combined and formulated as an organic halide-filled microsphere, such as a microsphere formed with the lipids dipalmitoylphosphatidylcholine (DPPC), dipalmitoylphosphatidylethanolamine coupled to polyethylene glycol 5000

(DPPE-PEG5000), and dipalmitoylphosphatidic acid (DPPA). DPPC:DPPE-PEG5000:DPPA may be combined in a ratio of about 82%:8%:10% (mole %) or 83%:8%:5%. DPPE-PEG5000 is comprised of DPPE and PEG5000 in a ratio of about 20%:80% (weight %). PEG5000 refers to PEG having an average molecular weight of about 5000. Generally speaking, the lipid and organic halide are preferably combined in a ratio of about 5:1 w/w.

The materials from which the vesicles are constructed are preferably biocompatible lipid, protein, polymer or surfactant materials, and of these, the biocompatible lipids are preferred. In addition, because of the ease of formulation, including the capability of preparing vesicles immediately prior to administration, these vesicles may be conveniently made on site.

The stability of vesicles may be attributable, at least in part, to the materials from which the vesicles are made, including, for example, the lipids, polymers, proteins and/or surfactants described above, and it is often not necessary to employ additional stabilizing materials, although it is optional and may be preferred to do so. In addition to, or instead of, the lipid, protein and/or polymer compounds discussed above, the compositions described herein may comprise one or more other stabilizing materials. Exemplary stabilizing materials include, for example, surfactants and biocompatible polymers. The stabilizing materials may be employed to desirably assist in the formation of vesicles and/or to assure substantial encapsulation of the gases, gaseous precursors and/or bioactive agents. Even for relatively insoluble, non-diffusible gases, such as perfluoropropane or sulfur hexafluoride, improved vesicle compositions may be obtained when one or more stabilizing materials are utilized in the formation of the gas and/or gaseous precursor filled vesicles. These compounds may help improve the stability and the integrity of the vesicles with regard to their size, shape and/or other attributes.

In certain embodiments of the present invention, the compositions may comprise vesicles which may be formulated as emulsions or particles entrapping a central droplet of a liquid perfluorocarbon, such as perfluorohexane or perfluorodecalin. Although an organic halide in the form of a gas may be employed, liquid perfluorocarbons and liquid perfluoroethers add desirable properties such as fusogenicity (*e.g.*, ability to fuse or tendency to bind to a membrane) and effectiveness of the resultant therapeutic delivery vehicles.

The organic halide gaseous precursor materials described herein generally comprise organic halide compounds which are sensitive to changes in temperature. Exemplary of suitable gaseous precursors which are sensitive to changes in temperature are the perfluorocarbons. As the artisan will appreciate, a particular perfluorocarbon may exist in the liquid state when the compositions of the present invention are first made, and are thus used as a gaseous precursor. Alternatively, the perfluorocarbon may exist in the gaseous state when the compositions are made, and are thus used directly as a gas. Whether the perfluorocarbon is used as a liquid or a gas generally depends on its liquid/gas phase transition temperature, or boiling point. For example, a preferred perfluorocarbon, perfluoropentane, has a liquid/gas phase transition temperature (boiling point) of 29.5°C. This means that perfluoropentane is generally a liquid at room temperature (about 25 °C), but is converted to a gas within the human body, the normal temperature of which is about 37°C, which is above the transition temperature of perfluoropentane. Thus, under normal circumstances, perfluoropentane is a gaseous precursor. As a further example, there are the homologs of perfluoropentane, namely perfluorobutane and perfluorohexane. The liquid/gas transition of perfluorobutane is 4°C and that of perfluorohexane is 57°C. Thus, perfluorobutane can be useful as a gaseous precursor, although more likely as a gas, whereas perfluorohexane can be useful as a gaseous precursor because of its relatively high boiling point. As known to one of ordinary skill in the art, the effective boiling point of a substance may be related to the pressure to which that substance is exposed. This relationship is exemplified by the ideal gas law: $PV = nRT$ , where P is pressure, V is volume, n is moles of substance, R is the gas constant, and T is temperature. The ideal gas law indicates that as pressure increases, the effective boiling point increases also. Conversely, as pressure decreases, the effective boiling point decreases.

The organic halides are preferably incorporated in the vesicles irrespective of the physical nature of the composition. Thus, it is contemplated that the organic halides may be incorporated, for example, in vesicles which are formulated from lipids, such as micelles and liposomes. Incorporation of the organic halides in the vesicles may be achieved by using any of a number of methods. For example, in the case of vesicles based on lipids, the formation of gas filled vesicles can be achieved by shaking or otherwise agitating an aqueous mixture which comprises an organic halide and one or more lipids. This promotes the formation of stabilized vesicles within which the organic halide is encapsulated.

In addition, a gaseous organic halide may be bubbled directly into an aqueous mixture of vesicle-forming compounds. Alternatively, a gas instillation method can be used as disclosed, for example, in U.S. Patent Nos. 5,352,435 and 5,228,446, the disclosures of each of which are hereby incorporated herein by reference in their entirety. Suitable methods for incorporating the organic halide in cationic lipid compositions are disclosed also in U.S. Patent No. 4,865,836, the disclosures of which are hereby incorporated herein by reference in their entirety. Other methods would be apparent to one skilled in the art based on the present disclosure. Preferably, the organic halide may be instilled in vesicles alter or during formation thereof.

In preferred embodiments, the organic halides may be incorporated in vesicle compositions, with micelles and liposomes being preferred. Vesicles in which an organic halide is encapsulated are advantageous in that they provide improved reflectivity *in vivo*.

It is preferred that the vesicles be formulated from lipids and optional stabilizing compounds to promote the formation of stable vesicles, as discussed in detail above. Additionally, it is preferred that the vesicles comprise a highly stable organic halide gas as well. The phrase "highly stable gas" refers to a gas which has limited solubility and diffusability in aqueous media. Exemplary highly stable gases include perfluorocarbons since they are generally less diffusible and relatively insoluble in aqueous media. Accordingly, their use may promote the formation of highly stable vesicles.

Compositions employed herein may also include, with respect to their preparation, formation and use, gaseous precursors that can be activated to change from a liquid or solid state into a gas by temperature, pH, light, and energy (such as ultrasound). The gaseous precursors may be made into gas by storing the precursors at reduced pressure. For example, a vial stored under reduced pressure may create a headspace of perfluoropentane or perfluorohexane gas, useful for creating a preformed gas prior to injection. Preferably, the gaseous precursors may be activated by temperature.

As noted above, it is preferred to optimize the utility of the vesicles, especially vesicles formulated from lipids, by using gases of limited solubility. The phrase "limited solubility" refers to the ability of the gas to diffuse out of the vesicles by virtue of its solubility in the surrounding aqueous medium. A greater solubility in the aqueous medium imposes a gradient with the gas in the vesicle such that the gas may have a tendency to diffuse out of the vesicle. A lesser solubility in the aqueous milieu, may, on the other hand, decrease or eliminate the gradient between the vesicle and the interface such that diffusion of the gas out of the vesicle may be impeded. Preferably, the gas entrapped in the vesicle has a solubility less than that of oxygen, that is, about 1 part gas in about 32 parts water. See *Matheson Gas Data Book*, 1966, Matheson Company Inc. More preferably, the gas entrapped in the vesicle possesses a solubility in water less than that of air; and even more preferably, the gas entrapped in the vesicle possesses a solubility in water less than that of nitrogen.

It may be desirable, in certain embodiments, to formulate vesicles from substantially impermeable polymeric materials. In these embodiments, it is generally unnecessary to employ a gas which is highly insoluble. For example, stable vesicles which comprise substantially impermeable polymeric materials may be formulated with gases having higher solubilities, for example, air or nitrogen.

In preferred embodiments of the invention the organic halide is incorporated into the core of a vesicle or in the interior of the vesicle.

The mute of administration of the compositions of the present invention varies depending upon the intended application. For cell culture applications, the composition is typically contacted with the cells by, for example, adding it to the cell culture media or applying it directly to the cells. Advantages of this invention for transfection in cell culture media include high activity in serum containing media and a single step transfection process with higher efficiency transfection than in other more complicated systems. Indeed, the present invention makes it possible to obtain gene expression in cells in which transfection was otherwise impossible or extremely difficult. For *in vivo* administration the composition may simply be injected, such as intravenously, intravascularly, intralymphatically, parenterally, subcutaneously, intramuscularly, intranasally, intrarectally, intraperitoneally, interstitially, into the airways via nebulizer, hyperbarically, orally, topically, or intratumorly, or otherwise administered.

In this regard, the composition may be targeted to coated pits of selected cells and taken up into endosomes via a process of receptor mediated endocytosis. If desired ultrasound energy may be applied to the target tissue to facilitate gene expression. For inhalation the composition may be inhaled via a nebulizer or via an inhaler. Also, oral or rectal routes may be utilized to administer these composition. Transcutaneous application may be accomplished by the use of penetration enhancing agents with or without the application of sonophoresis (*e.g*. low frequency sound in range of 10 to 100 Khz) or iontophoresis. Also interstitial (*e.g*. intratumoral) and subcutaneous injection may be performed to administer the composition.

Also the invention may be practiced with gene gun techniques or electroporation, or in combination with other transfection techniques known in the art. In either case, ultrasound may be applied to the cells before, after, and/or simultaneously with the gene gun or electroporation procedure. The electric fields of electroporation may also be pulsed in tandem with the ultrasound energy to further increase the efficacy of transfection.

The compounds and compositions may, in accordance with the present invention, be administered alone, or together with ultrasound. If ultrasound is employed, it is administered at a frequency and energy level sufficient to assist in inducing the uptake of the compound to the cell. Where organic halide gaseous precursors are employed, the ultrasound may be applied at a frequency and energy level sufficient to convert the organic halide gaseous precursor to a gas. For example, the methods of the present invention may comprise administering a nucleotide sequence (or other compound of interest to be delivered) to a cell and applying ultrasound to the cell for a time effective to induce the uptake of the nucleotide sequence (or other compound). This may result in enhanced delivery of the compound (and expression of the nucleotide sequence, in the case of nucleotide sequence being administered). Ultrasound is carried out at a frequency, energy level, and duty cycle for a therapeutically effective time in which to induce delivery of the

nucleotide sequence. Suitable frequencies, energy levels and duty cycles are disclosed herein, and other ranges will be readily apparent to one skilled in the art once armed with the present disclosure.

### *Targeting Ligands*

As indicated above, a targeting ligand is preferably incorporated into the compositions to facilitate targeting and/or uptake of the compound to be delivered by selected target cells. Exemplary targeting ligands include, for example, proteins, peptides, antibodies, antibody fragments, glycoproteins, carbohydrates, hormones, hormone analogs, lectins, polypeptide, amino acids, sugars, saccharides, vitamins, steroids, steroid analogs, cofactors, bioactive agents, and genetic material.

Generally speaking, peptides which are particularly useful as targeting ligands include natural, modified natural, or synthetic peptides that incorporate additional modes of resistance to degradation by vascularly circulating esterases, amidases, or peptidases. While many targeting ligands may be derived from natural sources, some may be synthesized by molecular biological recombinant techniques and others may be synthetic in origin. Peptides may be prepared by a variety of different combinatorial chemistry techniques as are now known in the art. One very useful method of stabilization of peptide moieties incorporates the use of cyclization techniques. For example, end-to-end cyclization, whereby the carboxy terminus is covalently linked to the amine terminus via an amide bond, may be useful to inhibit peptide degradation and increase circulating half life. Side chain-to-side chain cyclization may also be particularly useful in inducing stability. In addition, an end-to-side chain cyclization may be a useful modification as well. The substitution of an L-amino acid for a D-amino acid in a strategic region of the peptide may also provide resistance to biological degradation. Suitable targeting ligands, and methods for their preparation, will be readily apparent to one skilled in the art, once armed with the teachings of the present disclosure. Although the lengths of the peptides utilized as targeting ligands may vary, peptides having from about 5 to about 15 amino acid residues are generally preferred.

Antibodies may be used as whole antibody or antibody fragments, for example, Fab or Fab'2, either of natural or recombinant origin. The antibodies of natural origin may be of animal or human origin, or may be chimeric (mouse/human). Human recombinant or chimeric antibodies are preferred and fragments are preferred to whole antibody. Antibodies may be used as whole antibody or antibody fragments, for example, Fab or Fab'2, either of natural or recombinant origin. The antibodies of natural origin may be of animal or human origin, or may be chimeric (mouse/human). Human recombinant or chimeric antibodies are preferred and fragments are preferred to whole antibody.

As known to one of ordinary skill in the art, immunoglobulins typically comprise a flexible region which is identified as the "hinge" region. *See, e.g.*, "Concise Encyclopedia of Biochemistry", Second Edition, Walter de Gruyter & Co., pp. 282-283 (1988), the disclosures of which are hereby incorporated herein by reference, in their entirety. Fab' fragments may be linked to the carrier such as, for example, lipids, polymers, proteins and/or vesicles, using the well-defined sites of the thiols of the binge region. This is a preferred region for coupling Fab' fragments as the potential binding site may be remote from the antigen-recognition site. Generally speaking, it may be difficult to utilize the thiols of the hinge group unless they are adequately prepared. As described in Shahinian and Salvias, *Biochimica et Biophysica Acta*, Vol 1239 (1995) 157-167, the disclosures of which are hereby incorporated herein by reference in their entirety, it may be desirable to reduce the thiol groups so that they are available for coupling, for example, to maleimide derivatized linking groups. Examples of reducing agents which may be used include ethanedithiol, mercaptoethanol, mercaptoethylamine or the more commonly used dithiothreitol, commonly referred to as Cleland's reagent. However, as would be apparent to one of ordinary skill in the art, once armed with the teachings of the present applicaiton, care should be exercised when utilizing certain reducing agents, such as dithiothreitol, since overreduction may occur. Discriminating use of reducing agents may be necessary in connection with proteins whose activity or binding capacity may be compromised due to overreduction and subsequent denaturation or conformational change. *See, e.g.*, Shahinian and Salvias, *supra*.

$F(ab')_2$ antibody fragments may be prepared by incubating the antibodies with pepsin (60 μg/ml) in 0.1 M sodium acetate (pH 4.2) for 4 h at 37°C. Digestion may be terminated by adding 2 M Tris (pH 8.8) to a final concentration of 80 mM. The $F(ab')_2$ fragments may then be obtained by centrifugation (10,000 x g. 30 min. 4°C). The supernatant may then be dialyzed at 4°C against 150 mM NaCl, 20 mM phosphate at pH 7.0. This then may be chromatographed on a column of Protein A-Sepharose CL-4B to remove any undigested IgG. The Fab' fragments may then be prepared by extensively degassing the solutions and purging with nitrogen prior to use. The $F(ab')_2$ fragments may be provided at a concentration of 5 mg/ml and reduced under argon in 30 mM cysteine. Alternatively, cysteamine may be employed. 100 mM Tris, pH 7.6 may be used as a buffer for 15 min at 37°C. The solutions may then be diluted 2-fold with an equal volume of the appropriate experimental buffer and spun through a 0.4 ml spin column of Bio-Gel P-6DG. The resulting Fab' fragments may be more efficient in their coupling to maleimide linkers. Note also that the same procedure may be employed with other macromolecules containing cysteine residues for coupling, for example, to the maleimide spacers. Also, peptides may be utilized, especially if they contain a cysteine residue. If the peptides have not been made fresh

and there is a possibility of oxidation of cysteine residues within the peptide structure, it may be necessary to regenerate the thiol group using the approach outlined above.

In one embodiment of the invention, the targeting ligands may be directed toward lymphocytes which may be T-cells or B-cells, with T-cells being the preferred target. To select a class of targeted lymphocytes, a targeting ligand having specific affinity for that class may be preferably employed. For example, an anti CD-A antibody may be used for selecting the class of T-cells harboring CD-4 receptors, an anti CD-8 antibody may be used for selecting the class of T-cells harboring CD-8 receptors, an anti CD-34 antibody may be used for selecting the class of T-cells harboring CD-34 receptors, and so on. A lower molecular weight ligand may preferably be employed, *e.g*., Fab or a peptide fragment. For example, an OKT3 antibody or OKT3 antibody fragment may be used.

Another major area for targeted delivery preferably involves the interlekin-2 (IL-2) system. IL-2 is a T-cell growth factor generally produced following antigen or mitogen induced stimulation of lymphoid cells. Cell types which typically produce IL-2 include, for example, $CD4^+$ and $CD8^+$ T-cells and large granular lymphocytes, as well as certain T-cell tumors. Generally speaking, IL-2 receptors are glycoproteins which are expressed on responsive cells. They are notable in connection with the present invention because they are generally readily endocytosed into lysosomal inclusions when bound to IL-2.

In addition to IL-2 receptors, preferred targets include the anti-IL-2 receptor antibody, natural IL-2 and an IL-2 fragment of a 20-mer peptide or smaller generated by phage display which binds to the IL-2 receptor. In use, for example, IL-2 may be conjugated to stablizing materials, for example, in the form of vesicles, and thus may mediate the targeting of cells bearing IL-2 receptors. Endocytosis of the ligand-receptor complex may then deliver the compound to be delivered to the targeted cell. Additionally, an IL-2 peptide fragment which has binding affinity for IL-2 receptors may be incorporated, for example, by attachment directly to a reactive moiety on a stabilizing material such as a lipid, including lipids in the form of vesicles, or via a spacer or linker molecule with a reactive end such as an amine, hydroxyl, or carboxylic acid functional group. Such linkers are well known in the art and may comprise from 3 to 20 amino acid residues. In addition, D-amino acids or derivatized amino acids may be used which avoids proteolysis in the target tissue.

Still other systems which my be used in the present invention include IgM-mediated endocytosis in B-cells or a variant of the ligand-receptor interactions described above wherein the T-cell receptor is CD2 and the ligand is lymphocyte function-associated antigen 3 (LFA-3), as described, for example, in Wallner et al, *J. Experimental Med*., 166:923-932 (1987), the disclosures of which are hereby incorporated by reference herein in their entirety. Targeting ligands derived or modified from human leukocyte origin, such as CD11a/CD18, and leukocyte cell surface glycoprotein (LFA-1), may also be used as these may bind to the endothelial cell receptor ICAM-1. The cytokine inducible member of the immunoglobulin superfamily, VCAM-1, which is mononuclear leukocyte-selective, may also be used as a targeting ligand. VLA-4, derived from human monocytes, may be used to target VCAM-1.

Preferred targeting ligands in accordance with the present invention include, for example, Sialyl Lewis X, mucin, hyaluronic acid, LFA- 1, methyl-α-D-mannopyrano-side, N-formal peptide, C5a, leukotriene B4, platelet activating factor, IL-8/NAP-1, CTAP-III, β-thromboglobulin, NAP-2, gro/MGSA, ENA-78, MCP-1, MAP-1α,β, RANTES, and I-309. In addition, the integrins αIIbβ3, αvβ3, α4β1, β1 and β2 may be used as targeting ligands for targeting VLA-4, fibrinogen, von Willebrand factor, fibronectin, vitronectin, VCAM-1 and CD49d/CD29. A particularly preferred targeting ligand may be Sialyl Lewis X which binds to P-selectin and which has the following sequence: α-Sialic acid (2→3)βGa1(1→4)[αFuc(1→3)βGlc-NAc-OR. P-selectin may be a preferred target because it typically localizes on the luminal side of endothelium during inflammation, but generally not in non-inflammatory synovia where it is generally cytoplasmic only.

Other preferred targeting ligands include, for example, antibodies directed to autoantigens on T-cell receptors. Peptides having the amino acid sequences Leu Leu Ile Tyr Phe Asn Asn Asn Val Pro Ile Asp Asp Ser Gly Met (SEQ ID NO:1) and Lys Ile Gln Pro Ser Glu Pro Arg Asp Ser Ala Val Tyr Phe Cys Ala (SEQ ID NO:2) can be used to produce antibodies which amy bind to the autoantigen portions of T-cell receptors. In addition, antibodies to additional T-cell and B-cell receptors may be used as targeting ligands. T- and B-cell receptors involved in inflammation and rheumatoid arthritis are described in "T Cell Receptors In Rheumatoid Arthritis," Struyk, *et al., Arthritis & Rheumatism*, **1995**, *38*:577-89; "Naturally Occurring Human Autoantibodies To Defined T-Cell Receptor And Light Chain Peptides," Marchalonis, *et al*., *Immunobiol. Proteins Peptides*, **1994**, Plenum Press, New York, p.135-45; "Lack Of Preferential Vβ Usage In Synovial T Cells Of Rheumatoid Arthritis Pateints," Dedeoglu, *et al., Immunol. Res.,* **1993**, *12*:12-20; "Human Autoantibodies To A Synthetic Putative T Cell Receptor β-Chain Regulatory Idiotype: Expression In Autoimmunity And Aging," Marchalonis, *et al., Exp. Clin. Immunogenet.,* **1993**, *10*:1-15; "Peptide Epitope Binding Specificity And $V_K$ And $V_H$ Gene Usage In A Monoclonal IgM Natural Autoantibody To T Cell Receptor CDR1 From A Viable Motheaten Mouse," Dehghanpisheh, *et al., Immunological Invest.*, **1996**, *25*:241-52; "Autoregulation Of TCR V Region Epitopes In Autoimmune Disease," Schluter, *et al*., *Immunobiol. Proteins Peptides VIII*, **1995**, Plenum Press, New York, p.231-36; "Characterization Of Autoantibodies Directed Against T Cell Receptors," Lake, *et al*., *Immunobiol. Proteins Peptides VIII*, **1995**, Plenum Press, New York, p.223-29; "Construction And Serological Characterization Of A Recombinant Human Single Chain T Cell Receptor," Lake, *et al., Biochem. Biophys. Res. Comm*., **1994**, *201*:1502-1509; "T Cell

Receptor Usage In Rheumatoid Arthritis," Lanchbury, *et al., British Med. Bull.,* **1995**, *51*:346-58; "Synthetic Autoantigens Of Immunoglobulins And T-Cell Receptors: Their Recognition In Aging, Infection, And Autoimmunity," Marchalonis, *et al., Autoantibodies Immunoglobulins T Cell Receptors*., **1994**, 129-47; "Natural Human Antibodies To Synthetic Peptide Autoantigens: Correlations With Age And Autoimmune Disease," Marchalonis, *et al., Gerentology*., **1993**, *39*:65-79; "Human Autoantibodies Reactive With Synthetic Autoantigens From T-Cell Receptor β Chain," Marchalonis, *et al., Proc. Natl. Acad. Sci. USA.*, **1992**, *89*:3325-29; "T-Cell Antigen Receptors In Rheumatoid Arthritis," Sakkas, *et al., Immunol. Res.,* **1994**, *13*:117-38; and "B And T Cell Antigen Repertoires in Lupus/Arthritis Murine Models," Theofilpooulos, *et al., Springer Semin Immunopathol.,* **1989**, *11*:335-68, Cronstein, *et al., Curr. Opin. Rheum*., **1994**, 6, 300-304, Szekanecz, *et al., J. Invest. Med.*, **1996**, *44*, 124-135, Liao, *et al.*, *Rheum. Arth.*, **1995**, *21*, 715-740, Veale, *et al.*, *Drugs & Aging,* **1996**, *9*, 87-92, Cronstein, *et al*., *Curr. Opin. Rheum*., **1994**, *6*, 300-304, Haskard, *Curr Opin. Rheum*., **1995**, *7*, 229-234, Cronstein, *et al., Curr. Opin. Rheum.*, **1994**, *6*, 300-304, *Remy, et al*., *Proc. Natl. Acad. Sci. USA,* **1995**, *92*, 1744-1748, Ashkenas, *et al., Dev. Biol.,* **1996**, *180*, 433-444, Springer, *Cell*, **1994**, *76*, 301-314, Hynes, *Cell*, **1992**, *69*, 11-25, and Schwartz, *et al*., *Annu. Rev. Cell Dev. Biol.,* **1995**, *11*, 549-599, the disclosures of each of which are hereby incorporated herein by reference in their entirety.

Additional targeting ligands which may be employed in the compositions and methods of the present invention are described in "Targeting Gene Transfer To Human Hematopoietic Progenitor Cell Lines Through The c-kit Receptor," Schwarzenberger, *et al., Blood.*, **1996**, *87*:472-8; "Methyl-α-D-Mannopyranoside, Monooligosaccharides And Yeast Mannans Inhibit Development Of Rat Adjuvant Arthritis," Prokopova, *et al., J. Rheumatol.,* **1993**, *20*:673-7; U.S. Patent No. 5,627,263, "The Platelt Glycoprotein IIb/IIIa-Like Protein In Human Endothelial Cells Promotes Adhesion But Not Initial Attachment To Extracellular Matrix," Chen, *et al., J. Cell. Biol.*, **1987**, *105*:1885-92; and "Primary Structure Of Lymphocyte Function-Associated Antigen 3 (LFA-3)," Wallner, *et al., J. Exp. Med*., **1987**, *166*:923-32, the disclosures of each of which are hereby incorporated herein by reference in their entirety.

Still additional targeting ligands which may be employed in the compositions and methods of the present invention are described in PCT publication WO 96/37194, the disclosures of which are hereby incorporated herein by reference in their entirety, and include fetuin and asialofetuin, hexamine, spermine and spermidine, N-glutaryl DOPE, IgA, IgM, IgG and IgD, MHC and HLA markers, and CD1, CD4, CD8-11, CD15, Cdw17, CD18, CD21-25, CD27, CD30-45, CD46-48, Cdw49, Cdw50, CD51, CD53-54, Cdw60, CD61-64, Cdw65, CD66-69, Cdw70, CD71, CD73-74, Cdw75, CD76-77, LAMP-1 and LAMP-2.

As would be apparent to one of ordinary skill in the art, once armed with the teachings of the present disclosure, compounds to be delivered, including the compounds exemplified herein, may also serve as targeting ligands in the present compositions, in the presence or absence of other targeting ligands. Similarly, targeting ligands, including the targeting ligands exemplified herein, may also serve as compounds to be delivered in the presence or absence of other compounds to be delivered. Thus, for example, telomerase, which is an exemplary compound to be delivered, may also be included in the present compositions as a targeting ligand.

In preferred embodiments of the present invention, targeting ligands may be associated with stabilizing materials, including stablizing materials in the form of vesicles, covalently or non-covalently. Thus, in preferred form, the targeting ligand may be bound, for example, via a covalent or non-covalent bond, to at least one of the lipids, proteins, polymers or surfactants which may be incorporated in the vesicles. Preferably, the targeting ligand is bound to one of more of the stabilizing materials covalently. In the case of lipid compositions which comprise cholesterol, the targeting ligand may preferably be bound to the cholesterol substantially only non-covalently, and/or the targeting ligand may be bound covalently to a component of the composition, for example, another lipid, such as a phospholipid, other than the cholesterol.

It is contemplated that the covalent (or non-covalent) binding between the targeting ligand and one or more stabilizing materials, such as lipids, especially lipids in the form of vesicles, may desirably enhance the delivery to the target cell of the compound to be delivered. While the inventors do not wish to be bound by any theory or theories of operation, it is believed that this improved delivery of the compound to be delivered may involve, for example, a fusion-initiated capping and patching mechanism, the intervention of clathrin-coated pits or through classical endocytosis, depending on the mechanisms for engulfment peculiar to the target cell, or by other natural or induced means. These mechanisms may assist the compound to be delivered to gain access to the interior of the target cell once the composition, for example, vesicle, containing the compound, reaches and/or links to the target cell.

Exemplary covalent bonds through which the targeting ligands may be covalently linked to stabilizing materials, including lipids, proteins, polymers, and surfactants include, for example, amide (-CONH-); thioamide (-CSNH-); ether (ROR', where R and R' may be the same or different and are other than hydrogen); ester (-COO-); thioester (-COS-); -O-; -S-; $-S_n-$, where n is greater than 1, preferably about 2 to about 8, and more preferably about 2; carbamates; -NH-; -NR-, where R is alkyl, for example, alkyl of from 1 to about 4 carbons; urethane; substituted imidate; and combinations of two or more of these. Covalent bonds between targeting ligands and stabilizing materials, for example, lipids, may be achieved through the use of molecules that may act as spacers to increase the conformational and topographical flexibility of the ligand. Examples of such spacers include, for example, succinic acid, 1,6-hexanedioic acid, 1,8-octanedioic acid, and the like, as well as modified amino acids, such as, for example, 6-aminohexanoic acid, 4-aminobutanoic acid, and the like. In addition, in the case of targeting ligands which comprise peptide moieties, sidechain-to-sidechain crosslinking may be complemented with sidechain-to-end crosslinking and/or end-to-end crosslinking. Also, small spacer molecules, such as dimethylsuberimidate, may be used to accomplish similar objectives. The use of agents, including those used in Schiff's base-type reactions, such as gluteraldehyde, may also be employed. The Schiff's base linkages, which may be reversible linkages, can be rendered more permanent covalent linkages via the use of reductive amination procedures. This may involve, for example, chemical reducing agents, such as lithium aluminum hydride reducing agents or their milder analogs, including lithium aluminum diisobutyl hydride (DIBAL), sodium borohydride ($NaBH_4$) or sodium cyanoborohydride ($NaBH_3CN$).

The covalent linking of the targeting ligands to the stabilizing materials in the present compositions, including the lipids, proteins, polymers and/or surfactants, may be accomplished using synthetic organic techniques which would be readily apparent to one of ordinary skill in the art in view of the present disclosure. For example, the targeting ligands may be linked to the materials, including the lipids, via the use of well known coupling or activation agents. As known to the skilled artisan, activating agents are generally electrophilic, which can be employed to elicit the formation of a covalent bond. Exemplary activating agents which may be used include, for example, carbonyldiimidazole (CDI), dicyclohexylcarbodiimide (DCC), diisopropylcarbodiimide (DIC), methyl sulfonyl chloride, Castro's Reagent, and diphenyl phosphoryl chloride.

The covalent bonds may involve crosslinking and/or polymerization. Crosslinking preferably refers to the attachment of two chains of polymer molecules by bridges, composed of either an element, a group, or a compound, which join certain carbon atoms of the chains by covalent chemical bonds. For example, crosslinking may occur in polypeptides which are joined by the disulfide bonds of the cysteine residue. Crosslinking may be achieved, for example, by (1) adding a chemical substance (crosslinking agent) and exposing the mixture to heat, or (2) subjecting a polymer to high energy radiation. A variety of crosslinking agents, or "tethers", of different lengths and/or functionalities are described, for example, in R.L. Lunbland, *Techniques in Protein Modification,* CRC Press, Inc., Ann Arbor, MI, pp. 249-68 (1995), the disclosures of which are hereby incorporated herein by reference in their entirety. Exemplary crosslinkers include, for example, 3,3'-dithiobis(succinimidylpropionate), dimethyl suberimidate, and its variations thereof, based on hydrocarbon length, and bis-N-maleimido-1,8-octane.

In accordance with preferred embodiments, the targeting ligands may be linked or attached to the lipids, proteins, polymers, or surfactants or other stabilizing materials via a linking group. Preferably the targeting ligand may be attached to a linker which is attached to the surface of a vesicle which includes the stabilizing material. A variety of linking groups are available and would be apparent to one skilled in the art in view of the present disclosure. Preferably, the linking group comprises a hydrophilic polymer. Suitable hydrophilic linker polymers include, for example, polyalkyleneoxides such as, for example, polyethylene glycol (PEG) and polypropylene glycol (PPG), polyvinylpyrrolidones, polyvinylmethylethers, polyacrylamides, such as, for example, polymethacrylamides, polydimethylacrylamides and polyhydroxypropylmethacrylamides, polyhydroxyethyl acrylates, polyhydroxypropyl methacrylates, polymethyloxazolines, polyethyloxazolines, polyhydroxyethyloxazolines, polyhyhydroxypropyloxazolines, polyvinyl alcohols, polyphosphazenes, poly(hydroxyalkylcarboxylic acids), polyoxazolidines, polyaspartamide, and polymers of sialic acid (polysialics). The hydrophilic polymers are preferably selected from the group consisting of PEG, PPG, polyvinylalcohol and polyvinylpyrrolidone and copolymers thereof, with PEG and PPG polymers being more preferred and PEG polymers being even more prefered. Preferred among the PEG polymers are, for example, bifunctional PEG having a molecular weight of about 1,000 Da to about 10,000 Da, preferably about 5,000 Da. Preferably, the polymer is bifunctional with the targeting ligand bound to a terminus of the polymer. Generally, the targeting ligand may be incorporated into the vesicle at concentrations of from about 0.1 mole % to about 25 mole %, preferably from about 1 mole % to about 10 mole %. In the case of gas-filled lipid vesicles, the targeting ligand is preferably incoporated in an amount from about 0.5 mole % to about 10 mole %, with from about 1 to about 10 mole % being more preferred. Of course, the particular ratio employed may depend, for example, upon the particular targeting ligand, gas, and lipid employed.

In embodiments involving lipid compositions which comprise lipids bearing polymers including, for example, DPPE-PEG, the targeting ligand may be linked directly to the polymer which is attached to the lipid to provide, for example, a conjugate of DPPE-PEG-TL, where TL is a targeting ligand. Thus, using the example DPPE-PEG, such as, for example, DPPE-PEG5000, the aforementioned conjugate may be represented as DPPE-PEG5000-TL. The hydrophilic polymer used as a linking group is preferably a bifunctional polymer, for example, bifunctional PEG, such as diamino-PEG. In this case, one end of the PEG group is linked, for example, to a lipid compound, and is bound at the free end to the targeting ligand via an amide linkage. A hydrophilic polymer, for example, PEG, substituted with a terminal carboxylate group on one end and a terminal amino group on the other end, may also be used. These latter bifunctional hydrophilic polymer may be preferred since they possess various similarities to amino acids.

Standard peptide methodology may be used to link the targeting ligand to the stabilizing materials, including lipids, when utilizing linker groups having two unique terminal functional groups. Bifunctional hydrophilic polymers, and especially bifunctional PEGs, may be synthesized using standard organic synthetic methodologies. In addition, many

of these materials are available commercially, such as, for example, α-amino-ω-carboxy-PEG which is commercially available from Shearwater Polymers (Huntsville, AL). An advantage of using a PEG material as the linking group is that the size of the PEG may be varied such that the number of monomeric subunits of ethylene glycol may be as few as, for example, about 5, or as many as, for example, about 500 or even greater. Accordingly, the "tether" or length of the linkage may be varied, as desired. This may be important depending, for example, on the particular targeting ligand employed. For example, a targeting ligand which comprises a large protein molecule may require a short tether, and therdby simulate a membrane bound protein. A short tether may also allow for a vesicle to maintain a close proximity to the cell. This may be used advantageously in connection with vesicles which also comprise a bioactive agent in that the concentration of bioactive agent which may be delivered to the cell may be advantageously increased. Another suitable linking group which may provide a short tether is glyceraldehyde. Glyceraldehyde may be bound, for example, to DPPE via a Schiff's base reaction. Subsequent Amadori rearrangement can provide a substantially short linking group. The β carbonyl of the Schiff's base may then react with a lysine or arginine of the targeting protein or peptide to form the targeted lipid.

In certain embodiments, the targeting ligands may be incorporated in the present stabilizing materials via non-covalent associations. As known to those skilled in the art, non-covalent association is generally a function of a variety of factors, including, for example, the polarity of the involved molecules, the charge (positive or negative), if any, of the involved molecules, the extent of hydrogen bonding through the molecular network, and the like. Non-covalent bonds are preferably selected from the group consisting of ionic interaction, dipole-dipole interaction, hydrogen bonds, hydrophilic interactions, van der Waal's forces, and any combinations thereof Non-covalent interactions may be employed to bind the targeting ligand to a stabilizing material, such as a lipid and, in the case of vesicles, directly to the surface of the vesicle. For example, the amino acid sequence Gly-Gly-His may be bound to the surface of a vesicle, preferably by a linker, such as PEG, and copper, iron or vanadyl ion may then be added. Proteins, such as antibodies which contain histidine residues, may then bind to the vesicle via an ionic bridge with the copper ion, as described in U.S. Patent No. 5,466,467, the disclosures of which are hereby incorporated herein by reference in their entirety. An example of hydrogen bonding involves cardiolipin lipids which may be incorporated into the lipid compositions. Additional techniques which may be adapted for incorporating the targeting ligand into the present compositions are disclosed, for example, in U.S. Application Serial No. 09/218,660, filed December 28, 1998, the disclosures of which are hereby incorporated herein by reference in their entirety.

In preferred embodiments of the present invention, which may involve vesicles, changes, for example, in pH and/or temperature *in vivo* or *in vitro*, may be employed to promote a change in location in the targeting ligands, for example, from a location within the vesicle, to a location external to the outer wall of the vesicle. This may promote binding of the targeting ligands to targeting sites, for example, receptors, such as lymphocytes, and tissues, including endothelial and epithelial cells, since the targeting ligand has a greater likelihood of exposure to such targeting sites. In addition, high energy ultrasound can be used to promote rupturing of the vesicles. This can also expose the targeting ligand to the desired binding site.

Targeting ligands may be incorporated into the compositions of the present invention, for example, in the form of compounds of the formula:

$$L\text{-}P\text{-}T$$

wherein:

L is a lipid, protein, polymer, carbohydrate, surfactant or the like;
P is a hydrophilic polymer; and
T is a targeting ligand.

In a preferred embodiment, L is a lipid selected from the group consisting of lecithins, phosphatidylcholines, phosphatidylserines, phosphatidylinositols, cardiolipins, cholesterols, cholesterolamines, lysophosphatides, erythrosphingosines, sphingomyelins, ceramides, cerebrosides, saturated phospholipids, unsaturated phospholipids, and krill phospholipids. More preferably, L is a lipid is selected from the group consisting of lecithins, phosphatidylcholines, phosphatidylserines and phosphatidylinositols. In other preferred embodiments, L is a lipid selected from the group consisting of 1,2-diacyl-sn-glycero-3-phosphocholines, 1,2-diacyl-sn-glycero-3-phosphoethanolamines, 1,2-diacyl-sn-glycero-3-[phospho-rac-( 1 -glycerols)], 1,2-diacyl-sn-glycero-3-phosphates, 1,2-diacyl-sn-glycero-3-[phosphoserines], lysophosphatidyl-cholines, lysophosphatidylglycerols, 1,2-diacyl-sn-glycerols, 1,2-diacyl-ethylene glycols, N-(n-caproylamine)- 1,2-diacyl-sn-glycero-3-phosphoethanolamines, N-dodecanylamine- 1,2-diacyl-sn-glycero-3-phosphoethanol-amines, N-succinyl- 1,2-diacyl-sn-glycero-3-phosphoethanolamines, N-glutaryl- 1,2-diacyl-sn-glycero-3-phosphoethanolamines and N-dodecanyl- 1,2-diacyl-sn-glycero-3-phosphoethanolamines. More preferably, L is a lipid selected from the group consisting of 1,2-diacyl-sn-glycero-3-phosphocholines, 1,2-diacyl-sn-glycero-3-phosphoethanolamines, 1,2-diacyl-sn-glycero-3-[phospho-rac-(1-glycerols)], 1,2-diacyl-sn-glycero-3-phosphates, 1,2-diacyl-sn-glycero-3-[phosphoserines], lysophosphatidylcholines, lysophosphatidyl-glycerols and 1,2-diacyl-sn-glycerols.

In other preferred embodiments, L is a protein which comprises albumin.

In still other preferred embodiments, L may be a polymer which comprises synthetic polymers or copolymers prepared from monomers selected from the group consisting of acrylic acid, methacrylic acid, ethyleneimine, crotonic acid, acrylamide, ethyl acrylate, methyl methacrylate, 2-hydroxyethyl methacrylate, lactic acid, glycolic acid, ε-caprolactone, acrolein, cyanoacrylate, bisphenol A, epichlorhydrin, hydroxyalkylacrylates, siloxane, dimethylsiloxane, ethylene oxide, propylene oxide, ethylene glycol, hydroxyalkylmethacrylates, N-substituted acrylamides, N-substituted methacrylamides, N-vinyl-2-pyrrolidone, 2,4-pentadiene-1-ol, vinyl acetate, acrylonitrile, styrene, p-amino-styrene, p-aminobenzylstyrene, sodium styrene sulfonate, sodium 2-sulfoxyethylmethacrylate, vinyl pyridine, aminoethyl methacrylates, 2-methacryloyloxytrimethylammonium chloride and polyphosphazene. Also preferred are compounds where L is a polymer which comprises synthetic polymers or copolymers selected from the group consisting of polyacrylic acid, polyethyleneimine, polymethacrylic acid, polymethylmethacrylate, polysiloxane, polydimethylsiloxane, polylactic acid, poly(ε-caprolactone), epoxy resin, poly(ethylene oxide), poly(propylene oxide), poly(ethylene glycol), polyamide, polyvinylidene-polyacrylonitrile, polyvinylidene-polyacrylonitrile-polymethylmethacrylate and polystyrene-polyacrylonitrile. Preferred among these polymers is polyvinylidene-polyacrylonitrile copolymer.

In other preferred embodiments, L is a surfactant, preferably a fluorosurfactant, and more preferably a fluorosurfactant having polyethylene glycol attached thereto.

In the above compounds, P is a hydrophilic polymer. Preferably, P is a hydrophilic polymer selected from the group consisting of polyalkyleneoxides, polyvinyl alcohol, polyvinylpyrrolidones, polyacrylamides, polymethacrylamides, polyphosphazenes, phosphazene, poly(hydroxyalkylcarboxylic acids) and polyoxazolidines. More preferably, P is a polyalkyleneoxide polymer, with polyethylene glycol and polypropylene glycol being even more preferred and polyethylene glycol being particularly preferred.

In the above formula, T is a targeting ligand, including the preferred targeting ligands discussed above.

In the case of targeting ligands which comprise saccharide groups, suitable saccharide moieties include, for example, monosaccharides, disaccharides and polysaccharides. Exemplary monosaccharides may have six carbon atoms and these saccharides include allose, altrose, glucose, dextrose, mannose, gulose, idose, galactose, talose, fructose, psicose, verbose and tagatose. Five carbon saccharides include ribose, arabinose, xylose, lyxose, ribulose and xylulose. Four carbon saccharides include erythrose, threose and erythrulose. Disaccharides include sucrose, lactose, maltose, isomaltose and cellobiose. Saccharide bearing targeting lipids may be synthesized through a multistep organic synthesis approach, as described more fully hereinafter. For example, lipids bearing targeting glucose moieties may be prepared by reacting, for example, α-glucopyranosyl bromide tetrabenzyl with ω-trifluoroacetylaminopoly-ethyleneglycol to obtain ω-glucopyranosyl tetrabenzyl-ω'-trifluoroacetylaminopoly-ethyleneglycol. This may then be hydrolyzed in a sodium carbonate or potassium carbonate solution and then hydrogenated to obtain ω-glucopyranpsyl-ω'amino-polyethyleneglycol. Amino glycopyranosyl terminated polyethyleneglycol may then react with N-DPGS-succinimide to form the lipid bearing saccharide DPGS-NH-PEG-Glucose.

Compounds employed in the present compositions, including lipids, proteins, surfactants and/or polymers, may contain various functional groups, such as, for example, hydroxy, thio and amine groups, which can react with a carboxylic acid or carboxylic acid derivative of the hydrophilic polymeric linker using suitable coupling conditions which would be apparent to one of ordinary skill in the art, once armed with the present disclosure. After the carboxylic acid group (or derivative thereof) reacts with the functional group, for example, hydroxy, thio or amine group to form an ester, thioester or amide group, any protected functional group may be deprotected utilizing procedures which would be well known to those skilled in the art. The term protecting group, as used herein, refers to any moiety which may be used to block reaction of a functional group and which may be removed, as desired, to afford the unprotected functional group. Any of a variety of protecting groups may be employed and these will vary depending, for example, as to whether the group to be protected is an amine, hydroxyl or carboxyl moiety. If the functional group is a hydroxyl group, suitable protecting groups include, for example, certain ethers, esters and carbonates. Such protecting groups are described, for example, in in Greene, TW and Wuts, PGM "Protective Groups in Organic Synthesis" John Wiley, New York, 2nd Edition (1991), the disclosures of which are hereby incorporated herein by reference, in their entirety. Exemplary protecting groups for amine groups include, for example, t-butyloxycarbonyl (Boc), benzyloxycarbonyl(Cbz), o-nitrobenzyloxycarbonyl and and trifluoroacetate (TFA).

Amine groups which may be present, for example, on a backbone of a polymer which is included in the vesicles, may be coupled to amine groups on a hydrophilic linking polymer by forming a Schiff's base, for example, by using coupling agents, such as glutaraldehyde. An example of this coupling is described by Allcock et al.,. *Macromolecules* Vol. 19(6), pp. 1502-1508 (1986), the disclosures of which are hereby incorporated herein by reference, in their entirety. If, for example, vesicles are formulated from polylysine, free amino groups may be exposed on the surface of the vesicles, and these free amine groups may be activated as described above. The activated amine groups can be used, in turn, to couple to a functionalized hydrophilic polymer, such as, for example, α-amino-ω-hydroxy-PEG in which the ω-hydroxy group has been protected with a carbonate group. After the reaction is completed, the carbonate group can be cleaved, thereby enabling the terminal hydroxy group to be activated for reaction to a suitable targeting ligand. In certain embodiments, the surface of a vesicle may be activated, for example, by displacing chlorine atoms in chlorine-containing phosphazene residues, such as polydichlorophosphazine. Subsequent addition of a targeting ligand and quenching of the remaining chloride groups with water or aqueous methanol will yield the coupled product.

In addition, poly(diphenoxyphosphazene) can be synthesized (Allcock et al., *Macromolecules* Vol. (1986) 19(6), pp. 1502-1508) and immobilized, for example, on DPPE, followed by nitration of the phenoxy moieties by the addition of a mixture of nitric acid and acetic anhydride. The subsequent nitro groups may then be activated, for example, by (1) treatment with cyanogen bromide in 0.1 M phosphate buffer (pH 11), followed by addition of a targeting ligand containing a free amino moiety to generate a coupled urea analog, (2) formation of a diazonium salt using sodium nitrite/HCl, followed by addition of the targeting ligand to form a coupled ligand, and/or (3) the use of a dialdehyde, for example, glutaraldehyde as described above, to form a Schiff's base. After linking the DPPE to the hydrophilic polymer and the targeting ligand, the vesicles may be formulated utilizing the procedures described herein.

Aldehyde groups on polymers can be coupled with amines as described above by forming a Schiff's base. An example of this coupling procedure is described in Allcock and Austin *Macromolecules* vol 14. p1616 (1981), the disclosures of which are hereby incorporated herein by reference, in their entirety.

In the above procedures, the polymer or terminus of the lipid, for example, phosphatidylglycerol or phosphatidylethanolamine, is preferably activated and coupled to the hydrophilic polymeric linker, the terminus of which has been blocked in a suitable manner. As an example of this strategy, α-amino, ω-carboxy PEG-4000 having a t-Boc protected terminal amino group and a free carboxylate end, may be activated with 1,1'-carbonyldiimidazole in the presence of hydroxybenzotriazole in N-methylpyrrolidone. After the addition of phosphatidylethanolamine, the t-Boc group may be removed by using trifluoroacetic acid (TFA), leaving the free amine. The amine may then be reacted with a targeting ligand which may comprise, for example, a peptide, protein, alkaloid, or other moiety, by similar activation of the ligand, to provide the lipid-linker-targeting ligand conjugate. Other strategies, in addition to those exemplified above, may be utilized to prepare the lipid-linker-targeting ligand conjugates. Generally speaking, these methods employ synthetic strategies which are generally known to those skilled in the art of synthetic organic chemistry.

Additional linkers would include other derivatives of lipids useful for coupling to a bifunctional spacer. For example, phosphatidylethanolamine (PE) may be coupled to a bifunctional agent. For example N-succinimidyl 4-(p-maleimidophenyl)butyrate (SMPB) and N-succinimidyl 3-(2-pyridyldithiol) propionate (SPDP), N-succinimidyl trans-4-(N-maleimidylmethyl)cyclohexane- 1 -carboxylate (SMCC), and N-succinimidyl 3-maleimidylbenzoate (SMB) may be used among others, to produce, for example the functionalized lipids MPB-PE and PDP-PE.

The free end of the hydrophilic spacer, such as polyethylene glycol ethylamine, which contains a reactive group, such as an amine or hydroxyl group, may be used to bind a cofactor or other targeting ligand. For example, polyethylene glycol ethylamine may be reacted with N-succinimidylbiotin or p-nitrophenylbiotin to introduce onto the spacer a useful coupling group. For example, biotin may be coupled to the spacer and this may readily bind proteins non-covalently. As an example, MPB-PEG-DPPE may be synthesized as follows. DPPE-PEG with a free amino group at the terminus of the PEG may be provided as described previously. Synthesis of the SMPB-PEG-DPPE may then be carried out with 1 equivalent of triethylamine in chloroform at a molar ratio of 1:5 SMPB-DPPE-PEG. After 3 hours, the reaction mixture may be evaporated to dryness under argon. Excess unreacted SMPB and major by products may be removed by preparative thin layer chromatography (TLC, silica gel developed with 50% acetone in chloroform). The upper portion of the lipid band can be extracted from the silica with about 20-30% methanol in chloroform (V:V) resulting in the isolation of pure intact MPB-Peg-DPPE. Streptavidin may then be coupled to proteins so that the proteins in turn may then be coupled to the MPB-PEG-DPPE. Briefly, SPDP may be incubated with streptavidin at room temperature for 30 minutes and chromatography may be employed to remove unreacted SPDP. Dithiothreitol (DTT) may be added to the reaction mixture followed thereafter with 2-thiopyridone at a concentration, for example, of 343 nM. The remainder of the reaction mixture may be reduced with DTT (for example, 25 mM for 10 min). The thiolated product may be isolated by gel exclusion, and the resulting streptavidin labeled proteins may then be used to bind to the biotinylated spacers affixed to the lipid moieties.

### *Nuclear Localization Sequences*

The compositions of the present invention may further preferably comprise a nuclear localization sequence (NLS). Nuclear localization sequences may be incorporated into the present compositions, including vesicle compositions, to advantageously enhance import of the compound to be delivered into the nucleus of the target cell, allowing for localization or concentration of such compounds therein, where such compounds may interact with the cellular genetic material or otherwise exhibit an effect.

Preferred nuclear localization sequences include, for example, peptides having the following amino acids sequences: Pro Lys Lys Lys Arg Lys Val (SEQ ID NO:3), Asn Lys Ile Pro Ile Lys Asp (SEQ ID NO:4), Lys Lys Tyr Glu

Asn Val Val Ile Lys Arg Ser Pro Arg Lys Arg Gly Arg Pro Arg Lys Asp Gly Thr Ser Ser Val Ser Ser Ser (SEQ ID NO:5), Ser Ser Asp Asp Glu Ala Thr Ala Asp Ser Gln His Ser Thr Pro Pro Lys Lys Lys Arg Lys Val (SEQ ID NO:6), Arg Ser Ser Arg Gly Lys Arg Arg Arg Ile Glu (SEQ ID NO:7), Arg Lys Ser Ser Thr Pro Glu Glu Val Lys Lys Arg Lys Lys Ala (SEQ ID NO:8), Ala Lys Ser Ser Arg Lys Arg Lys Leu (SEQ ID NO:9), Ser Ser Asp Asp Glu Ala Thr Ala Asp Ser Gln His Ser Thr Pro Pro Lys Lys Lys Arg Lys Val (SEQ ID NO:10), Arg Arg Pro Ser Lys Ala Lys Arg Gln Arg (SEQ ID NO: 11), Arg Arg Pro Ser Arg Arg Lys Arg Arg Gln Arg (SEQ ID NO:12), Arg Arg Pro Ser Glu Glu Lys Arg Lys Arg (SEQ ID NO:13), Pro Lys Arg Arg Arg His Arg Gln Asp Ala Leu Pro Gly Pro Cys Ile Ala Ser Thr Pro Lys Lys His Arg (SEQ ID NO:14), Pro Lys Arg Arg Arg His Arg Gln Pro Asp Gln Pro Lys Arg Arg Arg His Arg (SEQ ID NO:15), Pro Lys Arg Arg Arg His Arg (SEQ ID NO:16), Pro Lys Lys Lys Arg Lys (SEQ ID NO: 17), Arg Lys Lys Lys Arg Lys (SEQ ID NO: 18), Pro Lys Lys His Arg (SEQ ID NO:19), Arg Arg Gly Leu Lys Arg (SEQ ID NO:20), Lys Lys Lys Arg Lys Val (SEQ ID NO:21), Ala Val Lys Arg Pro Ala Ala Thr Lys Lys Ala Gly Gln Ala Lys Lys Lys Lys Leu Asp (SEQ ID NO:22), Val Asn Arg Lys Arg Asn Lys Leu Met Pro (SEQ ID NO:23), Ile Glu Gln Lys Arg Lys Arg Thr Tyr Glu (SEQ ID NO:24), Ala Ala Phe Glu Asp Leu Arg Val Leu Ser (SEQ ID NO:25), Lys Arg Pro Arg Pro (SEQ ID NO:26), and Met Leu Ser Leu Arg Gln Ser Ile Arg Phe Phe Lys Pro Ala Thr Arg (SEQ ID NO:27). Preferred peptides also include peptides having basic amino acid residues followed by about 10 to about 12 intervening amino acids followed by basic amino acids. The preferred NLS has from about 2 to about 10 amino acids. A particularly preferred NLS is shown in SEQ ID NO:20. These peptides can be prepared by basic peptide synthesis techniques well known to those skilled in the art.

Additional NLS molecules which may be incorporated in the present compositions include, for example, influenza virus nucleoprotein, karyopherin β1, human stat1 gene, m-importin, mouse homolog of nuclear pore targeting complex, hepatitis B virus (HBV) polymerase, glucocortocoid receptor (GlucR), interferon-regulated factors ISGF-3 and GAF, yeast mating switch/HO endonuclease promoter SW15, *Drosophila melanogaster* morphogen *dorsal*, nuclear factors NF-κB abd NF-AT, T-ag, c-rel, lamin $B_2$, GrH receptor, c-fos, cofilin, rNFIL-6, NF-ATplc, PKA C-subunit, $p42^{mapk}/p44^{erk1}$, $p90^{rsk}$, PKC-α, *lodestar*, v-jun, cyclin B1 (B-type cyclins), adenovirus 5 E1a protein, xnf7, PwA33, Rb-1, p53, c-myc, PTF1, HMG1/2, and tegument protein pp65 (UL83) of human cytomegalovirus. Exemplary NLS molecules which may be employed in the methods and compositions of the present invention are described, for example, in U.S. Patent No. 5,576,201, U.S. Patent No. 5,580,766, Shieh, *et al., Plant Physiol.*, **1993**, *101*, 353-361, Nadler, *et al., J. Biol. Chem*., **1997**, *272*, 4310-4315, Rech, *et al., J. Cell Science*, **1994**, *107*, 3029-3036, Goldfarb, *Science*, **1997**, *276*, 1814-1816, International Publication No. WO 95/34295, Davey, *et al., J. Cell. Biochem. Supp. O (9 Part C),* **1985**, 292, Moroianu, *et al., Proc. Natl. Acad. Sci. USA*, **1995**, *92*, 2008-2011, Yokoya, *et al., Cell Structure & Function*, **1995**, *20(6)*, 600, Alberti, *et al., Gut*, **1979**, *20*, 190-195, Manz, *et al., J. Steroid Biochem*., **1982**, *17*, XXXVI, Petricoin, *et al., J. Interferon Res*., **1991**, *11 Suppl. 1*, S154, Massa, *et al., J. Interferon Cyt. Res*., **1996**, *15*, 799-810, Nasmyth, *et al., Cell*, **1987**, *49*, 549-558, Isoda, *et al*., *Genes Dev.*, **1992**, *6*, 619-630, Yurochko, *et al., J. Virol.*, **1995**, *69*, 5391-5400, Verwiej, *et al., J. Biol. Chem*., **1990**, *265*, 15788-15795, Byrnes, *et al., J. Cell Biochem Suppl O*, **1987**, *11 part C*, 138, Chen, *et al., J. Virol*., **1983**, *45*, 104-113, Beckmann, *et al., Biol. Chem. Hoppe-Seyler*, **1992**, *373(9)*, 752-753, Zeitler, *et al., Ped. Res.*, **1994**, *35 (4 Part 2)*, 109A, Kerr, *et al., Soc. Neurosci. Abstracts*, **1993**, *19 (1-3)*, 805, Nishida, *et al., Zool. Sci*., **1984**, *1*, 903, Metz, *et al., Oncogene*, **1991**, *6*, 2165-2178, Schroeder, *et al., FASEB J*., **1993**, *7(3-4)*, A643, Rosenbaum, *et al., J. Cell Biol*., **1989**, *109 (4 Part 2)*, 214A, Girdham, *et al., Genes Dev*., **1991**, *5*, 1786-1799, Ryder, *et al*., *Proc. Natl. Acad. Sci. USA*, **1988,** *85*, 1487-1491, Bailly, *et al., J. Cell Sci*., **1992**, *101*, 529-545, Buchou, *et al., J. Cell Biochem. Suppl. O*, **1992**, *16 Part B*, 151, Miller, *et al*., *Development*, **1991**, *113*, 569-576, Bellini, *et al., J. Cell Biol*., **1995**, *131*, 563-570, Nagai, *et al., Chem. Pharm. Bull.*, **1972**, *20*, 1212-1216, Sun, *et al., Mol. Cell. Biol*., **1995**, *15*, 4489-4496, Reuse, *et al., Biochem. Biophys. Res. Comm*., **1986**, *141*, 1066-1076, Roux, *et al., Nuc. Acids Res*., **1989**, *17*, 1197-1214, and Pande, *et al., Virol*., **1991**, *182*, 220-228, the disclosures of each of which are hereby incorporated herein by reference in their entirety.

As indicated above, tegument protein pp65 (UL83) of human cytomegalovirus may be used as an NLS. In addition, fragments within the tegument protein pp65 sequence may also be used as an NLS. For example, the C-terminal end of SEQ ID NO:14 starting at Cys Ile Ala Ser Thr Pro Lys Lys His Arg is about 90% homologous with the C-terminus of tegument protein pp65. Thus, fusion of the carboxy-terminal element in SEQ ID NO:14 to a peptide would be expected to provide an NLS. SEQ ID NO: 15 would similarly be expected to provide an NLS.

Fragments of tegument protein pp65 which may be used as an NLS or in the preparation of same are identified, for example, as basic boxes A, B, C and D in Schmolke, S. *Journal of Virology*, **1995**, *69(2)*:1071-1078, the disclosures of which are hereby incorporated herein by reference, in their entirety. For example, the NLS may be comprised of basic boxes C-D from the tegument protein. The amino-terminal box of C includes the sequence shown in SEQ ID NO:16 which is almost identical in basic structure to the NLS of SV40 shown in SEQ ID NO:17. Nuclear protein has been identified in Xenopus, the basic amino-terminal sequence shown in SEQ ID NO:18. In bipartite targeting sequences, only two basic residues at the amino terminus may be required for the function of the entire NLS. The carboxy-terminus of basic box D of the tegument protein includes the sequence shown in SEQ ID NO:19, a short oligopeptide which may also be used as an NLS. It may be more effective, however, when box D is fused to box C. Expression of two copies of C in bipartite configuration may be used for effective nuclear import. The resulting NLS may thus fuse

with the receptor on the nuclear envelope. Another example of a nuclear receptor is the recombinant protein hSRP1α, which may bind to simple or bipartite NLS motifs. Molecules which avidly bind hSRP1α may be used as NLS also.

Exemplary NLS molecules which may be employed in the methods and compositions of the present invention are described, for example, in U.S. Patent Nos. 5,576,201 and 5,580,766, "Nuclear Targeting Of The Maize R Protein Requires Two Nuclear Localization Sequences," Shieh, e*t al., Plant. Physiol*., **1993**, *101*:353-61; "Differential Expression And Sequence-Specific Interaction Of Karyopherin α With Nuclear Localization Sequences," Nadler, *et al., J. Biol. Chem.*, **1997**, *272*:4310-15; and "Nuclear Import Of Serum Response Factor (SRF) Requires A Short Amino-Terminal Nuclear Localization Sequence And Is Independent Of The Casein Kinase II Phosphorylation Site," Rech, *et al., J. Cell Science*., **1994**, *107*:3029-36, the disclosures of each of which are hereby incorporated herein by reference in their entirety.

In another preferred embodiment of the invention, optimal delivery of the compound to be delivered may be accomplished by overcoming lysosomal and endosomalytic digestion of transfected macromolecules. The compositions described herein may optionally include viral fragments or viral proteins which function *in situ* to inhibit lysis as described in Schwarzenberger, *et al., Blood*., **1996**, *87*:472-8, the disclosures .of which are hereby incorporated herein by reference in their entirety.

The NLS materials may be incorporated into the compositions of the present invention in various ways. In this connection, the nuclear localization sequences may, for example, be simply combined with the organic halide, compound to be delivered and optional carrier. In embodiments involving vesicles, for example, the nuclear localization sequence may, for example, be encapsulated within the vesicles. In addition, if desired, the nuclear localization sequence may be associated with stabilizing materials, including stablizing materials in the form of vesicles, covalently or non-covalently. Thus, in certain preferred embodiments, as in the case of targeting ligands, the nuclear localization sequence may be bound, for example, via a covalent or non-covalent bond, to at least one of the lipids, proteins, polymers or surfactants which may be incorporated in the vesicles. In addition, the nuclear localization sequence may be associated with the compound to be delivered covalently or non-covalently. The covalent and/or non-covalent association of the nuclear localization sequence with the stabilizing materials and/or compounds to be delivered may involve the same or similar techniques as described above in connection with targeting ligands. Accordingly, the above discussion respecting targeting ligands and methods for their incorporation into the present compositions is incorporated herein by reference, in its entirety.

It is contemplated tat the covalent (or non-covalent) binding between the nuclear localization sequence and one or more stabilizing materials, such as lipids, especially lipids in the form of vesicles, as well as compounds to be delivered, may desirably enhance the delivery to the nucleus of the compound to be delivered. In the case of compounds to be delivered which comprise genetic material (*i.e*., DNA or RNA) and nuclear localization sequences which comprise peptides, a variety of methodologies are available to couple the genetic material to the peptides. For example, Lin *et al., Proc. Natl. Acad. Sci, USA*, **1995**, *92*:11044-11048, the disclosures of which are hereby incorporated herein by reference in their entirety, describes methods in which the 3' end of the nucleic acid chain (ssDNA is generally preferred over dsDNA) may be modified with a thiol group using C3-S-SCPG reagent and an optional tether of phosphoamidite ethylene glycol units between the thiol and the 3' end. The thiolated nucleic acid may then be reacted with an excess of N-methoxysuccinyl-(NLS)-peptide in dimethylformamide with trimethylammonium acetate at pH 7.5 for for about 12 to about 16 hours at room temperature. Unreacted peptide may be removed by reaction with dithiothreitol.

Other variations for covalently linking NLS peptides to genetic material may involve, for example, attaching a disulfide moiety to the free 3'-OH via a cysteine reidue (if any) in the peptide, or preparing a peptide nucleic acid (see PCT publication WO 96/11205 and Norton et al., Bioorg. Med. Chem. (95) 3:437-445, the disclosures of each of which are hereby incorporated herein by reference, in their entirety).

The useful dosage of nuclear localization sequence to be administered or delivered, as well as the mode of administration, may vary depending upon, for example, the type and nature of the nuclear localization sequence involved, the age, weight, cells or patient (animal) to be treated, the particular diagnostic, therapeutic, or other application intended (including the disease state, if any, to be prevented and/or treated), the compound to be delivered, and the organic halide (if any) and carrier (if any) employed. Typically, dosage may be initiated at lower levels and may be increased until the desired therapeutic or diagnostic effect is achieved. The desired dosage, including any therapeutically or diagnostically effective dosage amounts, will be well within the ambit of one skilled in the art, armed with the prevailing medical literature and with the present disclosure. Representative amounts are provided in the examples herein. Of course, higher or lower amounts may be employed, as will be recognized by the skilled artisan.

### *Fusion Peptides*

The ability to fuse or bind to a membrane may also be enhanced by optionally including a fusion peptide in the present compositions. Exemplary fusion peptides include, for example, synexin, IL-2-diphtheria toxin related, TNFR:Fc (p75) IL-2, TNFα receptor antibody-IL-2, rif-L-myc, p65 synaptotagmin, PIXY321 (GM-CSF/IL-3),

PML1RARα (promyelocytic leukemia), and OmpA. Additional fusion proteins include, for example, PEN-1, C9, and muteins thereof. The amino acid sequence of native PEN-1 is as follows: Lys Leu Met Lys Lys Ile Pro Gln Ile Ser Asp Ala Glu Leu Glu Val Met Lys Val Ile Trp Lys His Ser Ser Ile Asn Thr Asn Glu Val Ile Lys Glu Leu Ser Lys Thr Ser Thr Trp Ser Pro Lys Thr Ile Gln Thr Met Leu Leu Arg Leu Ile Lys Lys Gly Ala Leu Asn His His Lys Glu Gly Arg Val Phe Val Tyr Thr Pro Asn Ile Asp Glu Ser Asp Tyr Ile Glu Val Lys Ser His Ser Phe Leu Asn Arg Phe Tyr Asn Gly Thr Leu Asn Ser Met Val Leu Asn Phe Leu Glu Asn Asp Gln Leu Ser Gly Glu Glu Ile Asn Glu Leu Tyr Gln Ile Leu Glu Glu His Lys Asn Arg Lys Lys Glu Pro Trp Asp Ser Asp Arg Ala Ile Glu Gly Arg (SEQ ID NO:28). The amino acid sequence of C9 fusion peptide is: Ala Ser Asp Arg Ala Ile Glu Gly Arg (SEQ ID NO:29). The amino acid sequences of exemplary C9 muteins include, but are not limited to: Asp Ala Asp Arg Ala Ile Glu Gly Arg (SEQ ID NO:30), Asp Ser Ala Arg Ala Ile Glu Gly Arg (SEQ ID NO:31), Asp Ser Asp Ala Ala Ile Glu Gly Arg (SEQ ID NO:32), Asp Ser Asp Arg Ser Ile Glu Gly Arg (SEQ ID NO:33), Asp Ser Asp Arg Ala Ala Glu Gly Arg (SEQ ID NO:34), Asp Ser Asp Arg Ala Ile Ala Gly Arg (SEQ ID NO:35), Asp Ser Asp Arg Ala Ile Glu Ala Arg (SEQ ID NO:36), and Asp Ser Asp Arg Ala Ile Glu Gly Ala (SEQ ID NO:37).

Fusion peptides which may be optionally included in the present compositions include fusion peptides that may contain all or a substantial portion of the MPS-1 sequence (or a homologous analog of MPS-1) which may be coupled to an additional polypeptide having one or more desired functions. For example, certain sequences which are usually present at the amino termini of secreted proteins may increase the rate at which polypeptides are secreted by cells; *see, e.g*., Ensoli, B. *et al., Nature*, **1990**, *345*:84-86 and Watson, J.D. *et al*., "Molecular Biology of the Gene", The Benjamin Cummings Publishing Co., Menlo Park, Calif., pp. 730-735 (1988), the disclosures of each of which are hereby incorporated herein by reference, in their entirety. As another example, NLS, mentioned above, may be involved in the transport of polypeptides into the nucleus; *see, e.g.*, Frankel, A. D. *et al., Cell*, **1988**, *55*:1189-1193, Watson, J.D. *et al.,* "Molecular Biology of the Gene", The Benjamin Cummings Publishing Co., Menlo Park, Calif., pp. 730-735 (1988), Kalderon, D. *et al*., *Cell*, **1984**, *39*:499-509, and Silver, P.A. *et al., Proc. Natl., Acad. Sci. USA*, **1984**, *81* :5951-5955, the disclosures of each of which are hereby incorporated herein by reference, in their entirety. Another example involves sequences that may be cleaved from polypeptides inside the cytoplasm to generate "mature" peptides that do not have a methionine residue at the amino terminus; *see, e.g*., Watson, J.D. *et al*., "Molecular Biology of the Gene", The Benjamin Cummings Publishing Co., Menlo Park, Calif., pp. 730-735 (1988), the disclosures of which are hereby incorporated herein by reference, in their entirety. If desired, a chimeric gene can be created which will express an MPS peptide sequence coupled to any of those three types of sequences to provide an MPS fusion peptide having the additional function conferred upon it by the secretion, NLS, or cleavage sequence.

Another class of fusion peptides which may have even greater potential importance in connection with cancer therapy and other types of therapy as well involves MPS sequences coupled to cell specific carrier sequences. As used herein, "cell-specific carrier sequences" refers to polypeptide sequences that may preferentially bind and enter into certain types of cells, preferably without having comparable affinity for other types of cells. For example, cancer cells may have growth factor receptors on their surfaces which may be absent from the surfaces of normal cells, or which may be present at much lower concentrations on the surfaces of normal cells. A number of cancer-related growth factors and their receptors are listed and reviewed in Cooper, G. M., *Oncogenes,* Jones and Barlett Publishers, Boston (1990), the disclosures of which are hereby incorporated herein by reference in their entirey. These include TGF alpha receptor (which may be present in abnormally high quantities on the surfaces of certain human epidermoid carcinoma cells) and interleukin-2 (which is a growth factor for both T cells and T lymphoma cells). Accordingly, it is possible to couple such ligands to the MPS-1 polypeptide sequence to create a fusion peptide, which may be expressed by a single chimeric gene in genetically transformed cells; *see e.g*. Siegall, C.B. *et al., FASEB J*., **1989**, *3*:2647-2652 and Lorberboum-Galski, H. *et al., Proc. Natl. Acad. Sci. USA*, **1988**, *85*:1922-1926, the disclosures of each of which are hereby incorporated herein by reference, in their entirety. The fusion peptide can be mixed with a source of metal ions other than zinc, such as cadmium or platinum, to generate an MPS analog which may be capable of suppressing rather than stimulating cell division. The carrier sequence may help to recognize and carry the MPS analog into the cancerous target cells expressing the specific type of cell surface receptor. Furthermore, these fusion peptides may make it possible to control MPS-1 mediated gene expression in specific cell types.

An important class of MPS-1 analogs includes analogs having one or more amino acid residues which may be replaced with other residues, for example, to enhance the stimulatory activity of the analog in comparison to natural MPS-1, or to provide some other desired benefit. For example, the cysteine 64 and/or cysteine 77 residues, which are not involved in the zinc finger domain of MPS-1, may be replaced by serine or another amino acid residue, which may desirably prevent the mutated analog from forming unwanted disulfide bridges at these sites. In addition, in the case of MPS antagonists, the purpose of a site-specific mutation may be to enhance the growth-inhibiting activity of the analog. While the specific effects of any such site-specific mutation on the biological activity of the resulting analog are not entirely clear, it is possible, using computers and molecular modeling, to identify promising MPS candidate analogs for screening tests. The selected candidate MPS analogs may be generated using the techniques of genetic engineering; this is easily done using M13 viral vectors. The resulting MPS analogs may then be screened to determine whether have a desirable increase in growth-stimulating or growth suppressing activity, using routine screening tests as

described herein. Such analogs which may be derived from the MPS-1 sequence disclosed herein, and which may have substantial homology with the MPS-1 sequence disclosed herein, where the screening tests require no more than routine skill in the art, are within the scope of the present invention.

Additional fusion peptides corresponding to regions of the MPS-1 protein include, but are not limited to, PLAKDLLGPSPEEEKR (SEQ ID NO:38), which corresponds to MPS-1 amino acid residues 2-17 and derives from the N-terminal region of the peptide, located between the N-terminus and the zinc finger domain, YKITTVFSHAQTVVL (SEQ ID NO:39), which corresponds to MPS-1 amino acid sequence 41-55 in the zinc finger domain, and TGGKARLTEGCSFRRQH (SEQ ID NO:40), which corresponds to MPS-1 amino acid sequence 67-84 located between the zinc finger and the C-terminus. All three peptides were synthesized on an Applied Biosystems peptide synthesized (Model 430A) using ter-butyloxycarbonyl (t-boc) chemistry.

***Methods Of Preparation***

A wide variety of methods are available for the preparation of the compositions, including compositions comprising vesicles, such as micelles and/or liposomes. Included among these methods are, for example, shaking, drying, gas-installation, spray drying, and the like. Suitable methods for preparing vesicle compositions are described, for example, in U.S. Patent No. 5,469,854, the disclosures of which are hereby incorporated herein by reference in their entirety. The vesicles are preferably prepared from lipids which remain in the gel state.

Micelles may be prepared using any one of a variety of conventional micellar preparatory methods which will be apparent to those skilled in the art. These methods typically involve suspension of a carrier material, such as a lipid compound, in an organic solvent, evaporation of the solvent, resuspension in an aqueous medium, sonication and centrifugation. The foregoing methods, as well as others, are discussed, for example, in Canfield et al., *Methods in Enzymology, 189*:418-422 (1990); El-Gorab et al, *Biochem. Biophys. Acta*, *306*:58-66 (1973); *Colloidal Surfactant*, Shinoda, K., Nakagana, Tamamushi and Isejura, Academic Press, NY (1963) (especially "The Formation of Micelles", Shinoda, Chapter 1, pp. 1-88); *Catalysis in Micellar and Macromolecular Systems*, Fendler and Fendler, Academic Press, NY (1975). The disclosures of each of the foregoing publications are hereby incorporated herein by reference in their entirety.

In liposomes, the lipid compound(s) may be in the form of a monolayer or bilayer, and the monolayer or bilayer lipids may be used to form one or more monolayers or bilayers. In the case of more than one monolayer or bilayer, the monolayers or bilayers are generally concentric. Thus, lipids may be used to form unilamellar liposomes (comprised of one monolayer or bilayer), oligolamellar liposomes (comprised of two or three monolayers or bilayers) or multilamellar liposomes (comprised of more than three monolayers or bilayers).

A wide variety of methods are available in connection with the preparation of vesicles, including liposomes. Accordingly, liposomes may be prepared using any one of a variety of conventional liposomal preparatory techniques which will be apparent to those skilled in the art, including, for example, solvent dialysis, French press, extrusion (with or without freeze-thaw), reverse phase evaporation, simple freeze-thaw, sonication, chelate dialysis, homogenization, solvent infusion, microemulsification, spontaneous formation, solvent vaporization, solvent dialysis, French pressure cell technique, controlled detergent dialysis, and others, each involving the preparation of the vesicles in various fashions. *See, e.g.*, Madden et al., *Chemistry and Physics of Lipids*, *53*:37-46 (1990), the disclosure of which is hereby incorporated herein by reference in its entirety. Suitable freeze-thaw techniques are described, for example, in International Application Serial No. PCT/US89/05040, filed November 8, 1989, the disclosure of which is hereby incorporated herein by reference in its entirety. Methods which involve freeze-thaw techniques are preferred in connection with the preparation of liposomes. Preparation of the liposomes may be carried out in a solution, such as an aqueous saline solution, aqueous phosphate buffer solution, or sterile water. The liposomes may also be prepared by various processes which involve shaking or vortexing, which may be achieved, for example, by the use of a mechanical shaking device, such as a Wig-L-Bug™ (Crescent Dental, Lyons, IL), a Mixomat (Degussa AG, Frankfurt, Germany), a Capmix (Espe Fabrik Pharmazeutischer Praeparate GMBH & Co., Seefeld, Oberay Germany), a Silamat Plus (Vivadent, Lechtenstein), or a Vibros (Quayle Dental, Sussex, England). Conventional microemulsification equipment, such as a Microfluidizer™ (Microfluidics, Woburn, MA) may also be used.

Spray drying may be employed to prepare gas filled vesicles. Utilizing this procedure, the carrier materials, such as lipids, may be pre-mixed in an aqueous environment and then spray dried to produce gas filled vesicles. The vesicles may be stored under a headspace of a desired gas.

Many liposomal preparatory techniques which may be adapted for use in the preparation of vesicle compositions are discussed, for example, in U.S. Patent Nos. 4,728,578, 4,728,575, 4,737,323, 4,533,254, 4,162,282, 4,310,505, and 4,921,706; U.K. Patent Application GB 2193095 A; International Application Serial No. PCT/US85/01161; Mayer et al., *Biochimica et Biophysica Acta*, *858*:161-168 (1986); Hope et al., *Biochimica et Biophysica Acta*, *812*:55-65 (1985); Mayhew et al., *Methods in Enzymology, 149*:64-77 (1987); Mayhew et al., *Biochimica et Biophysica Acta, 755*:169-74 (1984); Cheng et al, *Investigative Radiology*, *22*:47-55 (1987); International Application

Serial No. PCT/US89/05040; and *Liposome Technology*, Gregoriadis, ed., Vol. I, pp. 29-31, 51-67 and 79-108 (CRC Press Inc., Boca Raton, FL 1984), the disclosures of each of which are hereby incorporated by reference herein in their entirety.

In connection with carrier materials, and especially lipid compositions in the form of vesicles, it may be advantageous to prepare the lipid compositions at a temperature below the gel to liquid crystalline phase transition temperature of the lipids. This phase transition temperature is the temperature at which a lipid bilayer will convert from a gel state to a liquid crystalline state. See, for example, Chapman *et al., J. Biol. Chem., 249*:2512-2521 (1974), the disclosure of which is hereby incorporated by reference herein in its entirety. It is generally believed that vesicles which are prepared from lipids that possess higher gel state to liquid crystalline state phase transition temperatures tend to have enhanced impermeability at any given temperature. See Derek Marsh, *CRC Handbook of Lipid Bilayers* (CRC Press, Boca Raton, FL 1990), at p. 139 for main chain melting transitions of saturated diacyl-sn-glycero-3-phosphocholines. The gel state to liquid crystalline state phase transition temperatures of various lipids will be readily apparent to those skilled in the art and are described, for example, in Gregoriadis, ed., *Liposome Technology*, Vol. I, 1-18 (CRC Press, 1984). The following table lists some of the representative lipids and their phase transition temperatures.

TABLE 2

| **Saturated Diacyl-sn-Glycero-3-Phosphocholines: Main Chain Melting Transition Temperatures** | |
|---|---|
| Number of Carbons in Acyl Chains | Main Phase Transition Temperature (° C) |
| 1,2-(12:0) | -1.0 |
| 1,2-(13:0) | 13.7 |
| 1,2-(14:0) | 23.5 |
| 1,2-(15:0) | 34.5 |
| 1,2-(16:0) | 41.4 |
| 1,2-(17:0) | 48.2 |
| 1,2-(18:0) | 55.1 |
| 1,2-(19:0) | 61.8 |
| 1,2-(20:0) | 64.5 |
| 1,2-(21:0) | 71.1 |
| 1,2-(22:0) | 74.0 |
| 1,2-(23:0) | 79.5 |
| 1,2-(24:0) | 80.1 |

See, for example, Derek Marsh, *CRC Handbook of Lipid Bilayers*, p. 139 (CRC Press, Boca Raton, FL 1990).

Carrier materials, such as lipids, comprising a gas can be prepared by agitating an aqueous solution containing, if desired, a carrier material, in the presence of a gas. The term "agitating" means any shaking motion of an aqueous solution such that gas is introduced from the local ambient environment into the aqueous solution. This agitation is preferably conducted at a temperature below the gel to liquid crystalline phase transition temperature of the lipid. The shaking involved in the agitation of the solutions is preferably of sufficient force to result in the formation of a lipid composition, including vesicle compositions, and particularly vesicle compositions comprising gas filled vesicles. The shaking may be by swirling, such as by vortexing, side-to-side, or up and down motion. Different types of motions may be combined. Also, the shaking may occur by shaking the container holding the aqueous lipid solution, or by shaking the aqueous solution within the container without shaking the container itself.

The shaking may occur manually or by machine. Mechanical shakers that may be used include, for example, a shaker table such as a VWR Scientific (Cerritos, CA) shaker table, as well as any of the shaking devices described hereinbefore, with the Capmix (Espe Fabrik Pharmazeutischer Praeparate GMBH & Co., Seefeld, Oberay, Germany) being preferred. It has been found that certain modes of shaking or vortexing can be used to make vesicles within a

preferred size range. Shaking is preferred, and it is preferred that the shaking be carried out using the Espe Capmix mechanical shaker. In accordance with this preferred method, it is preferred that a reciprocating motion be utilized to generate the lipid compositions, and particularly vesicles. It is even more preferred that the motion be reciprocating in the form of an arc. It is contemplated that the rate of reciprocation, as well as the arc thereof, is particularly important in connection with the formation of vesicles. Preferably, the number of reciprocations or full cycle oscillations is from about 1000 to about 20,000 per minute. More preferably, the number of reciprocations or oscillations is from about 2500 to about 8000, with reciprocations or oscillations of from about 3300 to about 5000 being even more preferred. Of course, the number of oscillations can be dependent upon the mass of the contents being agitated. Generally speaking, a larger mass requires fewer oscillations. Another means for producing shaking includes the action of gas emitted under high velocity or pressure.

It will also be understood that preferably, with a larger volume of aqueous solution, the total amount of force will be correspondingly increased. Vigorous shaking is defined as at least about 60 shaking motions per minute, and is preferred. Vortexing at about 60 to about 300 revolutions per minute is more preferred. Vortexing at about 300 to about 1800 revolutions per minute is even more preferred.

In addition to the simple shaking methods described above, more elaborate methods can also be employed. Such elaborate methods include, for example, liquid crystalline shaking gas instillation processes and vacuum drying gas instillation processes, such as those described in U.S. Patent Nos. 5,469,854, 5,580,575, 5,585,112, and 5,542,935, and U.S. Application Serial No. 08/307,305, filed September 16, 1994, the disclosures of each of which are incorporated herein by reference in their entirety. Emulsion processes may also be employed in the preparation of compositions in accordance with the present invention. Such emulsification processes are described, for example, in Quay, U.S. Patent Nos. 5,558,094, 5,558,853, 5,558,854, and 5,573,751, the disclosures of each of which are hereby incorporated herein by reference in their entirety. Spray drying may be also employed to prepare the gaseous precursor filled vesicles. Utilizing this procedure, the lipids may be pre-mixed in an aqueous environment and then spray dried to produce gaseous precursor filled vesicles. The vesicles may be stored under a headspace of a desired gas. Although any of a number of varying techniques can be used, the vesicle compositions employed in the present invention are preferably prepared using a shaking technique. Preferably, the shaking technique involves agitation with a mechanical shaking apparatus, such as an Espe Capmix (Seefeld, Oberay, Germany), using, for example, the techniques disclosed in U.S. application Serial No. 160,232, filed November 30, 1993, the disclosures of which are hereby incorporated herein by reference in its entirety. In addition, after extrusion and sterilization procedures, which are discussed in detail below, agitation or shaking may provide vesicle compositions which can contain substantially no or minimal residual anhydrous lipid phase in the remainder of the solution. (Bangham, et al, *J. Mol. Biol*. *13*:238-252 (1965)). Other preparatory techniques include those described in Unger, U.S. Patent No. 5,205,290, the disclosure of which is hereby incorporated herein by reference in its entirety.

Foams exemplify an additional embodiment of the invention. Foams have biomedical applications, for example, in implants for local delivery of drugs, tissue augmentation, wound healing, and prevention of peritoneal adhesions. Phospholipid foams may generally be created by increasing the concentration of the phospholipids as well as by mixing with materials such as cetyl alcohol, surfactants, simethicone or polymers, such as methylcellulose. Fluorinated phospholipids may also be used to create stable, long-lasting foams. The most stable foams are generally prepared from materials which are polymerized or cross-linked, such as polymerizable phospholipids. Since foaming is also a function of surface tension reduction, detergents are generally useful foaming agents.

Foams may also be produced by shaking gas filled vesicles, wherein the foam appears on the top of the aqueous solution, and their formation is generally associated with a decrease in the volume of the aqueous solution. Preferably, the final volume of the foam is at least about two times the initial volume of the aqueous stabilizing material solution; more preferably, the final volume of the foam is at least about three times the initial volume of the aqueous solution; even more preferably, the final volume of the foam is at least about four times the initial volume of the aqueous solution; and most preferably, all of the aqueous stabilizing material solution is converted to foam.

The required duration of shaking time may be determined by detection of the formation of foam. For example, about 10 mL of lipid solution in a 50 mL centrifuge tube may be vortexed for approximately 15 to 20 minutes or until the viscosity of the gas filled liposomes becomes sufficiently thick so that it no longer clings to the side walls as it is swirled. At this time, the foam may cause the solution containing the gas filled liposomes to raise to a level of about 30 to about 35 mL.

The concentration of lipid required to form a preferred foam level will vary depending upon the type of lipid used, and may be readily determined by one skilled in the art, in view of the present disclosure. For example, in preferred embodiments, the concentration of 1,2-dipalmitoylphosphatidylcholine (DPPC) used to form gas filled liposomes according to the methods of the present invention is about 20 mg/mL to about 30 mg/mL saline solution. The concentration of distearoylphosphatidylcholine (DSPC) used in preferred embodiments is about 5 mg/mL to about 10 mg/mL saline solution.

Specifically, DPPC in a concentration of about 20 mg/mL to about 30 mg/mL, upon shaking, yields a total

suspension and entrapped gas volume about four times greater than the suspension volume alone. DSPC in a concentration of about 10 mg/mL, upon shaking, yields a total volume completely devoid of any liquid suspension volume and contains entirely foam.

Microemulsification is a common method of preparing an emulsion of a foam precursor. Temperature increases and/or lowered pressures will cause foaming as gas bubbles form in the liquid. As discussed above, the foam may be stabilized by, for example, surfactants, detergents or polymers.

The size of gas filled vesicles can be adjusted, if desired, by a variety of procedures, including, for example, microemulsification, vortexing, extrusion, filtration, sonication, homogenization, repeated freezing and thawing cycles, extrusion under pressure through pores of defined size, and similar methods. Gas filled vesicles prepared in accordance with the methods described herein can range in size from less than about 1 μm to greater than about 100 μm. In addition, after extrusion and sterilization procedures, which are discussed in detail below, agitation or shaking provides vesicle compositions which provide substantially no or minimal residual anhydrous lipid phase in the remainder of the solution. (Bangham, et al., *J. Mol. Biol*., *13*:238-252 (1965)). If desired, the vesicles of the present invention maybe used as they are formed, without any attempt at further modification of the size thereof. For intravascular use, the vesicles preferably have diameters of less than about 30 μm, and more preferably, less than about 12 μm. For targeted intravascular use including, for example, binding to certain tissue, such as cancerous tissue, the vesicles can be significantly smaller, for example, less than about 100 nm in diameter. For enteric or gastrointestinal use, the vesicles can be significantly larger, for example, up to a millimeter in size. Preferably, the vesicles are sized to have diameters of from about 2 μm to about 100 μm.

The gas filled vesicles may be sized by a simple process of extrusion through filters wherein the filter pore sizes control the size distribution of the resulting gas filled vesicles. By using two or more cascaded or stacked set of filters, for example, a 10 μm filter followed by an 8 μm filter, the gas filled vesicles can be selected to have a very narrow size distribution around 7 to 9 μm. After filtration, these gas filled vesicles can remain stable for over 24 hours.

The sizing or filtration step may be accomplished by the use, for example, of a filter assembly when the composition is removed from a sterile vial prior to use, or more preferably, the filter assembly may be incorporated into a syringe during use. The method of sizing the vesicles will then comprise using a syringe comprising a barrel, at least one filter, and a needle; and will be carried out by an extraction step which comprises extruding the vesicles from the barrel through the filter fitted to the syringe between the barrel and the needle, thereby sizing the vesicles before they are administered to a patient. The extraction step may also comprise drawing the vesicles into the syringe, where the filter will function in the same way to size the vesicles upon entrance into the syringe. Another alternative is to fill such a syringe with vesicles which have already been sized by some other means, in which case the filter now functions to ensure that only vesicles within the desired size range, or of the desired maximum size, are subsequently administered by extrusion from the syringe.

In certain preferred embodiments, the vesicle compositions may be heat sterilized or filter sterilized and extruded through a filter prior to shaking. Generally speaking, vesicle compositions comprising a gas may be heat sterilized, and vesicle compositions comprising gaseous precursors may be filter sterilized. Once gas filled vesicles are formed, they may be filtered for sizing as described above. Performing these steps prior to the formation of gas and/or gaseous precursor filled vesicles provide sterile gas and/or gaseous precursor filled vesicles ready for administration to a patient. For example, a mixing vessel such as a vial or syringe may be filled with a filtered lipid composition, and the composition may be sterilized within the mixing vessel, for example, by autoclaving. Gas may be instilled into the composition to form gas filled vesicles by shaking the sterile vessel. Preferably, the sterile vessel is equipped with a filter positioned such that the gas filled vesicles pass through the filter before contacting a patient.

The step of extruding the solution of lipid compound through a filter decreases the amount of unhydrated material by breaking up any dried materials and exposing a greater surface area for hydration. Preferably, the filter has a pore size of about 0.1 to about 5 μm, more preferably, about 0.1 to about 4 μm, even more preferably, about 0.1 to about 2 μm, and still more preferably, about 1 μm. Unhydrated compound, which is generally undesirable, appears as amorphous clumps of non-uniform size.

The sterilization step provides a composition that may be readily administered to a patient for diagnostic imaging including, for example, ultrasound or CT. In certain preferred embodiments, sterilization may be accomplished by heat sterilization, preferably, by autoclaving the solution at a temperature of at least about 100 °C, and more preferably, by autoclaving at about 100°C to about 130°C, even more preferably, about 110°C to about 130°C, still more preferably, about 120°C to about 130°C, and even more preferably, about 130 °C. Preferably, heating occurs for at least about 1 minute, more preferably, about 1 to about 30 minutes, even more preferably, about 10 to about 20 minutes, and still more preferably, about 15 minutes.

If desired, the extrusion and heating steps, as outlined above, may be reversed, or only one of the two steps can be used. Other modes of sterilization may be used, including, for example, exposure to gamma radiation.

In addition to the aforementioned embodiments, gaseous precursors contained in vesicles can be formulated which, upon activation, for example, by exposure to elevated temperature, undergo a phase transition from, for

example, a liquid, including a liquid entrapped in a vesicle, to a gas, expanding to create the gas filled vesicles described herein. This technique is described in detail, for example, in U.S. Patent Nos. 5,585,112 and 5,542,935, the disclosures of which are hereby incorporated herein by reference in their entirety.

The preferred method of activating the gaseous precursor is by exposure to elevated temperature. Activation or transition temperature, and like terms, refer to the boiling point of the gaseous precursor and is the temperature at which the liquid to gaseous phase transition of the gaseous precursor takes place. Useful gaseous precursors are those materials which have boiling points in the range of about -100°C to about 70°C. The activation temperature is particular to each gaseous precursor. An activation temperature of about 37°C, or about human body temperature, is preferred for gaseous precursors in the context of the present invention. Thus, in preferred form, a liquid gaseous precursor is activated to become a gas at about 37°C or below. The gaseous precursor may be in liquid or gaseous phase for use in the methods of the present invention.

The methods of preparing the gaseous precursor filled vesicles may be carried out below the boiling point of the gaseous precursor such that a liquid is incorporated, for example, into a vesicle. In addition, the methods may be conducted at the boiling point of the gaseous precursor, such that a gas is incorporated, for example, into a vesicle. For gaseous precursors having low temperature boiling points, liquid precursors may be emulsified using a microfluidizer device chilled to a low temperature. The boiling points may also be depressed using solvents in liquid media to utilize a precursor in liquid form. Further, the methods may be performed where the temperature is increased throughout the process, whereby the process starts with a gaseous precursor as a liquid and ends with a gas.

The gaseous precursor may be selected so as to form the gas *in situ* in the targeted tissue or fluid, *in vivo* upon entering the patient or animal, prior to use, during storage, or during manufacture. The methods of producing the temperature activated gaseous precursor filled vesicles may be carried out at a temperature below the boiling point of the gaseous precursor. In this embodiment, the gaseous precursor is entrapped within a vesicle such that the phase transition does not occur during manufacture. Instead, the gaseous precursor filled vesicles are manufactured in the liquid phase of the gaseous precursor. Activation of the phase transition may take place at any time as the temperature is allowed to exceed the boiling point of the precursor. Also, knowing the amount of liquid in a droplet of liquid gaseous precursor, the size of the vesicles upon attaining the gaseous state may be determined.

Alternatively, the gaseous precursors may be utilized to create stable gas filled vesicles which are preformed prior to use. In this embodiment, the gaseous precursor is added to a container housing a lipid composition at a temperature below the liquid-gaseous phase transition temperature of the respective gaseous precursor. As the temperature is increased, and an emulsion is formed between the gaseous precursor and liquid solution, the gaseous precursor undergoes transition from the liquid to the gaseous state. As a result of this heating and gas formation, the gas displaces the air in the head space above the liquid mixture so as to form gas filled vesicles which entrap the gas of the gaseous precursor, ambient gas (*e.g.* air), or coentrap gas state gaseous precursor and ambient air. This phase transition can be used for optimal mixing and formation of the contrast agent. For example, the gaseous precursor, perfluorobutane, can be entrapped in the lipid vesicles and as the temperature is raised beyond the boiling point of perfluorobutane (4°C), perfluorobutane gas is entrapped in the vesicles.

Accordingly, the gaseous precursors may be selected to form gas filled vesicles *in vivo* or may be designed to produce the gas filled vesicles *in situ*, during the manufacturing process, on storage, or at some time prior to use. A water bath, sonicator or hydrodynamic activation by pulling back the plunger of a syringe against a closed stopcock may be used to activate targeted gas filled vesicles from temperature-sensitive gaseous precursors prior to intravenous injection.

As a further embodiment of this invention, by pre-forming the gaseous precursor in the liquid state into an aqueous emulsion, the maximum size of the vesicle may be estimated by using the ideal gas law, once the transition to the gaseous state is effectuated. For the purpose of making gas filled vesicles from gaseous precursors, the gas phase is assumed to form instantaneously and substantially no gas in the newly formed vesicle has been depleted due to diffusion into the liquid, which is generally aqueous in nature. Hence, from a known liquid volume in the emulsion, one would be able to predict an upper limit to the size of the gas filled vesicle.

In embodiments of the present invention, a mixture of a lipid compound and a gaseous precursor, containing liquid droplets of defined size, may be formulated such that upon reaching a specific temperature, for example, the boiling point of the gaseous precursor, the droplets will expand into gas filled vesicles of defined size. The defined size represents an upper limit to the actual size because the ideal gas law cannot account for such factors as gas diffusion into solution, loss of gas to the atmosphere, and the effects of increased pressure.

The ideal gas law, which can be used for calculating the increase in the volume of the gas bubbles upon transitioning from liquid to gaseous states, is as follows:

$$PV = nRT$$

where: P is pressure in atmospheres (atm); V is volume in liters (L); n is moles of gas; T is temperature in degrees Kelvin (K); and R is the ideal gas constant (22.4 L-atm/K-mole).

With knowledge of volume, density, and temperature of the liquid in the mixture of liquids, the amount, for example, in moles, and volume of liquid precursor may be calculated which, when converted to a gas, will expand into a vesicle of known volume. The calculated volume will reflect an upper limit to the size of the gas filled vesicle, assuming instantaneous expansion into a gas filled vesicle and negligible diffusion of the gas over the time of the expansion.

Thus, for stabilization of the precursor in the liquid state in a mixture wherein the precursor droplet is spherical, the volume of the precursor droplet may be determined by the equation: Volume (spherical vesicle) = $4/3\ \pi r^3$, where r is the radius of the sphere.

Once the volume is predicted, and knowing the density of the liquid at the desired temperature, the amount of liquid gaseous precursor in the droplet may be determined. In more descriptive terms, the following can be applied:

$$V_{gas} = 4/3\pi(r_{gas})^3$$

by the ideal gas law,

$$PV=nRT$$

substituting reveals,

$$V_{gas} = nRT/P_{gas}$$

or,

$$n = 4/3[\pi r_{gas}^{\ 3}]P/RT \qquad (A)$$

amount $n = 4/3\ [\pi r_{gas}^{\ 3}\ P/RT] \bullet MW_n$

Converting back to a liquid volume

$$V_{liq} = [4/3\ [\pi r_{gas}^{\ 3}]\ P/RT] \bullet MW_n/D] \qquad (B)$$

where D is the density of the precursor.

Solving for the diameter of the liquid droplet,

$$diameter/2 = [3/4\pi\ [4/3 \bullet [\pi r_{gas}^{\ 3}]\ P/RT]\ Mw_n/D]^{1/3} \qquad (C)$$

which reduces to Diameter = $2[[r_{gas}^{\ 3}]\ P/RT\ [MW_n/D]]^{1/3}$.

As a further means of preparing vesicles of the desired size for use in the methods of the present invention, and with a knowledge of the volume and especially the radius of the liquid droplets, one can use appropriately sized filters to size the gaseous precursor droplets to the appropriate diameter sphere.

A representative gaseous precursor may be used to form a vesicle of defined size, for example, 10 μm diameter. In this example, the vesicle is formed in the bloodstream of a human being, thus the typical temperature would be 37°C or 310 K. At a pressure of 1 atmosphere and using the equation in (A), $7.54 \times 10^{-17}$ moles of gaseous precursor would be required to fill the volume of a 10 μm diameter vesicle.

Using the above calculated amount of gaseous precursor and 1-fluorobutane, which possesses a molecular weight of 76.11, a boiling point of 32.5°C and a density of 0.7789 g/mL at 20°C, further calculations predict that $5.74 \times 10^{-15}$ grams of this precursor would be required for a 10 μm vesicle. Extrapolating further, and with the knowledge of the density, equation (B) further predicts that $8.47 \times 10^{-16}$ mL of liquid precursor is necessary to form a vesicle with an upper limit of 10 μm.

Finally, using equation (C), a mixture, for example, an emulsion containing droplets with a radius of 0.0272 μm or a corresponding diameter of 0.0544 μm, is formed to make a gaseous precursor filled vesicle with an upper limit of a 10 μm vesicle.

An emulsion of this particular size could be easily achieved by the use of an appropriately sized filter. In addition, as seen by the size of the filter necessary to form gaseous precursor droplets of defined size, the size of the filter would also suffice to remove any possible bacterial contaminants and, hence, can be used as a sterile filtration as well.

This embodiment for preparing gas filled vesicles may be applied to all gaseous precursors activated by temperature. In fact, depression of the freezing point of the solvent system allows the use of gaseous precursors which would undergo liquid-to-gas phase transitions at temperatures below 0°C. The solvent system can be selected to provide a medium for suspension of the gaseous precursor. For example, 20% propylene glycol miscible in buffered saline

exhibits a freezing point depression well below the freezing point of water alone. By increasing the amount of propylene glycol or adding materials such as sodium chloride, the freezing point can be depressed even further.

The selection of appropriate solvent systems may be determined by physical methods as well. When substances, solid or liquid, herein referred to as solutes, are dissolved in a solvent, such as water based buffers, the freezing point is lowered by an amount that is dependent upon the composition of the solution. Thus, as defined by Wall, one can express the freezing point depression of the solvent by the following equation:

$$\ln x_a = \ln (1 - x_b) = \Delta H_{fus}/R(1/T_o - 1/T)$$

where $x_a$ is the mole fraction of the solvent; $x_b$ is the mole fraction of the solute; $\Delta H_{fus}$ is the heat of fusion of the solvent; and $T_o$ is the normal freezing point of the solvent.

The normal freezing point of the solvent can be obtained by solving the equation. If $x_b$ is small relative to $x_a$, then the above equation may be rewritten as:

$$x^b = \Delta H_{fus}/R[T - T_o/T_oT] \approx \Delta H_{fus}\Delta T/RT_o^{\ 2}$$

The above equation assumes the change in temperature $\Delta T$ is small compared to $T_2$. This equation can be simplified further by expressing the concentration of the solute in terms of molality, m (moles of solute per thousand grams of solvent). Thus, the equation can be rewritten as follows.

$$X_b = m/[m+ 1000/m_a] \approx mMa/1000$$

where Ma is the molecular weight of the solvent.

Thus, substituting for the fraction $x_b$:

$$\Delta T = [M_aRT_o^{\ 2}/1000\Delta H_{fus}]m$$

or

$$\Delta T = K_fm, \text{ where } K_f=M_aRT_o^{\ 2}/1000\Delta H_{fus}$$

$K_f$ is the molal freezing point and is equal to 1.86 degrees per unit of molal concentration for water at one atmosphere pressure. The above equation may be used to accurately determine the molal freezing point of solutions of gaseous-precursor filled vesicles. Accordingly, the above equation can be applied to estimate freezing point depressions and to determine the appropriate concentrations of liquid or solid solute necessary to depress the solvent freezing temperature to an appropriate value.

Methods of preparing the temperature activated gaseous precursor filled vesicles include:

(a) vortexing and/or shaking an aqueous mixture of gaseous precursor and additional materials as desired, including, for example, carrier materials, thickening agents and/or dispersing agents. Optional variations of this method include autoclaving before vortexing or shaking; heating an aqueous mixture of gaseous precursor; venting the vessel containing the mixture/suspension; shaking or permitting the gaseous precursor filled vesicle to form spontaneously and cooling down the suspension of gaseous precursor filled vesicles; and extruding an aqueous suspension of gaseous precursor through a filter of about 0.22 μm. Alternatively, filtering may be performed during *in vivo* administration of the vesicles such that a filter of about 0.22 μm is employed;
(b) microemulsification whereby an aqueous mixture of gaseous precursor is emulsified by agitation and heated to form, for example, vesicles prior to administration to a patient;
(c) heating a gaseous precursor in a mixture, with or without agitation, whereby the less dense gaseous precursor filled vesicles float to the top of the solution by expanding and displacing other vesicles in the vessel and venting the vessel to release air; and
(d) utilizing in any of the above methods a sealed vessel to hold the aqueous suspension of gaseous precursor and maintaining the suspension at a temperature below the phase transition temperature of the gaseous precursor, followed by autoclaving to raise the temperature above the phase transition temperature, optionally with shaking, or permitting the gaseous precursor vesicle to form spontaneously, whereby the expanded gaseous precursor m the sealed vessel increases the pressure in the vessel, and cooling down the gas filled vesicle suspension, after which shaking may also take place.

Freeze drying is useful to remove water and organic materials prior to the shaking installation method. Drying installation methods may be used to remove water from vesicles. By pre-entrapping the gaseous precursor in the dried vesicles (i.e. prior to drying) after warming, the gaseous precursor may expand to fill the vesicle. Gaseous precursors can also be used to fill dried vesicles after they have been subjected to vacuum. As the dried vesicles are kept at a temperature below their gel state to liquid crystalline temperature, the drying chamber can be slowly filled with the gaseous precursor in its gaseous state. For example, perfluorobutane can be used to fill dried vesicles at temperatures above 4°C (the boiling point of perfluorobutane).

Preferred methods for preparing the temperature activated gaseous precursor filled vesicles comprise shaking an aqueous solution having a lipid compound in the presence of a gaseous precursor at a temperature below the liquid state to gas state phase transition temperature of the gaseous precursor. This is preferably conducted at a temperature below the gel state to liquid crystalline state phase transition temperature of the lipid. The mixture is then heated to a temperature above the liquid state to gas state phase transition temperature of the gaseous precursor which causes the precursor to volatilize and expand. Heating is then discontinued, and the temperature of the mixture is then allowed to drop below the liquid state to gas state phase transition temperature of the gaseous precursor. Shaking of the mixture may take place during the heating step, or subsequently after the mixture is allowed to cool.

Other methods for preparing gaseous precursor filled vesicles can involve shaking an aqueous solution of, for example, a lipid and a gaseous precursor, and separating the resulting gaseous precursor filled vesicles.

Conventional, aqueous-filled liposomes of the prior art are routinely formed at a temperature above the phase transition temperature of the lipids used to make them, since they are more flexible and thus useful in biological systems in the liquid crystalline state. See, for example, Szoka and Papahadjopoulos, *Proc. Natl. Acad. Sci.* (1978) *75*:4194-4198. In contrast, the vesicles made according to certain preferred embodiments described herein are gaseous precursor filled, which imparts greater flexibility, since gaseous precursors after gas formation are more compressible and compliant than an aqueous solution.

The methods contemplated by the present invention provide for shaking an aqueous solution comprising a lipid, in the presence of a temperature activatable gaseous precursor. Preferably, the shaking is of sufficient force such that a foam is formed within a short period of time, such as about 30 minutes, and preferably within about 20 minutes, and more preferably, within about 10 minutes. The shaking may involve, for example, microemulsifying, microfluidizing, swirling (such as by vortexing), side-to-side, or up and down motion. In the case of the addition of gaseous precursor in the liquid state, sonication may be used in addition to the shaking methods set forth above. Further, different types of motion may be combined. Also, the shaking may occur by shaking the container holding the aqueous lipid solution, or by shaking the aqueous solution within the container without shaking the container itself. Further, the shaking may occur manually or by machine. Mechanical shakers that may be used include, for example, the mechanical shakers described hereinbefore, with an Espe Capmix (Seefeld, Oberay Germany) being preferred. Another means for producing shaking includes the action of gaseous precursor emitted under high velocity or pressure. Alternatively, the mixture may be spray dried, and the agitation may also be conducted in supercooled carbon dioxide at the critical point.

According to the methods described herein, a gas, such as air, may also be provided by the local ambient atmosphere. The local ambient atmosphere can include the atmosphere within a sealed container, as well as the external environment. Alternatively, for example, a gas may be injected into or otherwise added to the container having the aqueous lipid solution or into the aqueous lipid solution itself to provide a gas other than air. Gases that are lighter than air are generally added to a sealed container, while gases heavier than air can be added to a sealed or an unsealed container. Accordingly, the present invention includes co-entrapment of air and/or other gases along with gaseous precursors.

Hence, the gaseous precursor filled vesicles can be used in substantially the same manner as the gas filled vesicles described herein, once activated by application to the tissues of a host, where such factors as temperature or pH may be used to cause generation of the gas. It is preferred that the gaseous precursors undergo phase transitions from liquid to gaseous states at or near the normal body temperature of the host, and are thereby activated, for example, by the *in vivo* temperature of the host so as to undergo transition to the gaseous phase therein. Alternatively, activation prior to intravenous injection may be used, for example, by thermal, mechanical or optical means. This activation can occur where, for example, the host tissue is human tissue having a normal temperature of about 37°C and the gaseous precursors undergo phase transitions from liquid to gaseous states near 37°C.

In any of the techniques described above for the preparation of lipid-based vesicles, the targeting ligands may be incorporated with the lipids before, during or after formation of the vesicles, as would be apparent to one of ordinary skill in the art, in view of the present disclosure.

For example, conjugates of targeting ligands and fluorinated surfactants can be synthesized by variations on a theme suggested by the reaction sequence set forth in the present disclosure and according to methods known to those skilled in the art, as disclosed, for example, by Quay, et al, European Patent Publication EP 0 727 225 A2, the disclosure of which is hereby incorporated herein by reference in its entirety. If the targeting ligand of choice contains a fluorinated surfactant, such as ZONYL® FSN-100, the ZONYL® can be heated at reduced pressure to drive off volatile components, then the oily residue is reacted with a conjugation linker, the choice of which will ultimately depend on the

chemistry of the functional groups on the ligand. For example, targeting ligand and fluorinated surfactant conjugates can be prepared by the reaction schemes below, where "LIG" refers to a targeting ligand as described herein and "$R_f$" refers to a fluorinated surfactant as described herein.

$$R(CH_2CH_2O)_xCOCl + LIG\text{-}NH_2 \rightarrow R(CH_2CH_2O)_xCONH\text{-}LIG$$

$$R(OCH_2CH_2)_xCOCl + LIG\text{-}OH \rightarrow R(CH_2CH_2O)_xCO_2\text{-}LIG$$

$$RCH_2CH_2(OCH_2CH_2)_xSH + LIG\text{-}SH + ½\ O_2 \rightarrow RCH_2CH_2(OCH_2CH_2)_xSS\text{-}LIG$$

$$RSO_2Cl + LIG\text{-}NH_2 \rightarrow RSO_2NH\text{-}LIG$$

$$LIG\text{-}CHO + RCH_2CH_2(OCH_2CH_2)_xNH_2 + NaCNBH_3 \rightarrow RCH_2CH_2(OCH_2CH_2)_xNH\text{-}LIG$$

$$LIG\text{-}Br + RCH_2CH_2(OCH_2CH_2)_xSH \rightarrow RCH_2CH_2(OCH_2CH_2)_xS\text{-}LIG$$

$$LIG\text{-}Br + RCH_2 + Bu_3SnH \rightarrow RCH_2CH_2\text{-}LIG$$

$$RCOCl + LIG\text{-}NH_2 \rightarrow RCONH\text{-}LIG$$

$$RNCO + LIG\text{-}NH_2 \rightarrow RNCONH\text{-}LIG$$

$$LIG\text{-}CHO + RCH_2CH_2(OCH_2CH_2)_xNH_2 \rightarrow RCH_2CH_2(OCH_2CH_2)_xNH\text{-}LIG + RCO \rightarrow$$

$$(RCH_2CH_2(OCH_2CH_2)_x)(R_fCO)N\text{-}LIG$$

It is contemplated that targeting ligands, such as Sialyl Lewis X, can be conjugated with a wide variety of stablizing materials including, for example, poloxamers, polythylene glycols, fatty acids, fatty amines, fatty alcohols, and lipids, including phospholipids, such as phosphatidyl cholines, employing the exemplary synthetic methodology outlined above.

With respect to polyethylene glycol containing fragments, the following can be used, for example, PEG2-NHS ester, NHS-PEG-VS, NHS-PEG-MAL, methoxy-PEG-vinylsulfone, PEG-$(VS)_2$, methoxy-PEG-ald, PEG-$(ald)_2$, methoxy-PEG-epx, PEG-$(epx)_2$, methoxy-PEG-Tres, PEG-$(Tres)_2$, methoxy-PEG-NPC, PEG-$(NPC)_2$, methoxy-PEG-CDI, PEG-$(CDI)_2$, mPEG-Gly-OSu, mPEG-NLe-OSu, methoxy-SPA-PEG, $(SPA)_2$-PEG, methoxy-SS-PEG, $(SS)_2$-PEG all of which are commercially available from Shearwater Polymers, Inc. (Huntsville, Alabama). Where these types of fragments are used, *i.e.*, where the fragments may not themselves have surfactant properties adequate for a given ultrasound contrast formulation, or act only weakly as surfactants, the conjugate formed can be used in conjunction with other surfactants in the final formulation.

Vesicle compositions which comprise vesicles formulated from proteins, such as albumin vesicles, may be prepared by various processes, as will be readily apparent to those skilled in the art in view of the present disclosure. Suitable methods include those described, for example, in U.S. Patent Nos. 4,572,203, 4,718,433, 4,774,958, and 4,957,656, the disclosures of each of which are hereby incorporated herein by reference in their entirety. Included among the methods are those which involve sonicating a solution of a protein. In preferred form, the starting material may be an aqueous solution of a heat-denaturable, water-soluble biocompatible protein. The encapsulating protein is preferably heat-sensitive so that it can be partially insolubilized by heating during sonication. Suitable heat-sensitive proteins include, for example, albumin, hemoglobin, and collagen, preferably, the protein is a human protein, with human serum albumin (HSA) being more preferred. HSA is available commercially as a sterile 5% aqueous solution, which is suitable for use in the preparation of protein-based vesicles. As would be apparent to one of ordinary skill in the art, other concentrations of albumin, as well as other proteins which are heat-denaturable, can be used to prepare the vesicles. Generally speaking, the concentration of HSA can vary and may range from about 0.1 to about 25% by weight, and all combinations and subcombinations of ranges therein. It may be preferable, in connection with certain methods for the preparation of protein-based vesicles, to utilize the protein in the form of a dilute aqueous solution. For albumin, it may be preferred to utilize an aqueous solution containing from about 0.5 to about 7.5% by weight albumin, with concentrations of less than about 5% by weight being preferred, for example, from about 0.5 to about 3% by weight.

Protein-based vesicles may be prepared using equipment which is commercially available. For example, in connection with a feed perparation operation as disclosed, for example, in U.S. Patent No. 4,957,656, stainless steel tanks which are commercially available from Walker Stainless Equipment Co. (New Lisbon, WI), and process filters which are commercially available from Millipore (Bedford, MA), may be utilized.

The sonication operation may utilize both a heat exchanger and a flow through sonciating vessel, in series. Heat exhanger equipment of this type may be obtained from ITT Standard (Buffalo, NY). The heat exchanger maintains operating temperature for the sonciation process, with temperature controls ranging from about 65°C to about 80° C, depending on the makeup of the media. The vibration frequency of the sonication equipment may vary over a wide range, for example, from about 5 to about 40 kilohertz (kHz), with a majority of the commerically available sonicators operating at about 10 or 20 kHz. Suitable sonicating equipment include, for example, a Sonics & Materials Vibra-Cell, equipped with a flat-tipped sonicator horn, commercially available from Sonics & Materials, Inc. (Danbury, CT). The power applied to the sonicator horn can be varied over power settings scaled from 1 to 10 by the manufacturer, as with Sonics & Materials Vibra-Cell Model VL1500. An intermediate power setting, for example, from 5 to 9, can be used. It is preferred that the vibrational frequency and the power supplied be sufficeint to produce cavitation in the liquid being sonicated. Feed flow rates may range from about 50 mL/min to about 1000 mL/min, and all combinations and subcombinations of ranges therein. Residence times in the sonication vessel can range from about 1 second to about 4 minutes, and gaseous fluid addition rates may range from about 10 cubic centimeters (cc) per minute to about 100 cc/min, or 5% to 25% of the feed flow rate, and all combinations and subcombinations of ranges therein.

It may be preferable to carry out the sonication in such a manner to produce foaming, and especially intense foaming, of the solution. Generally speaking, intense foaming and aerosolating are important for obtaining a contrast agent having enhanced concentration and stability. To promote foaming, the power input to the sonicator horn may be increased, and the process may be operated under mild pressure, for example, about 1 to about 5 psi. Foaming may be easily detected by the cloudy appearance of the solution, and by the foam produced.

Suitable methods for the preparation of protein-based vesicles may also involve physically or chemically altering the protein or protein derivative in aqueous solution to denature or fix the material. For example, protein-based vesicles may be prepared from a 5% aqueous solution of HSA by heating after formation or during formation of the contrast agent via sonication. Chemical alteration may involve chemically denaturing or fixing by binding the protein with a difunctional aldehyde, such as gluteraldehyde. For example, the vesicles may be reacted with 0.25 grains of 50% aqueous gluteradehyde per gram of protein at pH 4.5 for 6 hours. The unreacted gluteraldehyde may then be washed away from the protein.

In any of the techniques described above for the preparation of protein-based stabilizing materials and/or vesicles, the targeting ligands may be incorporated with the proteins before, during or after formation of the vesicles, as would be apparent to one of ordinary skill in the art, based on the present disclosure.

Vesicle compositions which comprise vesicles formulated from polymers may be prepared by various processes, as will be readily apparent to those skilled in the art in view of the present disclosure. Exemplary processes include, for example, interfacial polymerization, phase separation and coacervation, multiorifice centrifugal preparation, and solvent evaporation. Suitable procedures which may be employed or modified in accordance with the present disclosure to prepare vesicles from polymers include those procedures disclosed in U.S. Patent Nos. 4,179,546, 3,945,956, 4,108,806, 3,293,114, 3,401,475, 3,479,811, 3,488,714, 3,615,972, 4,549,892, 4,540,629, 4,421,562, 4,420,442, 4,898,734, 4,822,534, 3,732,172, 3,594,326, and 3,015,128; Japan Kokai Tokkyo Koho 62 286534, British Patent No. 1,044,680, Deasy, *Microencapsulation and Related Drug Processes*, Vol. 20, Chs. 9 and 10, pp. 195-240 (Marcel Dekker, Inc., N.Y., 1984), Chang et al., *Canadian J. of Physiology and Pharmacology*, *44*:115-129 (1966), and Chang, *Science*, *146*:524-525 (1964), the disclosures of each of which are hereby incorporated herein by reference in their entirety.

In accordance with a preferred synthesis protocol, the vesicles may be prepared using a heat expansion process, such as, for example, the process described in U.S. Patent Nos. 4,179,546, 3,945,956, and 4,108,806, British Patent No. 1,044,680, and Japan Kokai Tokkyo Koho 62 286534. In general terms, the heat expansion process may be carried out by preparing vesicles of an expandable polymer or copolymer which may contain in their void (cavity) a volatile liquid (gaseous precursor). The vesicle may then be heated, plasticising the vesicle and converting the volatile liquid into a gas, causing the vesicle to expand to up to about several times its original size. When the heat is removed, the thermoplastic polymer may retain at least some of its expanded shape. Vesicles produced by this process are typically of particularly low density, and are thus preferred. The foregoing described process is well known in the art, and may be referred to as the heat expansion process for preparing low density vesicles.

Polymers useful in the heat expansion process will be readily apparent to those skilled in the art and include thermoplastic polymers or copolymers, including polymers or copolymers of many of the monomers described above. Preferable of the polymers and copolymers described above include the following copolymers: polyvinylidene-polyacrylo-nitrile, polyvinylidene-polyacrylonitrile-polymethylmethacrylate, and polystyrene-polyacrylonitrile. A particularly preferred copolymer is polyvinylidene-polyacrylonitrile.

Volatile liquids useful in the heat expansion process will also be well known to those skilled in the art and include: aliphatic hydrocarbons such as ethane, ethylene, propane, propene, butane, isobutane, neopentane, acetylene, hexane, heptane; chlorofluorocarbons such as $CCl_3F$, $CCl_2F_3$, $CClF_3$, $CClF_2$-$CCl_2F_2$, chloroheptafluorocyclobutane, and 1 ,2-dichlorohexafluorocyclobutane; tetraalkyl silanes, such as tetramethyl silane, trimethylethyl silane,

trimethylisopropyl silane, and trimethyl n-propyl silane; as well as perfluorocarbons, including the perfluorocarbons described above. In general, it is important that the volatile liquid not be a solvent for the polymer or copolymer being utilized. It is also preferred that the volatile liquid have a boiling point that is below the softening point of the involved polymer or copolymer. Boiling points of various volatile liquids and softening points of various polymers and copolymers will be readily ascertainable to one skilled in the art, and suitable combinations of polymers or copolymers and volatile liquids will be easily apparent to the skilled artisan. By way of guidance, and as one skilled in the art would recognize, generally as the length of the carbon chain of the volatile liquid increases, the boiling point of that liquid increases also. Also, mildly preheating the vesicles in water in the presence of hydrogen peroxide prior to definitive heating and expansion may pre-soften the vesicle to allow expansion to occur more readily.

For example, to produce vesicles from synthetic polymers, vinylidene and acrylonitrile may be copolymerized in a medium of isobutane liquid using one or more of the foregoing modified or unmodified literature procedures, such that isobutane becomes entrapped within the vesicles. When such vesicles are then heated to a temperature of from about 80°C to about 120°C, the isobutane gas expands, which in turn expands the vesicles. After heat is removed, the expanded polyvinylidene and acrylonitrile copolymer vesicles remain substantially fixed in their expanded position. The resulting low density vesicles are extremely stable both dry and suspended in an aqueous media. Isobutane is utilized herein merely as an illustrative liquid, with the understanding that other liquids which undergo liquid/gas transitions at temperatures useful for the synthesis of these vesicles and formation of the very low density vesicles upon heating can be substituted for isobutane. Similarly, monomers other than vinylidene and acrylonitrile may be employed in preparing the vesicles.

In certain preferred embodiments, the vesicles which are formulated from synthetic polymers and which may be employed in the methods of the present invention are commercially available from Expancel, Nobel Industries (Sundsvall, Sweden), including EXPANCEL 551 DE™ microspheres. The EXPANCEL 551 DE™ microspheres are composed of a copolymer of vinylidene and acrylonitrile which have encapsulated therein isobutane liquid. Such microspheres are sold as a dry composition and are approximately 50 microns in size. The EXPANCEL 551 DE™ microspheres have a specific gravity of only 0.02 to 0.05, which is between one-fiftieth and one-twentieth the density of water.

In any of the techniques described above for the preparation of polymer-based vesicles, the targeting ligands may be incorporated with the polymers before, during or after formation of the vesicles, as would be apparent to one of ordinary skill in the art, based on the present disclosure.

As those skilled in the art will recognize, any of the vesicle compositions may be lyophilized for storage, and reconstituted or rehydrated, for example, with an aqueous medium (such as sterile water, phosphate buffered solution, or aqueous saline solution), with the aid of vigorous agitation. Lyophilized preparations generally have the advantage of greater shelf life. To prevent agglutination or fusion of the lipids and/or vesicles as a result of lyophilization, it may be useful to include additives which prevent such fusion or agglutination from occurring. Additives which may be useful include sorbitol, mannitol, sodium chloride, glucose, dextrose, trehalose, polyvinyl-pyrrolidone and poly(ethylene glycol) (PEG), for example, PEG 400. These and other additives are described in the literature, such as in the U.S. Pharmacopeia, USP XXII, NF XVII, The United States Pharmacopeia, The National Formulary, United States Pharmacopeial Convention Inc., 12601 Twinbrook Parkway, Rockville, MD 20852, the disclosures of which are hereby incorporated herein by reference in their entirety.

The concentration of lipid required to form a desired stabilized vesicle level will vary depending upon the type of lipid used, and may be readily determined by routine experimentation. For example, in preferred embodiments, the concentration of 1,2-dipalmitoylphosphatidylcholine (DPPC) used to form stabilized vesicles according to the methods of the present invention is about 0.1 mg/mL to about 30 mg/mL of saline solution, more preferably from about 0.5 mg/mL to about 20 mg/mL of saline solution, and even more preferably from about 1 mg/mL to about 10 mg/mL of saline solution. The concentration of distearoylphosphatidylcholine (DSPC) used in preferred embodiments is about 0.1 mg/mL to about 30 mg/mL of saline solution, more preferably from about 0.5 mg/mL to about 20 mg/mL of saline solution, and even more preferably from about 1 mg/mL to about 10 mg/mL of saline solution. The amount of composition which is administered to a patient can vary. Typically, the intravenous dose may be less than about 10 mL for a 70 Kg patient, with lower doses being preferred.

Another embodiment of preparing a targeted therapeutic vesicle composition comprises combining at least one biocompatible lipid and a gaseous precursor; agitating until gas filled vesicles are formed; adding a targeting ligand to the gas filled vesicles such that the targeting ligand binds to the gas filled vesicle by a covalent bond or non-covalent bond; and agitating until a delivery vehicle comprising gas filled vesicles and a targeting ligand result. Rather than agitating until gas filled vesicles are formed before adding the targeting ligand, the gaseous precursor may remain a gaseous precursor until the time of use. That is, the gaseous precursor is used to prepare the delivery vehicle and the precursor is activated *in vivo*, by temperature for example.

Alternatively, a method of preparing targeted therapeutic vesicle compositions may comprise combining at least one biocompatible lipid and a targeting ligand such that the targeting ligand binds to the lipid by a covalent bond

or non-covalent bond, adding a gaseous precursor and agitating until a delivery vehicle comprising gas-filled vesicles and a targeting ligand result. In addition, the gaseous precursor may be added and remain a gaseous precursor until the time of use. That is, the gaseous precursor may be used to prepare the delivery vehicle having gaseous precursor filled vesicles and a targeting ligand which result for use *in vivo*.

Alternatively, the gaseous precursors may be utilized to create stable gas filled vesicles with targeting ligands which are pre-formed prior to use. In this embodiment, the gaseous precursor and targeting ligand may be added to a container housing a suspending and/or stabilizing medium at a temperature below the liquid-gaseous phase transition temperature of the respective gaseous precursor. As the temperature is then increased, and an emulsion is formed between the gaseous precursor and liquid solution, the gaseous precursor undergoes transition from the liquid to the gaseous state. As a result of this heating and gas formation, the gas displaces the air in the head space above the liquid suspension so as to form gas filled lipid spheres which entrap the gas of the gaseous precursor, ambient gas for example, air, or coentrap gas state gaseous precursor and ambient air. This phase transition can be used for optimal mixing and stabilization of the delivery vehicle. For example, the gaseous precursor, perfluorobutane, can be entrapped in the biocompatible lipid or other stabilizing compound, and as the temperature is raised, beyond 4°C (boiling point of perfluorobutane) carrier material entrapped fluorobutane gas results. As an additional example, the gaseous precursor fluorobutane, can be suspended in an aqueous suspension containing emulsifying and carrier materials, such as glycerol or propylene glycol and vortexed on a commercial vortexer. Vortexing may be commenced at a temperature low enough that the gaseous precursor is liquid and is continued as the temperature of the sample is raised past the phase transition temperature from the liquid to gaseous state. In so doing, the precursor converts to the gaseous state during the microemulsification process. In the presence of the appropriate carrier material, surprisingly stable gas filled vesicles and targeting ligand result.

Accordingly, the gaseous precursors may be selected to form a gas filled vesicle *in vivo* or may be designed to produce the gas filled vesicle *in situ*, during the manufacturing process, on storage, or at some time prior to use.

According to the methods contemplated by the present invention, the presence of gas, such as and not limited to air, may also be provided by the local ambient atmosphere. The local ambient atmosphere may be the atmosphere within a sealed container, or in an unsealed container, may be the external environment. Alternatively, for example, a gas may be injected into or otherwise added to the container having the aqueous lipid solution or into the aqueous lipid solution itself in order to provide a gas other than air. Gases that are not heavier than air may be added to a sealed container while gases heavier than air may be added to a sealed or an unsealed container. Accordingly, the present invention includes co-entrapment of air and/or other gases along with gaseous precursors.

Hence, the stabilized vesicle precursors described above, can be used in the same manner as the other stabilized vesicles used in the present invention, once activated by application to the tissues of a host, where such factors as temperature may be used to cause generation of the gas. It is preferred that this embodiment is one wherein the gaseous precursors undergo phase transitions from liquid to gaseous states at or near the normal body temperature of said host, and are thereby activated by the temperature of said host tissues so as to undergo transition to the gaseous phase therein. More preferably still, this method is one wherein the host tissue is human tissue having a normal temperature of about 37°C, and wherein the gaseous precursors undergo phase transitions from liquid to gaseous states near 37°C.

All of the above embodiments involving preparations of the stabilized gas filled vesicles used in the present invention, may be sterilized by autoclave or sterile filtration if these processes are performed before either the gas instillation step or prior to temperature mediated gas conversion of the temperature sensitive gaseous precursors within the suspension. Alternatively, one or more anti-bactericidal agents and/or preservatives may be included in the formulation of the compositions including, for example, sodium benzoate, all quaternary ammonium salts, sodium azide, methyl paraben, propyl paraben, sorbic acid, ascorbylpalmitate, butylated hydroxyanisole, butylated hydroxytoluene, chlorobutanol, dehydroacetic acid, ethylenediamine, monothioglycerol, potassium benzoate, potassium metabisulfite, potassium sorbate, sodium bisulfite, sulfur dioxide, and organic mercurial salts. Such sterilization, which may also be achieved by other conventional means, such as by irradiation, will be necessary where the stabilized microspheres are used for imaging under invasive circumstances, for example, intravascularly or intraperitoneally. The appropriate means of sterilization will be apparent to the artisan instructed by the present description of the stabilized gas filled vesicles and their use. The compositions are generally stored as an aqueous suspension but in the case of dried or lyophilized vesicles or dried or lyophilized lipidic spheres the compositions may be stored as a dried or lyophilized powder ready to be reconstituted or rehydrated prior to use.

Ultrasound can be used for both diagnostic and therapeutic purposes. Higher energy ultrasound, for example, ultrasound which is generated by therapeutic ultrasound equipment, is generally capable of causing rupture of the vesicle species. In general, devices for therapeutic ultrasound employ from about 10 to about 100% duty cycles, depending on the area of tissue to be treated with the ultrasound. Areas of the body which are generally characterized by larger amounts of muscle mass, for example, backs and thighs, as well as highly vascularized tissues, such as heart tissue, may require a larger duty cycle, for example, up to about 100%.

In therapeutic ultrasound, continuous wave ultrasound is used to deliver higher energy levels. For the rupture of vesicles, continuous wave ultrasound is preferred, although the sound energy may be pulsed also. If pulsed sound energy is used, the sound will generally be pulsed in echo train lengths of from about 8 to about 20 or more pulses at a time. Preferably, the echo train lengths are about 20 pulses at a time. In addition, the frequency of the sound used may vary from about 0.01 to about 100 megahertz (MHz). In general, frequency for therapeutic ultrasound preferably ranges between about 0.75 and about 3 MHz, with from about 1 and about 2 MHz being more preferred. Additional preferred ranges include, for example, 10 kHz to 25 MHz, 200 kHz to 500 kHz, and 20 kHz to 20 MHZ. Eenergy levels may vary from about 0.1 Waft (W) per square centimeter ($cm^2$) to about 10.0 $W/cm^2$. In addition, energy levels may vary from about 0.5 Watt (W) per square centimeter ($cm^2$) to about 5.0 $W/cm^2$, with energy levels of from about 0.5 to about 2.5 $W/cm^2$ being preferred. Additional preferred ranges include, for example, 200 $mW/cm^2$ to 500 $mW/cm^2$, and 100 $mW/cm^2$ to 200 $mW/cm^2$. Energy levels for therapeutic ultrasound involving hyperthermia are generally from about 5 $W/cm^2$ to about 50 $W/cm^2$. For very small vesicles, for example, vesicles having a diameter of less than about 0.5 µm, higher frequencies of sound are generally preferred. This is because smaller vesicles are capable of absorbing sonic energy more effectively at higher frequencies of sound. When very high frequencies are used, for example, greater than about 10 MHz, the sonic energy will generally penetrate fluids and tissues to a limited depth only. Thus, external application of the sonic energy may be suitable for skin and other superficial tissues. However, it is generally necessary for deep structures to focus the ultrasonic energy so that it is preferentially directed within a focal zone. Alternatively, the ultrasonic energy may be applied via interstitial probes, intravascular ultrasound catheters or endoluminal catheters.

A therapeutic ultrasound device may be used which employs two frequencies of ultrasound. The first frequency may be x, and the second frequency may be 2x. In preferred form, the device would be designed such that the focal zones of the first and second frequencies converge to a single focal zone. The focal zone of the device may then be directed to the targeted compositions, for example, targeted vesicle compositions, within the targeted tissue. This ultrasound device may provide second harmonic therapy with simultaneous application of the x and 2x frequencies of ultrasound energy. It is contemplated that, in the case of ultrasound involving vesicles, this second harmonic therapy may provide improved rupturing of vesicles as compared to ultrasound energy involving a single frequency. Also, it is contemplated that the preferred frequency range may reside within the fundamental harmonic frequencies of the vesicles. Lower energy may also be used with this device. An ultrasound device which may be employed in connection with the aforementioned second harmonic therapy is described, for example, in Kawabata, K. et al., *Ultrasonics Sonochemistry*, Vol. 3, pp. 1-5 (1996), the disclosures of which are hereby incorporated herein by reference, in their entirety.

The invention is further demonstrated in the following examples. Examples 5 and 6 are actual examples, and Examples 1 to 4, 7 to 12 and 14 are prophetic examples. Example 13 is both actual (in part) and prophetic (in part). The examples, however, are not intended to in any way limit the scope of the present invention.

## EXAMPLES

### Example 1

This example is directed to the preparation of DPGS-PEG-Pro-Lys-Lys-Lys-Arg-Lys-Val (DPGS-PEG-PKKKRKV).

A. Preparation of PKKKRKV on a photolabile resin.

3-Nitro-4-bromometylbenzoylamide polystyrene resin will be prepared as described in Rich, D.H. and Gurwara, S.K., *J. Am. Chem. Soc., 97*:6, 1575-1579, March 19, 1975, the disclosures of which are hereby incorporated herein by reference, in their entirety. To this resin will be added Boc-Valine (Bachem, Torrance, CA) (0.6 mmole, 1 equiv.) with diisopropylethylamine (Mallinckrodt, St. Louis, MO) (0.6 mmole, 1 eq.) in ethyl acetate (Mallinckrodt, St. Louis, MO) and refluxing. The remaining protected amino acids will then be coupled using standard coupling methodology. Deprotection of the Boc group will be achieved by exposing the resin to 45 % trifluoroacetic acid (TFA) in anhydrous methylene chloride (v:v) for 2 minutes followed by removal of the acid. A second rinse with 45% $TFA/CH_2Cl_2$ for 20 minutes will then be performed. The resin will be washed twice with $CH_2Cl_2$ and neutralized with 2 x 2 minute additions of 10% diisopropylethylamine (DIEA) (Mallinckrodt, St. Louis, MO) in $CH_2Cl_2$ followed by 2 x 2 minute rinses with $CH_2Cl_2$. Amino acids will be coupled to the amino terminus of the deprotected Valine residue via the addition of the appropriate Boc-amino acid (1.2 mmoles, 2 equiv.) and diisopropylcarbodiimide (DIC) (Aldrich Chemical, Milwaukee, WI) (1.2 mmole, 2 equiv.), hydroxybenzotriazole (HOBT) (Aldrich, Milwaukee, WI) (1.2 equiv.) and N-methylpyrrolidone (Mallinckrodt, St. Louis, MO). Coupling will be monitored for completion using the method of Kaiser et al., *Anal. Biochem*., 34, 595, 1970, the disclosures of which are hereby incorporated herein by reference, in their entirety. Incomplete couplings will require a repeat coupling. After coupling, the resin will be washed with 2 x 2 minute washes of $CH_2Cl_2$. The remaining protected amino acids will be coupled in the same manner to provide the PKKKRKV-resin complex.

B. Preparation of t-Boc-α-amino-ω-carboxy polyethylene glycol (t-Boc-α-amino-ω-carboxy-PEG).

To a solution of dioxane:water (1:1, v:v) will be added α-amino-ω-carboxy polyethylene glycol (MW 3350) (Shearwater Chemicals, Alabaster, AL), t-butyloxy carbonyloxy anhydride (Aldrich Chemical, Milwaukee, WI) (1 equivalent). The reaction mixture will be stirred at room temperature for two days, then concentrated *in vacuo*. The residue will be dissolved in ethyl acetate and the organic solution will then be added to a separatory funnel and washed 3 times with a 5% (w:v) solution of aqueous citric acid (Mallinckrodt, St. Louis, MO). The aqueous layer will be removed and discarded and the ethyl acetate layer will be dried ($MgSO_4$), filtered and concentrated *in vacuo.* The resulting t-Boc protected product will be stored in the refrigerator and tested for complete protection using the method of Kaiser. The product will then be used for further coupling.

C. Preparation of t-Boc-α-amino-ω-carboxy-PEG-PKKKRKV-resin complex.

The deprotected amino acid resin complex prepared in Step A will be coupled with the t-Boc-α-amino-ω-carboxy-PEG prepared in Step B using standard procedures. The completeness of the coupling will be analyzed utilizing the methods of Kaiser.

D. Preparation of DPGS-PEG-PKKKRKV-resin complex.

The t-Boc-α-amino-ω-carboxy-PEG-PKKKRKV-resin complex from Step C will be deprotected using the same procedure as used in Step A. The deprotected α-amino-ω-carboxy-PEG-PKKKRKV-resin complex will then be reacted with dipalmitoylphosphoglycerol succinate (Avanti, Alabaster, AL) by the addition of diisopropylcarbodiimide (DIC) (2 equiv., Aldrich Chemical, Milwaukee, WI) and N-methylpyrrolidone (Mallinckrodt, St. Louis, MO). Completeness of reaction will be monitored by the method of Kaiser.

E. Removal of DPGS-PEG-PKKKRKV from photolabile resin.

The DPGS-PEG-PKKKRKV resin complex will be subjected to photolysis at 3500 Angstroms wavelength as described by Rich, D.H. and Gurwara, S.K., *J. Am. Chem. Soc*., 97:6, 1575-1579, March 19, 1975, the disclosures of which are hereby incorporated herein by reference, in their entirety. Briefly, this will involve placing a suspension of the resin in anhydrous methanol or ethanol in a flask surrounded by a jacket containng 40% $CuSO_4$ solution. The solution will be purged with $O_2$-free nitrogen for 2 hours under slight vacuum, followed by irradiation at 3500 Angstroms for 18 to 24 horus. The suspension will be filtered and washed three times for 2 min. with portions of ethanol, methylene chloride, 50% methylene chloride-ethanol, and ethanol. The filtrates will be combined and concentrated *in vacuo*. The product will then be purified using standard chromatography methods known in the art.

## Example 2

Example 1 will be repeated except that the following procedure will be substituted for Step A.

A. Preparation of a complex of a polymeric resin and the peptide PKKKRKV.

This alternative procedure will involve the use of the fluorenylmethoxycarbonyl (FMOC) protecting group on the α-amino moiety of the protected amino acids. In this case, the protected Valine (FMOC-Val) will be bound to the resin utilizing the same method in Example 1, using diisopropylethylamine (DIEA) and ethyl acetate with subsequent refluxing. Following binding of the FMOC-Val to the resin, further couplings will be initiated as follows.

i. Deprotection

The FMOC-Val resin complex will be washed 5 x 1 min with dimethylformide DMF or N-methylpyrollidone (NMP). The FMOC groups will be removed with 20% piperidine/NMP x 20 min. This will be repeated, followed by a wash 5 x 1 min with DMF or NMP.

ii. Coupling

An appropriate FMOC protected amino acid (2 equiv.) will be added with 2 equiv of DIC or dicyclohexylcarbodiimide (DCC), hydroxybenzotriazole (2 equiv.) and DMF or NMP. Completeness of the coupling reactions will be monitored using the ninhydrin detection method of Kaiser.

iii. Steps 1 and 2 will be repeated for coupling of additional FMOC-protected amino acids.

The peptide PKKKRKV requires only the sidechain protection of the lysines. A number of protecting group schemes are compatible with the synthetic scheme. For example, Boc-Lys with a fluorenylmethoxycarbonyl (FMOC) sidechain protecting group may be used to prevent the reaction of other amino acids, PEG, or the DPGS with the side chain group. In addition, a Boc-Asp with a β-carboxyfluorenylmethyl (OFm) ester bond may be used to protect the sidechain carboxyl. Prior to removal of the DPGS-PEG-PKKKRKV from the resin, mild conditions as 20% piperidine and N-methylpyrollidone can be initiated to remove both the FMOC group from the lysine.

In addition, a number of sidechain protecting groups may be used compatible with FMOC methodology yet require only mild conditions for removal. For example, FMOC-Asp-(O-Dmab)-OH and FMOC Lys-(Dde)-OH, where Dmab is 4-(N)-[1-(4,4-dimethyl-2,6-dioxocyclohexylidene)-3-methylbutyl]amino)benzyl ester and Dde is 1-(4,4 dimethyl-2,6-dioxocyclohex-1-ylidene)ethyl, can be used in part B of the synthesis with subsequent cleavage of the sidechain groups using 2% hydrazine in NMP, DMF, or $CH_2Cl_2$ for one hour. After removal of the sidechains, the DPGS-PEG-PKKKRKV will be washed with 2 x 2 min. NMP.

**Example 3**

This example is directed to the preparation of DPGS-PEG-LLIYFNNNVPIDDSGM.

A. Preparation of a complex of a polymeric resin and the peptide LLIYFINNNVPIDDSGM.

3-Nitro-4-bromometylbenzoylamide polystyrene resin will be prepared as described in Rich, D.H. and Gurwara, S.K., *J. Am. Chem. Soc.*, 97:6, 1575-1579, March 19, 1975, the disclosures of which are hereby incorporated herein by reference, in their entirety. To this resin will be added Boc-Methionine (Bachem, Torrance, CA) (0.6 mmole, 1 equiv.) with diisopropylethylamine (Mallinckrodt, St. Louis, MO) (0.6 mmole, 1 equiv) in ethyl acetate (Mallinckrodt, St. Louis, MO) and refluxing. The remaining protected amino acids will then be coupled using standard coupling methodology. Deprotection of the Boc-group will be achieved by exposing the resin to 45 % trifluoroacetic acid (TFA) in anhydrous methylene chloride (v:v) for 2 minutes followed by removal of the acid. Then, a second rinse with 45% TFA / $CH_2Cl_2$ for 20 minutes will be performed. The resin will then be washed with two rinses of $CH_2Cl_2$. The resin will be neutralized with 2 x 2 minute additions of 10% diisopropylethylamine (DIEA) (Mallinckrodt, St. Louis, MO) in $CH_2Cl_2$ followed by 2 x 2 minute rinses with $CH_2Cl_2$. Amino acids will be coupled to the amino terminus of the deprotected Methionine residue via the addition of 1.2 mmoles (2 equiv.) of the appropriate Boc-amino acid and 1.2 mmoles of diisopropylcarbodiimide (DIC) (1.2 mmole, 2 equiv., Aldrich Chemical, Milwaukee, WI), hydroxybenzotriazole (HOBT, Aldrich, Milwaukee, WI) (1.2 equiv) and N-methylpyrrolidone (Mallinckrodt, St. Louis, MO). Coupling will be monitored for completion using the method of Kaiser et al., *Anal. Biochem*., 34, 595, 1970, the disclosures of which are hereby incorporated herein by reference, in their entirety. Incomplete couplings will require a repeat coupling. After coupling, the resin will be washed with 2 x 2 minute washes of $CH_2Cl_2$. The remaining protected amino acids will be coupled in the same manner.

B. Preparation of t-Boc-α-amino-ω-carboxy polyethylene glycol (t-Boc-α-amino-ω-carboxy-PEG).

The t-Boc-α-amino-ω-carboxy-PEG will be prepared utilizing the same procedure as described in Step B of Example 1 above.

C. Preparation of t-Boc-α-amino-ω-carboxy-PEG-LLIYFNNNVPIDDSGM-resin complex.

The deprotected amino acid resin complex prepared in Step A will be coupled with the t-Boc-α-amino-ω-carboxy-PEG prepared in Step B using standard procedures. The completeness of the coupling will be analyzed utilizing the methods of Kaiser.

D. Preparation of DPGS-PEG-LLIYFNNNVPIDDSGM-resin complex.

The t-Boc-α-amino-ω-carboxy-PEG-LLIYFNNNVPIDDSGM-resin complex from Step C will be deprotected using the same procedure as used in Step A. The deprotected α-amino-ω-carboxy-PEG-LLIYFNNNVPIDDSGM-resin complex will then be reacted with dipalmitoylphosphoglycerol succinate (Avanti, Alabaster, AL) by the addition of diisopropylcarbodiimide (DIC) (2 equiv., Aldrich Chemical, Milwaukee, WI) and N-methylpyrrolidone (Mallinckrodt, St. Louis, MO). Completeness of reaction will be monitored by the method of Kaiser.

E. Removal of DPGS-PEG-LLIYFNNNVPIDDSGM from photolabile resin.

The DPGS-PEG-LLIYFNNNVPIDDSGM resin complex will be subjected to photolysis at 3500 Angstroms wavelength as described by Rich, D.H. and Gurwara, S.K., *J. Am. Chem. Soc.*, 97:6, 1575-1579, March 19, 1975, the disclosures of which are hereby incorporated herein by reference, in their entirety. Briefly, this will involve placing a suspension of the resin in anhydrous methanol or ethanol in a flask surrounded by a jacket containng 40% $CuSO_4$ solution. The solution will be purged with $O_2$-free nitrogen for 2 hours under slight vacuum, followed by irradiation at 3500 Angstroms for 18 to 24 horus. The suspension will be filtered and washed three times for 2 min. with portions of ethanol, methylene chloride, 50% methylene chloride-ethanol, and ethanol. The filtrates will be combined and concentrated *in vacuo*. The product will then be purified using standard chromatography methods known in the art.

## Example 4

Example 1 will be repeated except that the following procedure will be substituted for Step A.

A. Preparation of a complex of a polymeric resin and the peptide LLIYFNNNVPIDDSGM.

This alternative procedure will involve the use of the fluorenylmethoxycarbonyl (FMOC) protecting group on the α-amino moiety of the protected amino acids. In this case, the protected Methionine (FMOC-Met) will be bound to the resin utilizing the same method in Example 1, using diisopropylethylamine (DIEA) and ethyl acetate with subsequent refluxing. Following binding of the FMOC-Met to the resin, further couplings will be initiated as follows.

i. Deprotection

The FMOC-Met resin complex will be washed 5 x 1 min with dimethylformide DMF or N-methylpyrollidone (NMP). The FMOC groups will be removed with 20% piperidine/NMP x 20 min. This will be repeated, followed by a wash 5 x 1 min with DMF or NMP.

ii. Coupling

An appropriate FMOC protected amino acid (2 equiv.) will be added with 2 equiv of DIC or dicyclohexylcarbodiimide (DCC), hydroxybenzotriazole (2 equiv.) and DMF or NMP. Completeness of the coupling reactions will be monitored using the ninhydrin detection method of Kaiser.

iii. Steps 1 and 2 will be repeated for coupling of additional FMOC-protected amino acids.

The peptide LLIYFNNNVPIDDSGM requires only the sidechain protection of the lysine and aspartic acid residues. If desired, the glutamine residue may also be protected with an O-nitrophenyl group. A number of protecting group schemes are compatible with the synthetic scheme. For example, Boc-Lys with an FMOC sidechain protecting group may be used to prevent the reaction of other amino acids, PEG, or the DPGS with the side chain group. In addition, a Boc-Asp with a β-carboxyfluorenylmethyl (OFm) ester bond may be used to protect the sidechain carboxyl. Prior to removal of the DPGS-PEG-LLIYFNNNVPIDDSGM from the resin, mild conditions such as 20% piperidine and N-methylpyrollidone can be initiated to remove both the FMOC group from the lysine and the OFm group from the aspartic acid.

In addition, a number of sidechain protecting groups may be used compatible with FMOC methodology yet require only mild conditions for removal. For example, FMOC-Asp-(O-Dmab)-OH and FMOC Lys-(Dde)-OH, can be used in Step B of the preparatory methods with subsequent cleavage of the sidechain groups using 2% hydrazine in NMP, DMF, or $CH_2Cl_2$ for one hour. After removal of the sidechains, the DPGS-PEG-LLIYFNNNVPIDDSGM will be washed with 2 x 2 min. NMP.

## Example 5

This example is directed to the preparation of DPPE-MPB-Cys-Arg-Lys-Leu-Gly-Arg-Arg (DPPE-MPB-CRKLGRR) which is a conjugate of the lipid DPPE and a nuclear localization sequence (NLS) having the following formula

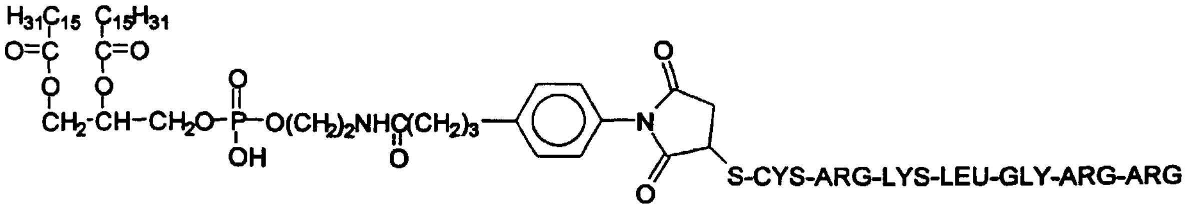

A. Preparation of DPPE-MPB.

DPPE (138.9 mg) and triethylamine (80 mg) were combined in 1:1 chloroform/methanol (15 mL). To the resulting mixture was added 4-(p-maleimidophenyl)butyric acid N-hydroxysuccinimide ester in chloroform (15 mL). The resulting mixture was stirred for about 30 minutes at 10°C, then overnight at room temperature. The clear solution was concentrated *in vacuo*. The residue was washed with chloroform (2 x 20 mL) and the pH was adjusted to pH 3-4 with 2% Hcl. The chloroform layer was washed with water (3x), dried ($Na_2SO_4$), filtered and concentrated *in vacuo*. Lyophilization for 4 hours afforded DPPE-MPB (0.16 g).

B. Preparation of DPPE-MPB-CRKLGRR.

The DPPE-MPB from Step A was coupled to CRKLGRR through the terminal cysteine residue using dicyclohexylcarbodiimide using standard methodology.

## Example 6

This example is directed to the transfection of cells with vesicles containing nuclear localization sequence within the scope of the present invention.

Vesicles were prepared from the cationic lipids DPEPC:DOPE (referred to as "DPDO" in the following table), DPPE-MPB-CRKLGRR from Example 5 (referred to as "DMC" in the following table) and a synthetic cationic lipid (referred to as "SCL" in the following table) having the following formula

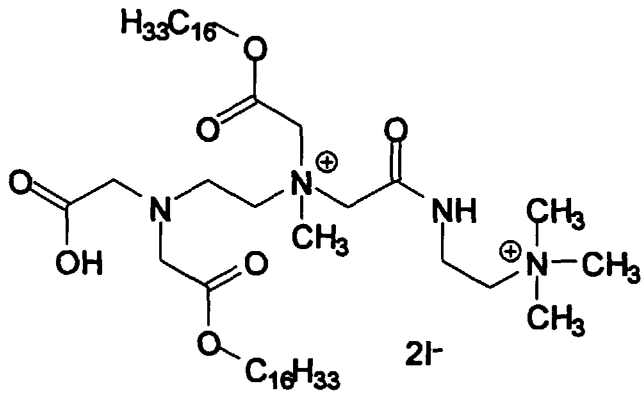

Methods for preparing the above SCL cationic lipid are described, for example, in U.S. Patent No. 5,830,430, the disclosures of which are hereby incorporated herein by reference, in their entirety. Vesicle preparation involved dissolving the above lipids in ethanol at varying concentrations. 20 mL samples were stirred at room temperature for 30 minutes.

Transfections were run in six well plates of NIH/3T3 cells using 30 µL of lipid to 5 µg of DNA (pcmvCAT) diluted in HEPES buffered-saline. The total volume of each well was 0.2 mL. The mixtures were incubated at room temperature for 45 mins followed by plate incubations of 48 hrs at 37°C. Protein expression was measured by CAT ELISA (Boehringer Mannheim). The results are shown in Table 3.

Table 3

| Example No. | Composition | CAT Expression (ng/mL) |
|---|---|---|
| 6A | Control | 2.3 |
| 6B | DPEPC:DOPE | 2605 |
| 6C | SCL | 3396.1 |
| 6D | DMC | 3.1 |
| 6E | DMC:DOPE (6:1 w/w) | 5.3 |
| 6F | DMC:DOPE (1:1 w/w) | 0.1 |
| 6G | DMC:DOPE (1:6 w/w) | 5.6 |
| 6H | DMC (5 %) + DPDO | 2692.8 |
| 6I | DMC (10%) + DPDO | 1802.9 |
| 6J | DMC (5%) + SCL | 3561.4 |
| 6K | DMC (10%) + SCL | 3715.1 |

The results tabulated above indicate that improved gene expression may be obtained with vesicles of the present invention, particularly vesicles which include nuclear localization sequences as well as cationic lipid.

**Example 7**

This example is directed to the preparation and use of a targeted composition comprising both a nuclear localization sequence and and Sialyl Lewis X targeting.
The product obtained in Example 3 (DPGS-PEG-LLIYFNNNVPIDDSGM) will be incorporated into a cationic lipid composition which further includes Sialyl Lewis X conjugated to a lipid carrier. As discussed above, the preparation of conjugates of Sialyl Lewis X and lipids is described in EPO 727 225 A2, the disclosures of which are hereby incorporated herein by reference, in their entirety. The preparation of this targeted composition may be achieved as follows.
The Sialyl Lewis X conjugate, DPGS-PEG-LLIYFNNNVPIDDSGM, DPEPC and DOPE will be mixed in a molar ratio of 1:1:8:8 in the presence of perfluorohexane (100 µg/mL) with a lipid concentration of 1 mg/mL in an ESPE Capmix. The perfluorohexane may be employed in concentrations of 10 to 100 µL/mL of lipid/conjugate mixture. To this suspension will then be added a plasmid construct containing the HLA-B7 gene with a CMV promoter in a 1:2 w/w ratio DNA/lipid mixture. This suspension will be agitated by hand and genetic transfection will be performed utilizing the methods described herein.

**Example 8**

Example 3 will be repeated except a polyethylene glycol linker will not be employed and the peptide LLIYFNNNVPIDDSGM will be attached directly to DPGS via an anhydride bond.

**Example 9**

This example is directed to the preparation of N-(1,2-dipalmitoyl-sn-glycero-3-succinyl)-PEG-Anti-interleukin-2 fab (DPGS-PEG-anti-IL-2 fab) and its use in methods and compositions of the present invention.

A. Preparation of DPGS-PEG-anti-IL-2 fab

Anti-interleukin-2 (anti-IL-2) monoclonal antibody will be obtained from a commercial source, such as Oncogene Research Products, Cambridge, MA. The fab fragment from the anti-IL-2 will be isolated using any of a number of protein separation techniques which are well known to those skilled in the art. The fab fragment will be conjugated to a lipid, such as DPPE or DPGS, as described below.
To a cooled (5 to 10°C), stirred solution of human anti-IL-2 fab in aqueous buffer (20 mL) at a pH of 8.5 will be added dropwise a solution of 3-succinamoyl-oxycarbonyl-polyethyleneglycol-imino-succinate- 1,2-dipalmitoyl-sn-glycerol (DPGS-ω-carboxy-PEG-succinimide) and acetonitrile (10 mL). The DPGS-ω-carboxy-PEG-succinimide may be prepared utilizing the methods described in copending U.S. application Serial No. 08/851,780, filed May 6, 1997, the disclosures of which are hereby incorporated herein by reference, in their entirety. The temperature of the resulting mixture will be equilibrated to room temperature and the reaction mixture will be stirred for about 48 hours. The mixture will be concentrated *in vacuo* and the residual salts will be dialyzed away using a dialysis bag having a molecular weight cutoff of about 3500, equilibrated against water. The resulting dialyzed solution will be frozen and lyophilized to yield 12 mg of the title material (DPGS-PEG-anti-IL-2 fab) as a white solid.

B. Preparation of Targeted Composition

The DPGS-PEG-anti-IL-2 fab from Step A will then be incubated with DPPE-MPB-CRKLGRR from Example 5 above and a 40-mer antisense oligonuceotide. The antisense oligonucleotide will be prepared to bind to the noncoding strand of the tumor necrosis factor (TNF) gene in arthritic synovial tissue cells. The gene sequence of TNF will be analyzed and a section of a coding region will be used to generate the theoretical sequence of an antisense oligonucleotide complementary to the non-coding strand. The oligonucleotide will be synthesized according to any of a number of methods well known to those skilled in the art. The ratio of antisense oligonucleotide to lipid in the incubation mix is optimally 1:5 (w/w) and its activity will be enhanced by incubating in the presence of 10.0 millimolar $CaCl_2$. About 1.5 mL of the resulting composition, having a concentration of 1 mg/mL, will be injected into the knee joint of a patient afflicted with rheumatoid arthritis.

**Example 10**

This example is directed to the treatment of rheumatoid arthritis with ribozyme facilitated by anti IL-2 antibody targeting.

Example 7 will be repeated except that the nucleic acid to be delivered are hammerhead ribozymes. Suitable procedures for making and using ribozymes is disclosed in U.S. Patent No. 4,987,071, the disclosures of which are hereby incorporated herein by reference, in their entirety.

Ribozymes targeting selected regions of mRNA associated with IL-2 antibody will be chosen to cleave the target RNA in a manner which preferably inhibits translation of the RNA. Genes will be selected such that inhibition of translation will preferably inhibit cell replication, for example, by inhibiting production of a necessary protein.

Ribozymes useful in this invention may be produced by gene transcription as described in U.S. Patent No. 4,987,071, the disclosures of which are hereby incorporated herein by reference in their entirety, or by chemical synthesis. Chemical synthesis of RNA is similar to that for DNA synthesis. The additional 2'-OH group in RNA, however, generally requires an alternative protecting group strategy to deal with selective 3'-5' internucleotide bond formation, as well as RNA susceptibility to degradation in the presence of bases. The recently developed method of RNA synthesis utilizing the t-butyldimethylsilyl group for the protection of the 2'- hydroxyl is the most reliable method for synthesis of ribozymes.

Blocked ribozymes will be cleaved from the solid support (*e.g*., CPG), and the bases and diphosphoester moiety will be deprotected in a sterile vial by dry ethanolic ammonia (2 mL) at 55°C for 16 hours. The reaction mixture will be cooled on dry ice, transferred into a sterile screw cap vial and lyophilized.

To remove the 2'-t-butyldimethylsilyl groups from the ribozyme, the residue will be suspended in 1M t-n-butylammonium fluoride in dry THF (TBAF), using a 20 fold excess of the reagent for each silyl group, for 16 hours at ambient temperature (e.g., about 15° to 25°C). The reaction will be quenched by adding an equal volume of sterile 1M triethylamine acetate, pH 6.5. The sample will be cooled and concentrated on a SpeedVac to about half of the initial volume.

The second step is purification of a completely deblocked ribozyme by a treatment of 2% trifluoroacetic acid on a C4 300.ANG. 5 mm DeltaPak column in an acetonitrile gradient. Solvents used for this second step will be: (A) 0.1M triethylammonium acetate, pH 6.8) and (B) 80% acetonitrile, 0.1 M triethylammonium acetate, pH 6.8). The elution profile will be: 5% (B) for 5 minutes, a linear gradient of 5% (B) to 15% (B) over 60 minutes, 15% (B) for 10 minutes, and a linear gradient of 15% (B) to 0% (B) over 10 minutes.

The fraction containing ribozyme will be cooled and lyophilized on a SpeedVac. Solid residue will be dissolved in a minimum amount of ethanol and sodium perchlorate will be dissolved in a minium amount of acetone. The ribozyme will be collected by centrifugation, washed three times with acetone, and lyophilized.

A hairpin at the 5' end of the ribozyme will be useful to ensure that the required transcription initiation sequence (GG or GGG or GGGAG) will not bind to some other part of the ribozyme and thus affect regulation of the transcription process. The 5' hairpin will also be useful to protect the ribozyme from 5'-3' exonucleases. A selected hairpin at the 3' end of the ribozyme gene will also be useful since it acts as a transcription termination signal, and protects the ribozyme from 3'-5' exonuclease activity. One example of a known termination signal is that present on the T7 RNA

polymerase system. This signal is about 30 nucleotides in length. Other 3' hairpins of shorter length may be used to provide desirable termination and RNA stability. Such hairpins may be inserted within the vector sequences to allow standard ribozymes to be placed in an appropriate orientation and expressed with such sequences attached.

Poly(A) tails will also be useful to protect the 3' end of the ribozyme. These may be provided, for example, by including a poly(A) signal site in the expression vector (to signal a cell to add the poly(A) tail *in vivo*), or by introducing a poly(A) sequence directly into the expression vector. In the former approach, the signal will preferably be located to prevent unwanted secondary structure formation with other parts of the ribozyme. In the latter approach, the poly(A) stretch may reduce in size over time when expressed *in vivo*, and thus the vector may need to be checked over time. Care must be taken in the addition of a poly(A) tail which binds poly(A) binding proteins to prevent the ribozyme from acting.

Selected ribozymes may be administered prophylactically, i.e., to prevent the occurrence of rheumatoid arthritis in patients, or to patients already suffering from rheumatoid arthritis. Such administration may involve, for example, exogenous delivery of the ribozyme to an infected tissue by means of an appropriate targeted composition according to the invention as described herein.

## Example 11

This example is directed to the preparation of a nuclear delivery vehicle with enhanced protection of macromolecules against lysosomal action.

The targeted composition described in Example 6 will be prepared, further incorporating a plasmid construct comprised of an adenovirus (AD)-linked to polylysine (PL) according to the method described in Schwarzenberger et al., *Blood*, **87**, No. 2, 1996, 472-478, the disclosures of which are hereby incorporated herein by reference, in their entirety. The intracellular release of the AD-PL molecule will protect the nuclear localization sequence peptide from endosomolysis.

## Example 12

This example is directed to bone marrow transplantation therapy as a treatment for gene deficiency.

Bone marrow aspirate (100 mL) will be obtained from a patient diagnosed with adenosine deaminase deficiency. Targeted carriers will be made to incorporate the gene coding adenosine deaminase in a plasmid construct using the CMV promoter. The gene will be complexed to AD polylysine as described in Example 11. Polylysine AD will further be complexed with streptavidin according to the procedure of Schwarzenberger et al., *Blood*, **87**, No. 2, 1996, 472-478, the disclosures of which are hereby incorporated herein by reference, in their entirety. DPGS-PEG-LLIYFNNNVPIDDSGM, as prepared according to the method described, for example, in Example 3, will be reacted at its C-terminal with the polylysine AD-streptavidin complex to construct the NLS conjugate, SA-PL-AD-DPGS-PEG-LLIYFNNNVPIDDSGM. The method may involve a variation of the same protection-deprotection procedures described in Examples 1 and 2.

Steel factor (SLF) will be biotinylated and added to the washed bone marrow sample. After one hour, the SA-PL-AD-DPGS-PEG-LLIYFNNNVPIDDSGM and the adenosine deaminase plasmid construct will be co-administered to the cells. Transfection of the bone marrow pluripotent stem cells will result in cells competent to make adenosine deaminase, and the marrow cells will be transplanted back into the patient.

## Example 13

This example is directed to the preparation and use of a conjugate of dipalmitoylglycerylsuccinyl (DPGS), polyethylene glycol (PEG) and basic fibroblast growth factor (bFGF), also referred to herein as DPGS-PEG-bFGF.

A. Preparation of DPGS-NH-PEG-NH-maleimide, which has the following formula.

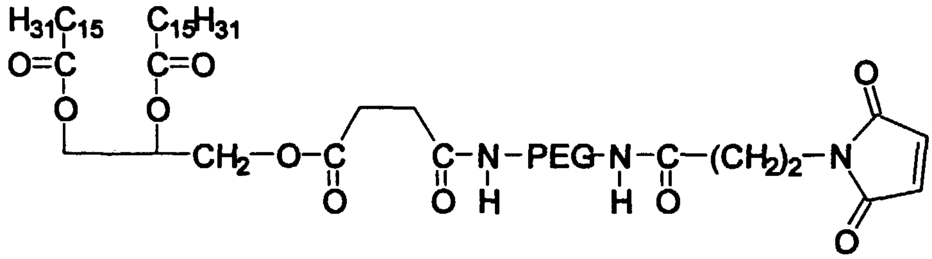

i. Preparation of dipalmitoyl glycerol succinate-polyethyleneglycolamine (DPGS-NH-PEG-$NH_2$).

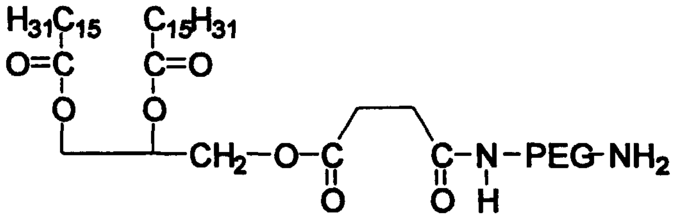

Into a round bottom flask (100 mL) was added a solution of DPGS (98 mg, 0.147 mmole, 1 eq.) in $CH_2Cl_2$. Into a separate round bottom flask (100 mL) was added a solution of $H_2N$-PEG-$NH_2$ (PEG-diamine) (50 mg, 0.147 mmole, 1 eq.) in $CH_2Cl_2$. To the PEG-diamine solution was added immediately a solution of DCC (31 mg, 0.15 mmole, 1.02 eq.) in $CHCl_3$ (5 mL). The resulting mixture of DCC and PEG-diamine was added dropwise to the DPGS at about 0 to about 5°C. The resulting reaction mixture was equilibrated with stirring to room temperature. After stirring for 24 hours, distilled-deionized water (50 mL) was added to the reaction mixture and the resulting white precipitate (dicyclohexylurea) was removed by filtration. The lower organic layer was isolated using a 250 mL separatory funnel and dried ($Na_2SO_4$). The dried organic solution was filtered and $CH_3CN$ (80 mL) was added to the filtrate. The remaining white precipitate was removed by filtration and the filtrate was concentrated *in vacuo* to yield 0.44 g of DPGS-NH-PEG-$NH_2$ as a solid product. IR: 1700 $cm^{-1}$. TLC: $R_f$ 0.65.

ii. Preparation of DPGS-PEG-NH-PEG-NH-maleimide.

Into a round bottom flask (100 mL) was added a solution of DPGS-NH-PEG-$NH_2$ from Step A (0.44 g, 0,13 mmole, 1 eq.) in $CH_2Cl_2$ (10 mL). To this solution was added a solution of triethylamine (13 mg, 0.13 mmole, 1 eq.) in $CH_2Cl_2$. To this mixture was added dropwise a solution of 34.6 mg N-maleoyl-β-alanine N-hydroxysuccinimide ester (34.6 mg) in $CH_2Cl_2$ (10 mL). After stirring overnight at room temperature, deionized-distilled water (30 mL) was added to the reaction mixture and stirring was continued an additional 3 hours. The lower organic layer was isolated using a separatory funnel and dried ($NaSO_4$). The organic layer was filtered and the filtrate was concentrated *in vacuo* to afford 360 mg of the title compound as a wax-like white solid. IR: 1740 $cm^{-1}$, 1670 $cm^{-1}$, 1100 $cm^{-1}$. TLC: Rf 0.75.

B. Preparation of DPGS-PEG-bFGF.

Basic fibroblast growth factor (bFGF), modified to contain an additional C-terminal cysteine was purchased from Prizm Pharmaceutical (San Diego, CA) and was conjugated to the DPGS-NH-PEG-NH-maleimide from Step A as follows.

bFGF was suspended at a concentration of 1 mg/mL in coupling buffer of 150 mM NaCl, 10 mM MOPS, 0.1

mM EDTA and 50 μm DTPA (pH 6.5) (all buffer materials obtained from Sigma Chemical, St. Louis, MO). The DPGS-NH-PEG-NH-maleimide from Step A (0.2 to 10 mM) will be added to the bFGF/buffer suspension and the resulting mixture will be incubated for 16 hours at 4°C. The resulting reaction mixture will be analyzed on a polyacrylamide electrophoresis gel to confirm that the bFGF became bound to the intermediate from Step A. This can be readily determined by observing changes in molecular weight.

Targeted vesicles will be prepared containing 1 weight % percent of the DPGS-PEG-bFGF conjugate, as per the procedure described in Example 7.

## Example 14

Example 9 will be repeated except telomerase will be substitued for the anti-interleukin-2 fab targeting agent to provide N-(1 ,2-dipalmitoyl-sn-glycero-3-succinyl)-PEG-telomerase (DPGS-PEG-telomerase).

The disclosures of each patent, patent application and publication cited or described in this document are hereby incorporated herein by reference, in their entirety.

Various modification of the invention, in addition to those described herein, will be apparent to those skilled in the art from the foregoing description. Such modifications are also intended to fall within the scope of the appended claims.

## Claims

**1.** A composition for delivering a compound into a cell comprising, in combination with the compound to be delivered, an organic halide, a targeting ligand and a nuclear localization sequence.

**2.** A composition according to Claim 1 which further comprises a carrier.

**3.** A composition according to Claim 1 or 2 wherein said organic halide is selected from the group consisting of a gaseous organic halide and a liquid organic halide.

**4.** A composition according to Claim 3 wherein said organic halide is a gaseous organic halide.

**5.** A composition according to Claim 3 wherein said organic halide is a liquid.

**6.** A composition according to Claim 1 or 2 wherein said organic halide is a gaseous precursor.

**7.** A composition according to any preceding Claim wherein said organic halide is a fluorinated compound.

**8.** A composition according to Claim 7 wherein said fluorinated compound is a perfluorinated compound.

**9.** A composition according to Claim 8 wherein said perfluorinated compound is a perfluorocarbon.

**10.** A composition according to Claim 2 wherein said organic halide is a perfluoroether compound.

**11.** A composition according to Claim 7 wherein said perfluorocarbon is a liquid.

**12.** A composition according to Claim 11 wherein said liquid perfluorocarbon is a gaseous precursor.

**13.** A composition according to Claim 9 wherein said perfluorocarbon is a gas.

**14.** A composition according to Claim 1 or 2 wherein said organic halide is selected from the group consisting of 1-bromo-nonafluorobutane, perfluorooctyliodide, perfluorooctylbromide, 1-chloro-1 -fluoro-1 -bromomethane, 1,1,1 -trichloro-2,2,2-trifluoroethane, 1 ,2-dichloro-2,2-difluoroethane, 1,1 -dichloro-1 ,2-difluoroethane, 1,2-dichloro-1, 1 ,3-trifluoropropane, 1 -bromoperfluorobutane, 1-bromo-2 ,4-difluorobenzene, 2-iodo-1, 1,1 -trifluoroethane, 5-bromovaleryl chloride, 1 ,3-dichlorotetrafluoroacetone, bromine pentafluoride, 1-bromo-1 ,1 ,2,3,3,3-hexafluoropropane, 2-chloro-1 ,1,1,4,4 ,4-hexafluoro-2-butene, 2-chloropentafluoro-1 ,3-butadiene, iodotrifluoroethylene, 1,1 ,2-trifluoro-2-chloroethane, 1,2-difluorochloroethane, 1,1 -difluoro-2-chloroethane, 1,1 -dichlorofluoroethane, heptafluoro-2-iodopropane, bromotrifluoroethane, chlorotrifluoromethane, dichlorodifluoromethane, dibromofluoromethane, chloropentafluoroethane, bromochlorodifluoromethane, dichloro-1 ,1 ,2,2-tetrafluoroethane, 1,1,1,3,3-pentafluoropentane, perfluorotributylamine, perfluorotripropylamine, 3-fluorobenzaldehyde, 2-fluoro-5-nitrotoluene,

3-fluorostyrene, 3,5-difluoroaniline, 2,2 ,2-trifluoroethylacrylate, 3-(trifluoromethoxy)-acetophenone, 1,2,2,3,3,4,4-octafluorobutane, 1,1,1,3,3-pentafluorobutane, 1-fluorobutane, 1,1,2,2,3,3,4,4-octafluorobutane, 1,1,1 ,3,3-pentafluorobutane, perfluoro-4 methylquinolizidine, perfluoro-N-methyl-decahydroquinone, perfluoro-N-methyl-decahydroisoquinone, perfluoro-N-cyclohexyl-pyrrolidine, perfluoroheptane, perfluorocyclohexane, perfluoromethane, perfluoroethane, perfluoropropane, perfluorobutane, perfluoropentane, perfluorohexane, perfluoroheptane, perfluorooctane, perfluorononane, perfluorodecane, perfluorododecane, perfluoro-2-methyl-2-pentene, perfluorocyclohexane, perfluorodecalin, perfluorododecalin, perfluoropropylene, perfluorocyclobutane, perfluoro-2-butyne, perfluoro-2-butene, perfluorobuta-1,3-diene, perfluorobutylethyl ether, bis(perfluoroisopropyl) ether, bis(perfluoropropyl) ether, perfluorotetrahydropyran, perfluoromethyl tetrahydrofluran, perfluoro-t-butyl methyl ether, perfluoro isobutyl methyl ether, perfluoro-n-butyl methyl ether, perfluoro isopropyl ethyl ether, perfluoro-n-propyl ethyl ether, perfluorocyclobutyl methyl ether, perfluorocyclopropyl ethyl ether, perfluoroisopropyl methyl ether, perfluoro-n-propyl methyl ether, perfluorodiethyl ether, perfluorocyclopropyl methyl ether, perfluoromethyl ethyl ether, perfluorodimethyl ether, sulfur hexafluoride and selenium hexafluoride.

**15.** A composition according to Claim 14 wherein said organic halide is selected from the group consisting of perfluoromethane, perfluoroethane, perfluoropropane, perfluorobutane, perfluoropentane, perfluorohexane, perfluoroheptane, perfluorooctane, perfluorononane, perfluorodecane, perfluorododecane, perfluoro-2-methyl-2-pentene, perfluorocyclohexane, perfluorodecalin, perfluorododecalin, perfluorohexane and perfluorocyclohexane.

**16.** A composition according to Claim 14 wherein said organic halide is selected from the group consisting of perfluorobutylethyl ether, bis(perfluoroisopropyl) ether, bis(perfluoropropyl) ether, perfluorotetrahydropyran, perfluoromethyl tetrahydrofluran, perfluoro-t-butyl methyl ether, perfluoroisobutyl methyl ether, perfluoro-t-butyl methyl ether, perfluoroisopropyl ethyl ether, perfluoro-n-propyl ethyl ether, perfluorocyclobutyl methyl ether, perfluorocyclopropyl ethyl ether, perfluoroisopropyl methyl ether, perfluoro-n-propyl methyl ether, perfluorodiethyl ether, perfluorocyclopropyl methyl ether, perfluoromethyl ethyl ether and perfluorodimethyl ether.

**17.** A composition according to Claim 14 wherein said organic halide is selected from the group consisting of perfluorohexane and perfluorocyclohexane.

**18.** A composition according to any preceding Claim wherein said targeting ligand is selected from the group consisting of a protein, an antibody, an antibody fragment, a hormone, a hormone analog, a glycoprotein, a lectin, a peptide, a polypeptide, an amino acid, a sugar, a saccharide, a carbohydrate, a vitamin, a steroid, a steroid analog, a cofactor, a bioactive agent and genetic material.

**19.** A composition according to Claim 18 wherein said targeting ligand is selected from the group consisting of Sialyl Lewis X, mucin, hyaluronic acid, LFA-1, VLA-4, fibrinogen, von Willebrand factor, vitronectin, VCAM-1, CD49d/CD29, methyl-$\alpha$-D-mannopyranoside, N-formal peptide, C5a, leukotriene B4, platelet activating factor, IL-8/NAP- 1, CTAP-III, $\beta$-thromboglobulin, NAP-2,gro/MGSA, ENA-78, MCP-1, MAP-1$\alpha$,$\beta$, RANTES, and I-309.

**20.** A composition according to Claim 18 wherein said targeting ligand is Sialyl Lewis X.

**21.** A composition according to any preceding Claim wherein said nuclear localization sequence is selected from the group consisting of a peptide, a protein, a receptor, a transcription factor and an enzyme.

**22.** A composition according to any preceding Claim wherein said nuclear localization sequence is selected from the group consisting of influenza virus nucleoprotein, karyopherin $\beta$1, human stat1 gene, m-importin, mouse homolog of nuclear pore targeting complex, hepatitis B virus (HBV) polymerase, glucocortocoid receptor (GlucR), interferon-regulated factors ISGF-3 and GAF, yeast mating switch/HO endonuclease promoter SW15, *Drosophila melanogaster* morphogen *dorsal*, nuclear factors NF-$\kappa\beta$ abd NF-AT, T-ag, c-rel, lamin $B_2$, GrH receptor, c-fos, cofilin, rNFIL-6, NF-ATplc, PICA C-subunit, $p42^{mapk}$/$p44^{erk1}$, $p90^{rsk}$, PKC-$\alpha$, *lodestar*, v-jun, cyclin B (B-type cyclins), adenovirus 5 E1a protein, xnf7, PwA33, Rb-1, p53, c-myc, PTF1, HMG1/2 and tegument protein pp65 (UL83) of human cytomegalovirus.

**23.** A composition according to any one of Claims 1 to 21 wherein said nuclear localization sequence is a peptide comprising an amino acid sequence selected from the group consisting of SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26 and SEQ

ID NO:27.

**24.** A composition according to Claim 23 wherein said nuclear localization sequence is a peptide comprising an amino acid sequence selected from the group consisting SEQ ID NO:17, SEQ ID NO:20, SEQ ID NO:21, SEQ ID NO:22, SEQ ID NO:23, SEQ ID NO:24, SEQ ID NO:25 and SEQ ID NO:26.

**25.** A composition according to any preceding Claim wherein said compound to be delivered is selected from the group consisting of a pharmaceutical agent, a synthetic organic molecule, a protein, a peptide and genetic material.

**26.** A composition according to Claim 25 wherein said compound to be delivered is genetic material.

**27.** A composition according to Claim 26 wherein said genetic material is selected from the group consisting of a mutant gene, an antisense oligonucleotide and a ribozyme.

**28.** A composition according to Claim 27 wherein said genetic material is an antisense oligonucleotide which hybridizes to a nucleic acid molecule encoding a protein selected from the group consisting of tumor necrosis factor receptor, gamma interferon receptor and interleukin 1 receptor.

**29.** A composition according to Claim 27 wherein said genetic material is a ribozyme which disrupts nucleic acid molecules encoding a protein selected from the group consisting of tumor necrosis factor receptor, gamma interferon receptor and interleukin 1 receptor.

**30.** A composition according to Claim 27 wherein said genetic material is a mutant gene which encodes a defective receptor selected from the group consisting of tumor necrosis factor, gamma interferon and interleukin 1.

**31.** A composition according to Claim 2 wherein said carrier is selected from the group consisting of polymers, lipids, proteins and metal ions.

**32.** A composition according to Claim 31 wherein said carrier comprises a lipid.

**33.** A composition according to Claim 32 wherein said lipid is a cationic lipid.

**34.** A composition according to Claim 38 wherein said cationic lipid is N-[ 1 - (2 ,3-dioleoyloxy)propyl]-N, N ,N-trimethylammonium chloride.

**35.** A composition according to Claim 31 wherein said carrier comprises a polymer.

**36.** A composition according to Claim 35 wherein said polymer is selected from the group consisting of polyethylenes, polyoxyethylenes, polypropylenes, pluronic acids and alcohols, polyvinyls, polyvinylpyrrolidone, arabinans, fructans, fucans, galactans, galacturonans, glucans, mannans, xylans, levan, fucoidan, carrageenan, galactocarolose, pectin, pectic acid, amylose, pullulan, glycogen, amylopectin, cellulose, carboxylmethylcellulose, hydroxypropyl methylcellulose, dextran, pustulan, chitin, agarose, keratan, chondroitin, dermatan, hyaluronic acid, alginic acid, homopolymers and heteropolymers containing one or more of an aldose, ketose, acid, amine, erythrose, threose, ribose, arabinose, xylose, lyxose, allose, altrose, glucose, mannose, gulose, idose, galactose, talose, erythrulose, ribulose, xylulose, psicose, fructose, sorbose, tagatose, glucuronic acid, gluconic acid, glucaric acid, galacturonic acid, mannuronic acid, guluronic acid, glucosamine, galactosamine and neuraminic acid.

**37.** A composition according to Claim 31 wherein said carrier is selected from the group consisting of Lipofectin, Lipofectamine, Transfectace, Transfectam, Cytofectin, DMRIE, DLRIE, GAP-DLRIE, DOTAP, DOPE, DMEAP, DODMP, DOPC, DDAB, DOSPA, EDLPC, EDMPC, DPH, TMADPH, CTAB, lysyl-PE, DC-Chol, -alanyl cholesterol, DCGS, DPPES, DCPE, DMAP, DMPE, DOGS, DOFIME, DPEPC, Pluronic, Tween, BRIJ, plasmalogen, phosphatidylethanolamine, phosphatidylcholine, glycero-3-ethylphosphatidylcholine, dimethyl ammonium propane, trimethyl ammonium propane, diethylammonium propane, triethylammonium propane, dimethyldioctadecylammonium bromide, a sphingolipid, sphingomyelin, a lysolipid, a glycolipid, a sulfatide, a glycosphingolipid, cholesterol, cholesterol ester, cholesterol salt, oil, 1 ,2,-dioleoyl-sn-glycerol, N-succinyldioleoylphosphatidylethanolamine, 1,3-dipalmitoyl-2-succinyl-glycerol, 1,2-dipalmitoyl-sn-3-succinylglycerol, palmitoylhomocystiene, 1-hexadecyl-2-palmitoylglycerophosphatidylethanolamine, N,N''-bis(dodecylaminocarbonylmethylene)-N ,N'-bis((-N ,N ,N-trimethylammoniumethylaminocarbonylethylene) ethylenediamine tetraiodide, N,N''-bis(hexadecylaminocarbonylmethylene)-N , N,N''-tris((-N ,N ,N-trimethylammoniumethylaminocarbonylmethylenediethylenetriamine hexaiodide, N,N'-bis(dodecylaminocarbonylmethylene)-N , N''-bis((-N , N, N-trimethylammoniumethylaminocarbonylmethylene) cyclohexylene-1,4-diamine tetra iodide, 1,1 ,7,7-tetra((-N ,N , N ,N-tetramethylammoniumethylaminocarbonylmethylene)-3-hexadecylaminocarbonylmethylene-1 ,3,7-triaazaheptane heptaiodide, and N ,N ,N'N'-tetra((N ,N ,N-trimethylammoniumethylaminocarbonylmethylene)-N'-( 1,2-dioleoylglycero-3-phosphoethanolaminocarbonylmethylene) diethylenetriamine tetraiodide.

**38.** A composition according to Claim 31 wherein said carrier comprises a compound selected from the group consisting of dioleoylphosphatidylethanolamine, a fatty acid, a lysolipid, phosphatidylcholine, phosphatidylethanolamine, phosphatidylserine, phosphatidylglycerol, phosphatidylinositol, a sphingolipid, a glycolipid, a glucolipid, a sulfatide, a glycosphingolipid, phosphatidic acid, palmitic acid, stearic acid, arachidonic acid, oleic acid, a lipid bearing a polymer, a lipid bearing a sulfonated saccharide, cholesterol, tocopherol hemisuccinate, a lipid with an ether-linked fatty acid, a lipid with an ester-linked fatty acid, a polymerized lipid, diacetyl phosphate, stearylamine, cardiolipin, a phospholipid with a fatty acid of 6-8 carbons in length, a phospholipid with asymmetric acyl chains, 6-(5-cholesten-3b-yloxy)-1-thio-b-D-galactopyranoside, digalactosyldiglyceride, 6-(5-cholesten-3β-yloxy)hexyl-6-amino-6-deoxy-1-thio-b-D-galactopyranoside, 6-(5-cholesten-3b)-yloxy)hexyl-6-amino-6-deoxyl-1 -thio-α-D-mannopyranoside, 12-(((7'-diethylamino-coumarin-3-yl )carbonyl )methylamino)octadecanoic acid, N-[12-(((7'-diethylaminocoumarin-3-yl )carbonyl )methyl-amino) octadecanoyl]-2-aminopalmitic acid, cholesteryl )4'-trimethyl-ammonio)butanoate, N-succinyldioleoylphosphatidylethanolamine, 1,2-dioleoyl-sn-glycerol ,1,2-dipalmitoyl-sn-3-succinyl-glycerol, 1,3-dipalmitoyl-2-succinylglycerol, 1-hexadecyl-2-palmitoylglycerophosphoethanolamine, palmitoylhomocysteine and combinations thereof.

**39.** A composition according to Claim 38 wherein said carrier comprises phosphatidylcholine.

**40.** A composition according to Claim 38 wherein said phosphatidylcholine is selected from the group consisting of dioleoylphosphatidylcholine, dimyristoylphosphatidylcholine, dipentadecanoylphosphatidylcholine, dilauroylphosphatidylcholine, dioleoylphosphatidylcholine, dipalmitoylphosphatidylcholine and distearoylphosphatidylcholine.

**41.** A composition according to Claim 38 wherein said carrier comprises phosphatidylethanolamine.

**42.** A composition according to Claim 41 wherein said phosphatidylethanolamine is dioleoylphosphatidylethanolamine.

**43.** A composition according to Claim 38 wherein said carrier comprises sphingolipid.

**44.** A composition according to Claim 43 wherein said sphingolipid is sphingomyelin.

**45.** A composition according to Claim 38 wherein said carrier comprises a glycolipid.

**46.** A composition according to Claim 45 wherein said glycolipid is selected from the group consisting of ganglioside GM1 and ganglioside GM2.

**47.** A composition according to Claim 38 wherein said carder comprises a lipid bearing a polymer.

**48.** A composition according to Claim 47 wherein said polymer of said lipid bearing a polymer is selected from the group consisting of polyethylene glycol, chitin, hyaluronic acid and polyvinylpyrrolidone.

**49.** A composition according to Claim 48 wherein said polymer of said lipid bearing a polymer is polyethylethylene glycol.

**50.** A composition according to Claim 49 wherein said polyethylene glycol is selected from the group consisting of polyethylene glycol having a molecular weight of about 2000, 5000, and 8000.

**51.** A composition according to Claim 38 wherein said carrier comprises a phospholipid with asymmetric acyl chains having one acyl chain of about 6 carbons in length and another acyl chain of about 12 carbons in length.

**52.** A composition according to Claim 2 wherein said carrier comprises about 82 mole percent dipalmitoylphosphatidylcholine, about 8 mole percent dipalmitoylphosphatidylethanolamine-polyethyleneglycol 5000 and about 10 mole percent dipalmitoylphosphatidic acid.

**53.** A composition according to Claim 2 wherein said carrier comprises a surfactant.

**54.** A composition according to Claim 53 wherein said surfactant is a fluorosurfactant.

**55.** A composition according to claim 25 which comprises telomerase.

**56.** A composition according to any preceding claim further comprising a fusion peptide.

**57.** A method for delivery a compound into a cell comprising administering to the cell a composition according to any one of claims 1 to 54.

**58.** A method according to claim 57 further comprising applying ultrasound to said cell.

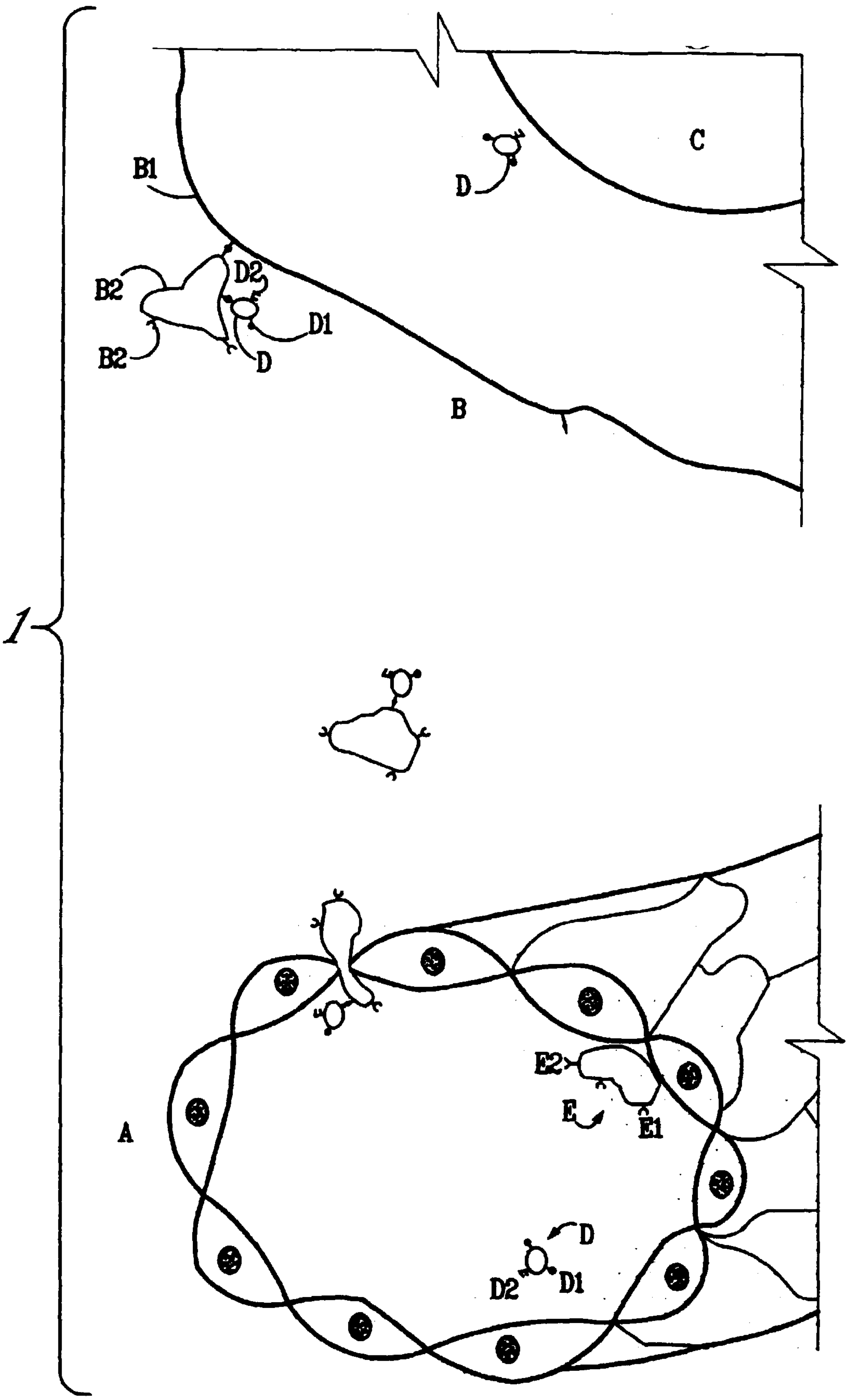


FIG. 1

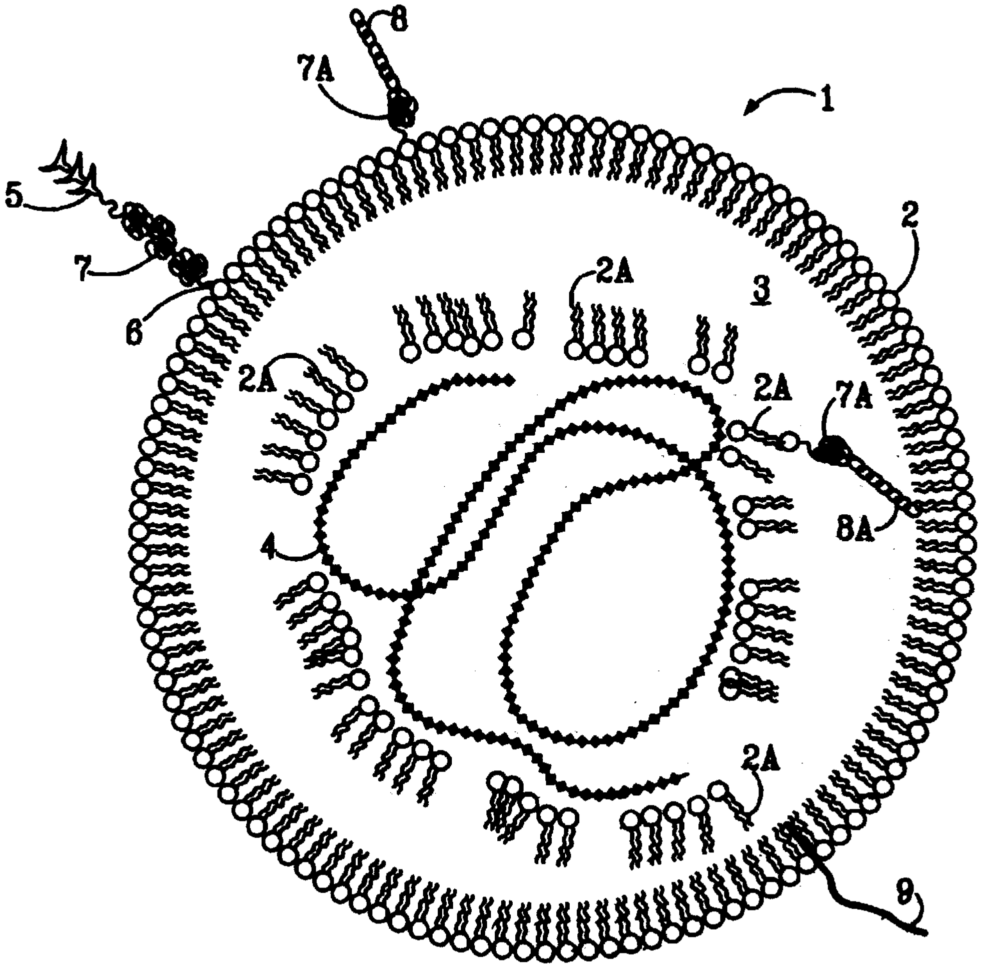


FIG. 2